(19) Europäisches Patentamt
European Patent Office
Office européen des brevets

(11) **EP 3 219 810 B1**

(12) **EUROPEAN PATENT SPECIFICATION**

(45) Date of publication and mention
of the grant of the patent:
**05.01.2022  Bulletin 2022/01**

(21) Application number: **15858194.2**

(22) Date of filing: **13.11.2015**

(51) Int Cl.:
*C12Q 1/68* *(2018.01)*        *G16B 30/00* *(2019.01)*
*C12N 9/22* *(2006.01)*

(86) International application number:
**PCT/KR2015/012255**

(87) International publication number:
**WO 2016/076672 (19.05.2016 Gazette 2016/20)**

(54) **METHOD FOR DETECTING OFF-TARGET SITE OF GENETIC SCISSORS IN GENOME**

VERFAHREN ZUR DETEKTION EINER OFF-TARGET-STELLE EINER GENETISCHEN SCHERE IN EINEM GENOM

PROCÉDÉ DE DÉTECTION DE SITE HORS-CIBLE DE CISEAUX GÉNÉTIQUE DANS LE GÉNOME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2014  US 201462079945 P
24.09.2015  KR 20150135702**

(43) Date of publication of application:
**20.09.2017  Bulletin 2017/38**

(73) Proprietor: **Institute for Basic Science
Daejeon 34047 (KR)**

(72) Inventors:
• **KIM, Jin Soo
Seoul 08826 (KR)**
• **KIM, Dae Sik
Incheon 21596 (KR)**
• **BAE, Sang Su
Seoul 08826 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
**WO-A1-2014/065596    WO-A2-2013/169398
US-A1- 2014 295 556**

• **T. J. CRADICK ET AL: "CRISPR/Cas9 systems
targeting ?-globin and CCR5 genes have
substantial off-target activity", NUCLEIC ACIDS
RESEARCH, vol. 41, no. 20, 1 November 2013
(2013-11-01), pages 9584-9592, XP055186069,
ISSN: 0305-1048, DOI: 10.1093/nar/gkt714**
• **S. W. CHO ET AL: "Analysis of off-target effects
of CRISPR/Cas-derived RNA-guided
endonucleases and nickases", GENOME
RESEARCH, vol. 24, no. 1, 19 November 2013
(2013-11-19), pages 132-141, XP055227885, US
ISSN: 1088-9051, DOI: 10.1101/gr.162339.113**
• **VIVIANA COBOS JIMÉNEZ ET AL:
"Next-generation sequencing of microRNAs in
primary human polarized macrophages",
GENOMICS DATA, vol. 2, 27 June 2014
(2014-06-27), pages 181-183, XP055450175, ISSN:
2213-5960, DOI: 10.1016/j.gdata.2014.06.019**
• **CHO ET AL.: 'Analysis of Off-Target Effects of
CRISPR/Cas-derived RNA-Guided
Endonucleases and Nickases' GENOME
RESEARCH vol. 24, January 2014, pages 132 -
141, XP055227885**
• **KUSCU ET AL.: 'Genome-Wide Analysis Reveals
Characteristics of Off-Target Sites Bound by the
Cas9 Endonuclease' NATURE BIOTECHNOLOGY
vol. 32, no. 7, July 2014, pages 677 - 683,
XP055382577**
• **PATTANAYAK ET AL.: 'High-Throughput
Profiling of Off-Target DNA Cleavage Reveals
RNA-Programmed Cas9 Nuclease Specificity'
NATURE BIOTECHNOLOGY vol. 31, no. 9, 2013,
pages 839 - 843, XP055294934**

**(Cont. next page)**

- **SHEN ET AL.: 'Efficient Genome Modification by CRISPR-Cas9 Nickase with Minimal Off-Target Effects' NATURE METHODS vol. 11, no. 4, April 2014, pages 399 - 402, XP055227888**
- **KIM ET AL.: 'Digenome-Seq: Genome-Wide Profiling of CRISPR-Cas9 Off-Target Effects in Human Cells' NATURE METHODS vol. 12, no. 3, March 2015, pages 237 - 243, XP055287797**

- **None**

**Description**

**[TECHNICAL FIELD]**

The present disclosure relates to a method for detecting off-target sites of a programmable nuclease in a genome, and specifically, to a method for detecting off-target sites through data analysis comprising cleaving genome by treating the genome (cell-free genomic DNA) isolated *in vitro* with programmable nucleases, and then performing whole genome sequencing, and to a method for selecting on-target sites of a programmable nucleases, which minimizes the off-target effect, using this method.

**[BACKGROUND ART]**

Programmable nucleases such as ZFNs (zinc finger nucleases), TALENs (transcriptional activator-like effector nucleases), and RGENs (RNA-guided engineered nucleases) derived from the type II CRISPR/Cas (clustered regularly interspaced repeat/CRISPR-associated) prokaryotic adaptive immunity system, etc. are widely used for genome editing in cultured cells and whole organisms. The genome editing technology using programmable nucleases is very useful technology that can be used for various purposes in life science, biotechnology, and medicine fields. For example, gene/cell therapy for diverse genetic or acquired diseases has become possible by causing targeted genetic modifications in stem cells or somatic cells. However, the programmable nucleases can mutate not only on-target sites but also off-target sites that are homologous thereto (Nucleic acids research, 2013, 41 (20): 9584- 9592).

As a representative example, RGENs, which comprise the Cas9 protein derived from S. pyogenes and small guide RNA (sgRNA) recognize 23-bp (base pair) target DNA sequences composed of a 20-bp (base pair) sequence that hybridizes with the sgRNA and a 5'-NGG-3' protospacer-adjacent motif (PAM) sequence recognized by Cas9, but can tolerate mismatches at up to several nucleotide sequences (Genome Res, 2014, 24: 132-141). Furthermore, RGENs can also cleave off-target DNA sequences harboring an extra base sequence (DNA bulge) or lacking a base (RNA bulge) compared to the sgRNA sequences. Likewise, both ZFNs and TALENs can also cleave sequences that differ in some bases. This suggests that there might be vast numbers of off-target sites in addition to on-target sites in case where programmable nucleases are applied to a genome.

Off-target DNA cleavages can lead to mutations at unintended gene such as proto-oncogenes and tumor suppressor genes, as well as gross genome recombination such as translocations, deletions, and inversions, and raise serious concerns about the use of programmable nucleases in research and medicine (Proc Natl Acad Sci, 2009, 106: 10620-10625). In this regard, various strategies have been reported to reduce off-target effects of programmable nucleases, the programmable nucleases specifically working at on-target sites without off-target effects in the entire genomic scale have not yet been reported. To address this issue, it is imperative to develop methods to interrogate the specificities of programmable nucleases on a genomic scale. WO 2013/169398 A2 discloses systems and methods for identifying the off-site cleavage loci and predicting the activity of engineered endonucleases for a given genome. Cradick, T.J., Fine, E.J., Antico, C.J. and Bao, G., 2013. CRISPR/Cas9 systems targeting β-globin and CCR5 genes have substantial off-target activity. Nucleic acids research, 41(20), pp.9584-9592, discloses that CRISPR/Cas9 systems targeting the human hemoglobin β and C-C chemokine receptor type 5 genes have substantial off-target cleavage, especially within the hemoglobin δ and C-C chemokine receptor type 2 genes, respectively, causing gross chromosomal deletions.

**[DISCLOSURE]**

**[TECHNICAL PROBLEM]**

As a result that the present inventors did their best to develop a system capable of detecting and analyzing the target and off-target sites of programmable nucleases on a genomic scale, it has been developed to complete the present invention that a method for detecting off-target sites of programmable nucleases by performing next generation sequencing (NGS) after cleaving a genome with a programmable nuclease (Digenome-seq, nuclease-cleaved genomic DNA sequencing).

**[TECHNICAL SOLUTION]**

It is an object of the present disclosure to provide a method for detecting an off-target sites of a programmable nuclease, comprising: (a) cleaving an isolated genomic DNA with a target-specific programmable nuclease; (b) performing next generation sequencing of the cleaved DNA; and (c) determining a cleaved site in a sequence read obtained by the sequencing. The embodiment of the present invention is reflected in independent claim 1. The preferred embodiments of the present invention are reflected in dependent claims 2 to 12.

It is another object of the present disclosure to provide a method for reducing off-target effects in genome editing, comprising: introducing *in vitro* transcribed guide RNA into a cell using a plasmid as a template.

**[EFFECT]**

Digenome-seq of the present disclosure can detect off-target sites of a programmable nuclease on a genomic scale with high reproducibility, and thus can be used for the production and study of programmable nucleases with high target specificity.

**[DESCRIPTION OF DRAWINGS]**

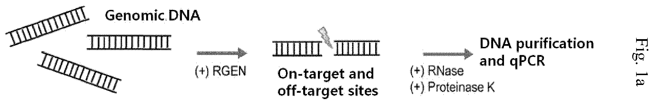

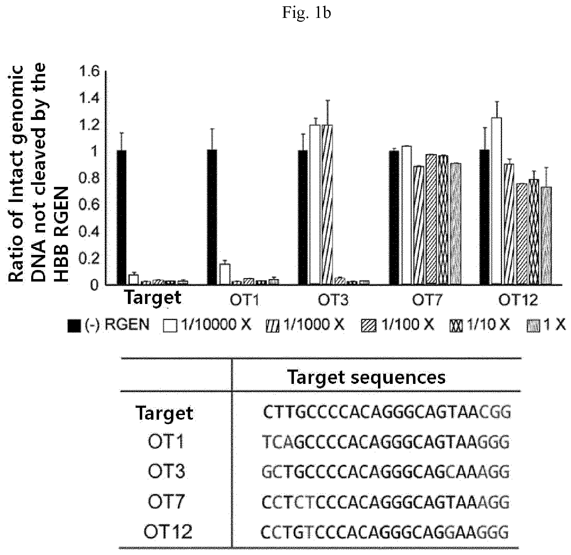

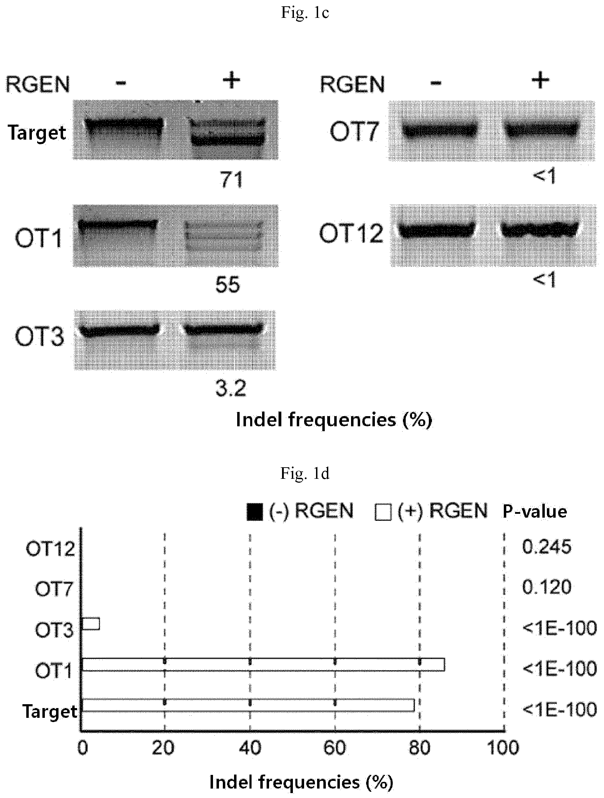

Fig. 1 relates to an RGEN-mediated genomic DNA cleavage *in vitro*. (a) It is a mimetic diagram of RGEN-mediated genomic DNA cleavage *in vitro*. (b) It identifies whether genomic DNA is cleaved by the HBB-targeting RGEN at on-target and four potential off-target sites. For the IX reaction, Cas9 protein (40 $\mu$g, 300 nM) and sgRNA (30 $\mu$g, 900 nM) were reacted with 8 $\mu$g of HAP1 genomic DNA for 8 hours. Cas9 and sgRNA were serially diluted by 10-fold to 10,000-fold. The uncleaved DNA was measured by qPCR. (Bottom) It illustrates DNA sequences of the on-target and the four potential off-target sites. Mismatched nucleotides are shown in red and the PAM sequence is shown in blue. (c) It measures the mutation frequencies by RGEN with the T7E1 assay at the on-target and potential off-target sites. (d) It performs targeted deep sequencing to measure indel frequencies.

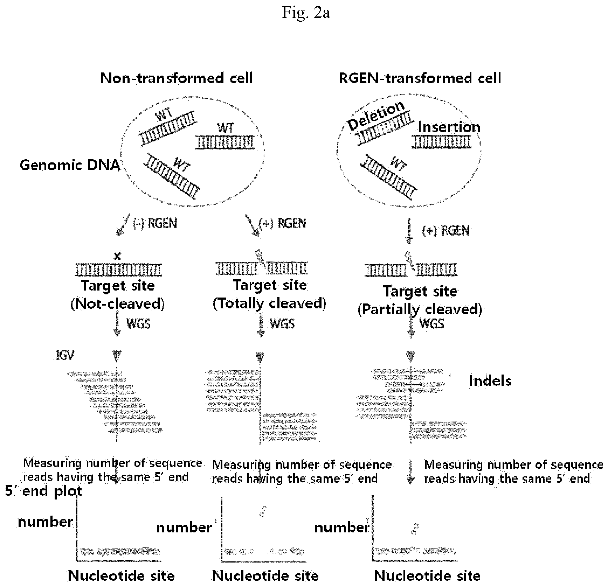

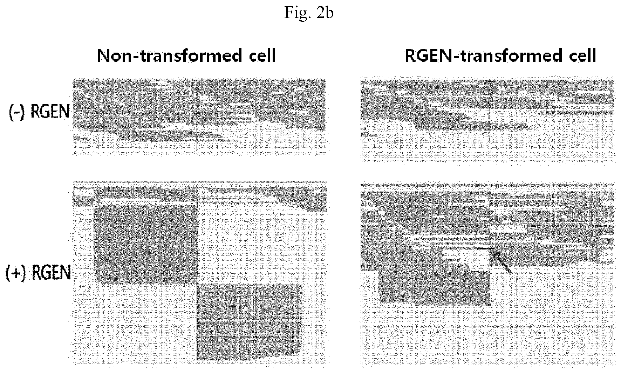

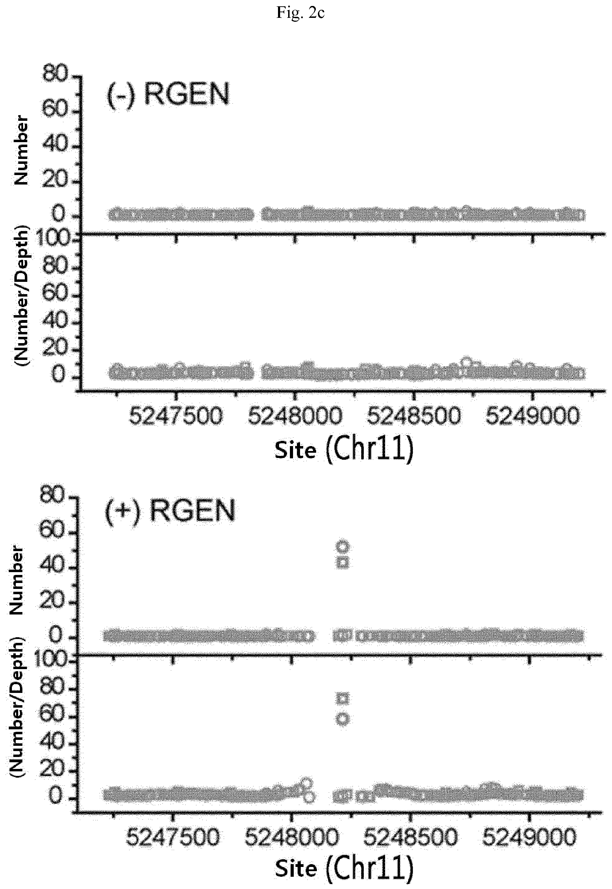

Fig. 2 relates to an RGEN-induced Digenome-seq to identify off-target sites. (a) It is a mimetic diagram of nuclease-cleaved whole genome sequencing (WGS) for the identification of off-target sites. Genomic DNA isolated from non-transfomed or RGEN-transfomed cells is cleaved by the RGEN, and subjected to WGS. Sequence reads are aligned to the reference genome (hg19) and visualized using the IGV program. Forward and reverse sequence reads are shown in orange and sky-blue, respectively. Red triangles and vertical dotted lines indicate cleavage positions. (b) It is the representative IGV data obtained using the HBB-specific RGEN at the on-target site. An indel is indicated by an arrow. (c) It shows the absolute and relative number of sequence reads with the same 5' end according to nucleotide positions.

Fig. 3 relates to an RGEN-induced Digenome-seq to identify off-target sites. (a-d) It is the representative IGV data obtained using the HBB-specific RGEN at the potential off-target sites OT1 (a), OT3 (b), OT7 (c), and OT12 (d). An indel is indicated by an arrow (a) or shown in a box (b).

Fig. 4 illustrates a plot of the number of 5' ends at a particular location on a genome. (a) It shows IGV data at a nuclease cleavage site. (b, c) It illustrates 5' end plots showing the absolute and relative number of sequence reads with the same 5' end according to nucleotide positions at the OT1 (b) and OT3 (c) sites.

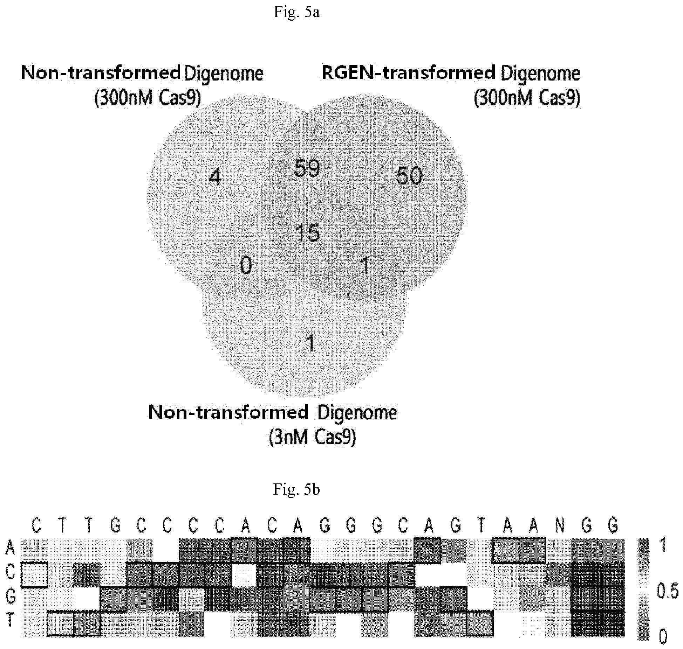

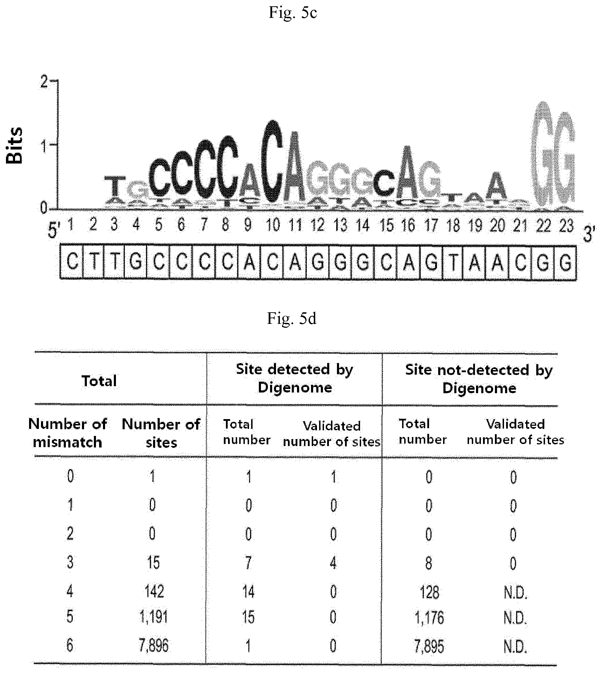

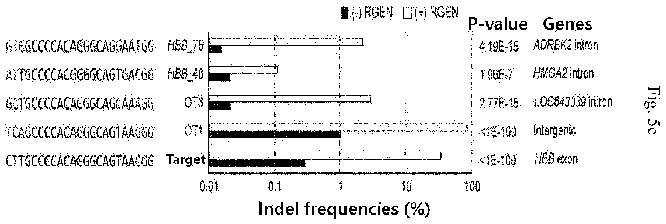

Fig. 5 illustrates off-target sites of the HBB RGEN identified by Digenome-Seq and validated by targeted deep sequencing. (a) It is a Venn diagram showing the number of on-target sites and off-target sites identified by Digenome-seq using the HBB RGEN in non-transformed or RGEN-transformed cells. (b) It illustrates a heatmap comparing sites identified by Digenome-seq with the on-target site. (c) It illustrates a sequence logo obtained by WebLogo using DNA sequences at sites identified by Digenome-seq. (d) It is a summary of the results of Digenome-seq and targeted deep sequencing. N.D. means that nothing is determined. (e) It illustrates off-target sites validated by targeted deep sequencing. Blue and red bars represent indel frequencies obtained using non-transformed HAP1 cells and the HBB RGEN-transformed HAP1 cells. (Left) It illustrates DNA sequences of on-target and off-target sites. Mismatched bases are shown in red, and the PAM sequences are shown in blue. (Right) P value was calculated by the Fisher exact test.

Fig. 6 illustrates false positive positions identified in the intact genome sequence. (a-c) It is the representative IGV data around false positive sites that resulted from naturally occurring indels in HAP1 cells.

Fig. 7 illustrates indel sequences induced by the HBB RGEN at newly validated off-target sites. (a, b) Off-target indels were detected by targeted deep sequencing. Inserted nucleotides are shown in red and the PAM sequence is shown in blue.

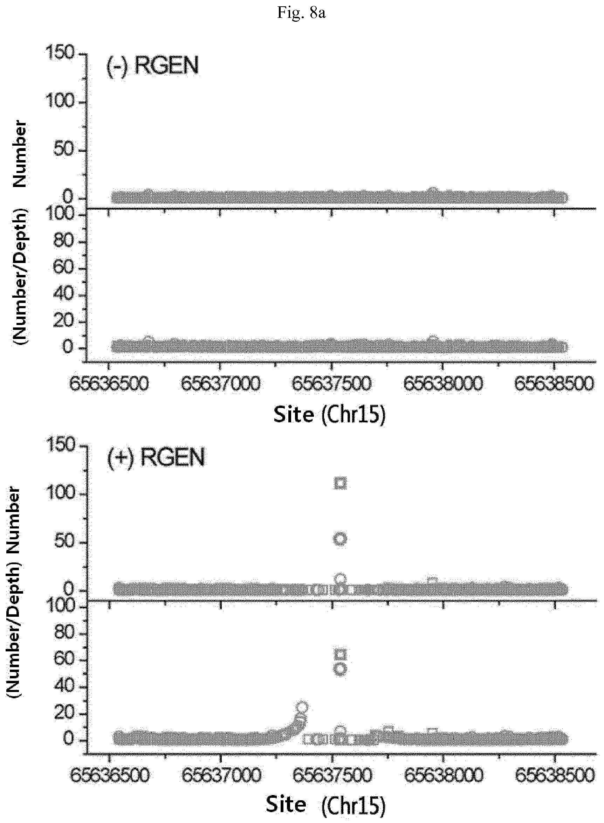

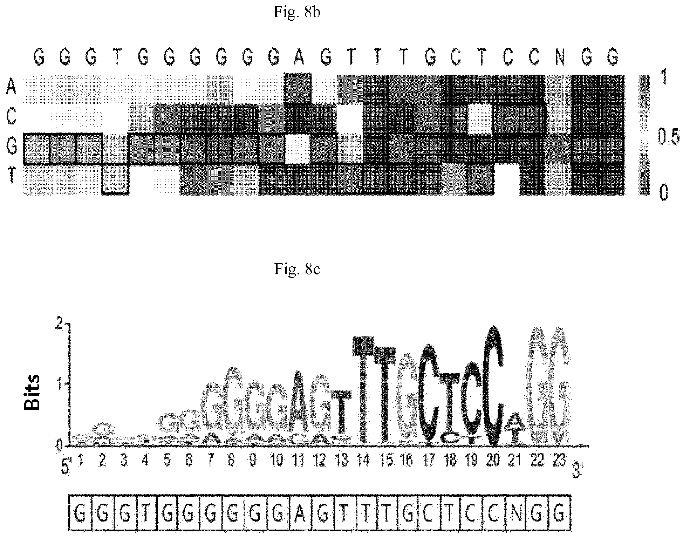

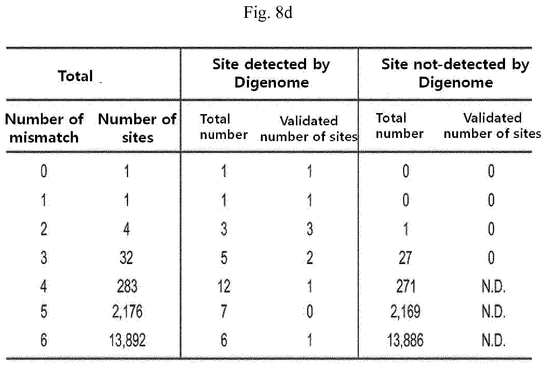

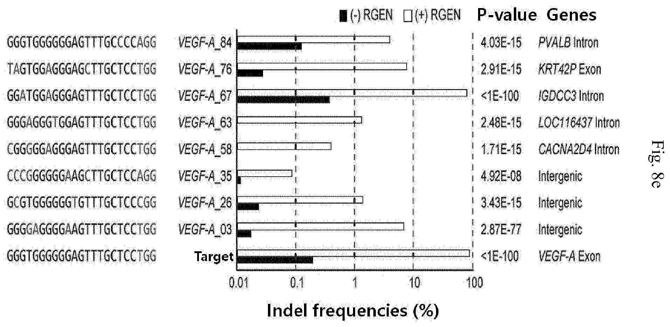

Fig. 8 illustrates off-target sites of the VEGF-A RGEN identified by Digenome-seq. (a) It illustrates a plot of the number of 5' ends at one of the VEGF-A off-target sites. (b) It is a heatmap comparing the site identified by Digenome-seq with the on-target site. Dark red and dark blue correspond to 100% and 0% match at a given position. (c) It illustrates sequence logo obtained by WebLogo using DNA sequences at the site identified by Digenome-seq. (d) It is a summary of the result of Digenome-seq and targeted deep sequencing. N.D. means that nothing is determined. (e) It illustrates off-target sites validated by targeted deep sequencing. Blue and red bars represent indel frequencies obtained using non-transformed HAP1 cells and the VEGF-A RGEN-transformed HAP1 cells. (Left) It illustrates DNA sequences of on-target and off-target sites. Mismatched bases are shown in red, and the PAM sequence is shown in blue. (Right) P value was calculated by the Fisher exact test.

Fig. 9 illustrates an RGEN-induced Digenome-seq to identify off-target sites of the VEGF-A RGEN. (a-d) It illustrates 5' end plots showing the absolute and relative number of sequence reads with the same 5' end according to nucleotide positions in on-target (a) and off-target sites (b-d).

Fig. 10 illustrates indel sequences induced by the VEGF-A RGEN at newly validated off-target sites. (a-d) Off-target indels were detected by targeted deep sequencing. Inserted nucleotides are shown in red and the PAM sequence is shown in blue.

Fig. 11 illustrates an *in vitro* DNA cleavage scoring system for Digenome-seq analysis.

Fig. 12 illustrates an improved Digenome-seq analysis. (a) It illustrates genomic scale Circos plot of *in vitro* DNA cleavage score. Whole genome sequencing (WGS) was performed using human genomic DNA (red) and genomic DNA (green) cleaved with RGEN. (b) It illustrates a mimetic diagram of Digenome-seq using oligonucleotide double strand or sgRNA transcribed from a plasmid. (C) It illustrates a sequence logo obtained using an oligonucleotide double strand or sgRNA transcribed from a plasmid.

Fig. 13 illustrates the reproducibility of the *in vitro* DNA cleavage scoring system.

Fig. 14 illustrates a bulge-type off-target site identified by Digenome-seq using sgRNA transcribed from an oligonucleotide double strand.

Fig. 15 illustrates a multiplex Digenome-seq. (a) It illustrates a mimetic diagram of a multiplex Digenome-seq. (b) It illustrates a Venn diagram showing the number of *in vitro* cleavage sites identified by single and multiplex Digenome-seq analyses. (c) It illustrates an *in vitro* DNA cleavage score on the X-chromosome obtained by single or multiplex Digenome-seq.

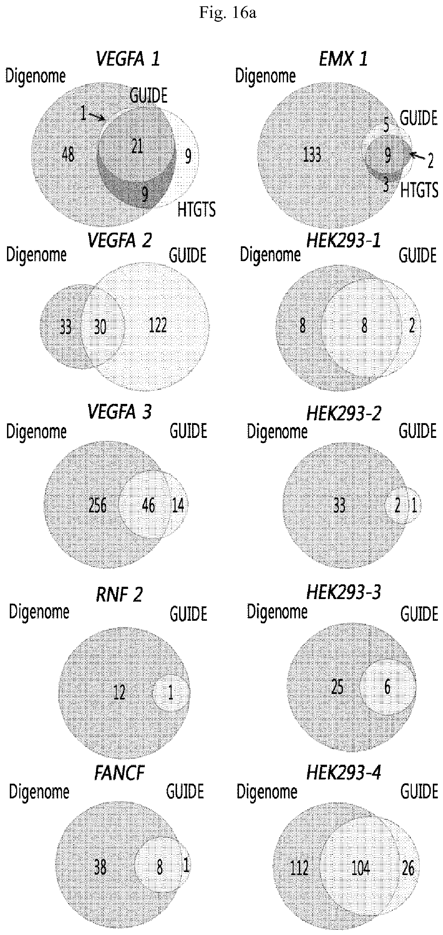

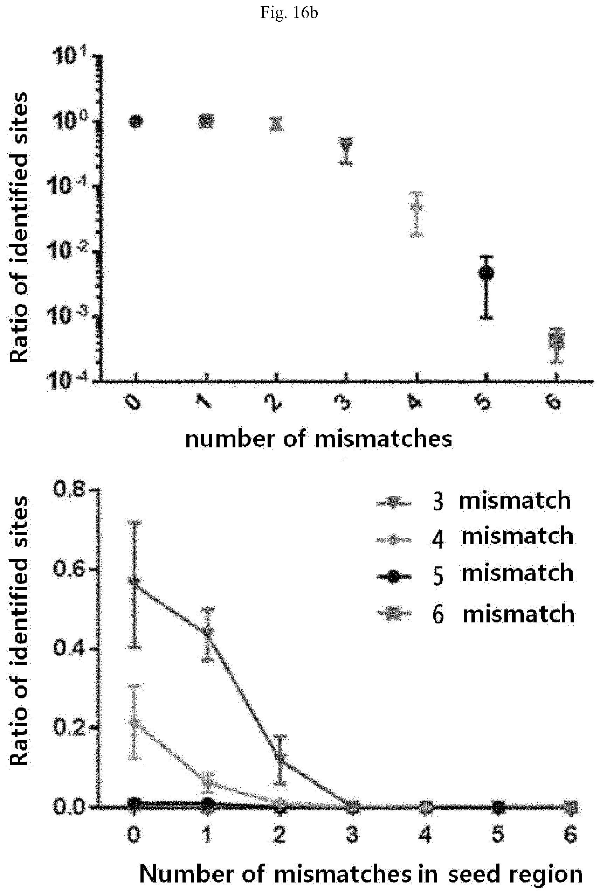

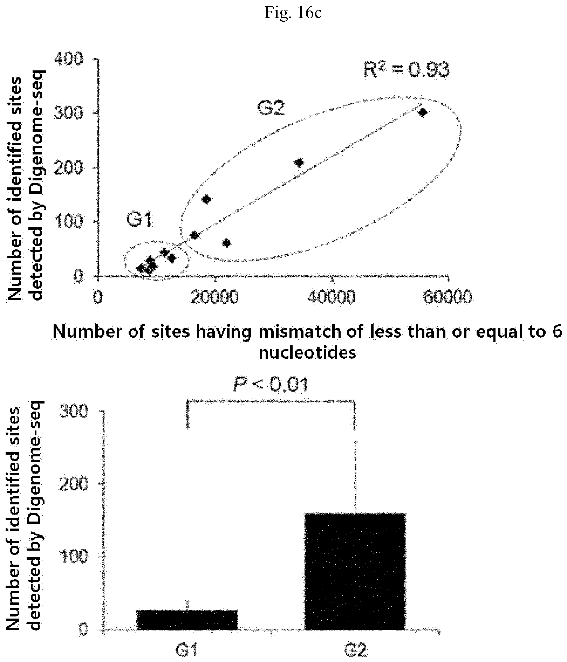

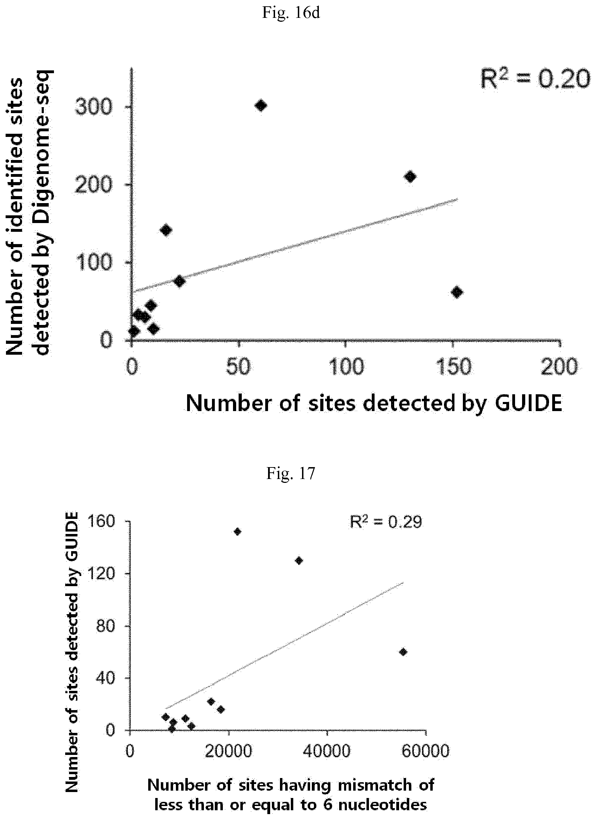

Fig. 16 illustrates an analysis of the sites identified by the multiplex Digenome-seq. (a) The number of sites identified by Digenome-seq, GUIDE-seq, and HTGTS is shown in a Venn diagram. (b) It illustrates the percentage of sites identified by Digenome-seq according to the total number of mismatches (top) and the number of mismatches in a seed region (bottom). (c) The number of sites with mismatches less than or equal to 6 nucleotides in the human genome and the number of sites identified by Digenome-seq are shown by a scatterplot (top). 11 RGEN on-target sites were divided into two groups of G1 (less than 13,000 sites with a mismatch of less than or equal to 6 nucleotides in the human genome) and G2 (greater than or equal to 16,000 sites with a mismatch of less than or equal to 6 nucleotides in the human genome) (bottom). The error bar represents the SEM. P values were calculated by Student's t-test. (d) The number of sites identified by GUIDE-seq and the number of sites identified by Digenome-seq are shown by a scatterplot.

Fig. 17 illustrates the lack of correlation between the number of GUIDE-seq positive sites and the number of homologous sites with a mismatch of less than or equal to 6 nucleotides in the human genome.

Fig. 18 illustrates two EMX1 off-target sites that are identified by HTGTS and GUIDE-seq but are not identified by Digenome-seq.

Fig. 19 illustrates the number of sites identified by Digenome-seq and CHIP-seq as a Venn diagram.

Fig. 20 illustrates the indel frequencies at on-target and off-target sites in RNF2-specific sgRNA-transformed HeLa cells in a log scale.

Fig. 21 identifies the indel frequencies using targeted deep sequencing at off-target sites. (a) It mimetically illustrates a general sgRNA ($gX_{19}$ sgRNA) and a modified sgRNA ($ggX_{20}$ sgRNA). (b-d) It illustrates the indel frequencies at on-target and off-target sites of (b) EMX1, (c) HEK293-3, and (d) RNF2 sgRNA validated by NGS. (e-g) It illustrates the specificity ratios calculated by dividing the indel frequencies at on-target sites of (e) EMX1, (f) HEK293-3, and (g) RNF2 sgRNA into the indel frequencies at off-target sites.

Fig. 22 illustrates an analysis of off-target sites that are validated by NGS and off-target sites that are not validated by NGS. (a-c) It illustrates a relative indel frequency (log scale) plot at off-target sites according to a mismatch shown in (a) the entire 20-nt sequence or (b and c) 10-nt seed sequence. The sites (a) identified by NGS were divided into two groups of a validated site (b) and invalidated site (c).

Fig. 23 illustrates the results of Digenome-seq performed on 100 on-target sites. (a) It mimetically illustrates a test process, and (b) it illustrates the results of comparing programs that predict off-target sites based on Digenome-seq with the other programs (Crop-it).

Fig. 24 illustrates the off-target effect of ZFN (zinc finger nuclease) through the Digenome-seq on a genomic scale. (a) It is the representative IGV photograph of on-target sites before and after ZFN-224 treatment. (b) It illustrates a Circos plot showing *in vitro* DNA cleavage score on a genomic scale of untreated genome DNA (red), DNA cleaved with ZFN-224 (WT FokI) (green), and DNA cleaved with ZFN-224 (KK/EL FokI) (blue) (c-d) It illustrates a sequence logo obtained using off-target candidate sites in ZFN-224 (WT FokI) or ZFN-224 (KK/EL FokI).

Fig. 25 illustrates the results of detecting off-target sites in Digenome-seq of ZFN. (a) Indel frequencies were measured by using targeted deep sequencing at off-target candidate sites of ZFN-224 (KK/EL FokI). (b-c) It is a Venn diagram showing Digenome-seq, ILDV, and the numbers of (b) off-target candidate sites detected *in vitro* and (c) validated on-target sites.

**[BEST MODE]**

According to one aspect in order to achieve this object of the present disclosure, there is provided a method for detecting off-target sites in a genome comprising: (a) cleaving an isolated genomic DNA with a target-specific programmable nuclease; (b) performing next generation sequencing of the cleaved DNA; and (c) determining a cleaved site in a sequence read obtained by the sequencing. The present inventors named said method "Digenome-seq," which refers to nuclease-cleaved genomic DNA sequencing.

Genome editing / gene editing technology are the technologies that can introduce a target-directed mutation into the genomic base sequence of animal and plant cells including human cells. It can knock-out or knock-in specific genes, or can introduce a mutation into non-coding DNA sequences that do not produce proteins. The method of the present disclosure detects the off-target site of programmable nucleases used in this genome editing / gene editing technology, which can be usefully used to develop programmable nucleases that specifically work only at on-target sites.

The step (a) is a step of cleaving the isolated genomic DNA with a target-specific programmable nuclease, that is, a step of cleaving the isolated genomic DNA *in vitro* with the programmable nucleases specifically working at on-target sites. However, even if the programmable nucleases are produced specifically for the target, other sites, that is, off-target sites, can also be cleaved depending on the specificity. Accordingly, as a result, by the step (a), the used target specific programmable nucleases cleaves a on-target site position which may has an activity with respect to the genomic DNA and a plurality of off-target sites, thereby obtaining genomic DNA whose specific site is cleaved. The type of the genomic DNA is not particularly limited, and may be a genomic DNA of a wild-type cell or a transformed cell. In addition, the transformed cell may be transformed to express specific programmable nucleases depending on the purpose of Digenome-seq.

The term "programmable nuclease" used in the present disclosure refers to all forms of nuclease that is capable of recognizing and cleaving a specific site on a desired genome. In particular, it may include, but is not limited to, a transcription activator-like effector nuclease (TALEN) fused with a transcription activator-like effector (TAL) domain derived from a plant pathogenic gene, which is a domain recognizing a specific target sequence on a genome, and a cleavage domain, zinc-finger nuclease, meganuclease, RGEN (RNA-guided engineered nuclease) derived from CRISPR, which is a microbial immune system, Cpfl, Ago homolog (DNA-guided endonuclease), etc.

The programmable nucleases recognize specific base sequences in the genome of animal and plant cells, including human cells, to cause double strand breaks (DSBs). The double strand breaks include both the blunt end or the cohesive end by cleaving the double strands of DNA. DSBs are efficiently repaired by homologous recombination or non-homologous end-joining (NHEJ) mechanisms within the cell, which allows researchers to introduce desired mutations into on-target sites during this process. The programmable nucleases may be artificial or manipulated non-naturally occurring.

The term "on-target site" used in the present disclosure means a site to which a mutation is to be introduced by using programmable nucleases, and may be selected arbitrarily depending on the purpose thereof. It may be a non-coding DNA sequence that can be present within a specific gene and does not produce a protein.

The programmable nucleases have sequence specificity, and thus work at an on-target site, but may work at an off-target site depending on the target sequence. The term "off-target site" used in the present disclosure refers to a site where the programmable nucleases have activity at a site having a sequence that is not identical to the target sequence of the programmable nucleases. That is, it refers to a site other than an on-target site that is cleaved by the programmable nucleases. In particular, the off-target site in the present disclosure includes not only the actual off-target site for a specific programmable nuclease but also the site where it is likely to become an off-target site. The off-target site may be, but is not limited to, a site cleaved by programmable nucleases *in vitro.*

The fact that programmable nucleases have activity even at sites other than on-target sites may be due to a phenomenon that can be caused by various causes. However, in particular, in the case of off-target sequences with high sequence homology to on-target sites having a target sequence designed for the on-target site and a nucleotide mismatch, there is a possibility that the programmable nucleases would work. The off-target site may be, but is not limited to, a site with a target sequence and one or more nucleotide mismatches.

It can lead to mutations of unintended gene in a genome, and raises serious concerns about the use of the programmable nucleases. In this regard, the process of accurately detecting and analyzing off-target sites as well as the activity at on-target sites of gene programmable nucleases may also be very important, and can be usefully used for developing programmable nucleases that specifically work only at on-target sites without off-target effects.

The programmable nucleases may be selected from the group consisting of meganuclease, ZFN (zinc finger nuclease), TALEN (transcription activator-like effector nuclease), RGEN (RNA-guided engineered nuclease), Cpfl, and Ago homolog. It may be included, but is not limited to, in the scope of the present disclosure as long as it recognizes a specific sequence of a target gene and has a nucleotide-cleaving activity and can cause insertion and deletion (indels) in a target gene.

The meganuclease may be, but is not limited to, a naturally-occurring meganuclease, which recognizes 15 to 40 base pair cleavage sites, which are usually classified into four families: LAGLIDADG family, the GIY-YIG family, His-Cyst box family, and HNH family. The exemplary meganuclease includes I-SceI, I-CeuI, PI-PspI, PI-SceI, I-SceIV, I-CsmI, I-PanI, I-SceII, I-PpoI, I-SceIII, I-CreI, I-TevI, I-TevII, and I-TevIII.

Site-specific genomic modifications have been promoted in plants, yeast, Drosophila, mammalian cells and mice using DNA binding domains derived from naturally-occurring meganuclease, mainly from LAGLIDADG family. This approach is based on the modification of the homologous gene in which the meganuclease target sequence is conserved (Monet et al. (1999) Biochem. Biophysics Res. Common. 255: 88-93), and there was a limit to the modification of the pre-engineered genome into which the target sequence is introduced. Accordingly, there has been an attempt to engineer meganuclease to exhibit novel binding specificities at medically or biotechnologically relevant sites. In addition, the naturally-occurring or engineered DNA binding domain derived from meganuclease is operably linked to a cleavage domain derived from a heterologous nuclease (e.g., FokI).

The ZFN comprises a selected gene and a zinc-finger protein engineered to be bound to a cleavage domain or an on-target site of a cleavage half-domain. The ZFN may be an artificial restriction enzyme comprising a zinc-finger DNA binding domain and a DNA cleavage domain. Here, the zinc-finger DNA binding domain may be engineered to be bound to the selected sequence. For example, Beerli et al. (2002) Nature Biotechnol. 20: 135-141; Pabo et al. (2001) Ann. Rev. Biochem. 70: 313-340; Isalan et al., (2001) Nature Biotechnol. 19: 656-660; Segal et al. (2001) Curr. Opin. Biotechnol. 12: 632-637; Choo et al. (2000) Curr. Opin. Struct. Biol. 10: 411-416 may be included as reference material in the present specification. In comparison of naturally-occurring zinc finger proteins, the engineered zinc finger binding domains may have novel binding specificities. The engineering method includes, but is not limited to, a rational design and a selection of various types. The rational design includes the use of databases containing, for example, triple (or quadruple) nucleotide sequences, and individual zinc finger amino acid sequences, wherein each triple or quadruple nucleotide sequence is associated with one or more sequences of zinc fingers that bind to a particular triple or quadruple sequence.

The selection of target sequences and the design and construction of fusion proteins (and polynucleotide encoding thereon) are well known to those skilled in the art, and are described in detail in the full text of U.S. Patent Application Publication Nos. 2005/0064474 and 2006/0188987. The entire disclosure of said publications is included in the present specification as reference of the present disclosure. In addition, as disclosed in these references and other references in the pertinent art, zinc finger domains and/or multi-finger zinc finger proteins may be linked together by a linker comprising any suitable linker sequence, such as a linker of five or more amino acids in length. Examples of linker sequences of six or more amino acids in length are disclosed in U.S. Patent No. 6,479,626; 6,903,185; 7,153,949. The proteins explained herein may include any combination of suitable linkers between each zinc finger of the protein.

In addition, nuclease such as ZFN contains a nuclease active portion (cleavage domain, cleavage half-domain). As is well known, the cleavage domain may be heterologous to the DNA binding domain, such as, for example, a cleavage domain from a nuclease that is different from a zinc finger DNA binding domain. The heterologous cleavage domain may be obtained from any endonuclease or exonuclease. The exemplary endonuclease from which the cleavage domain may be derived include, but is not limited to, restriction endonuclease and meganuclease.

Similarly, a cleavage half-domain may be derived from any nuclease, or a portion thereof, that requires dimerization for cleavage activity, as indicated above. Where the fusion protein comprises a cleavage half-domain, generally two fusion proteins require cleavage. Alternatively, a single protein comprising two cleavage half-domains may be used. The two cleavage half-domains may be derived from the same endonuclease (or functional fragments thereof), or each cleavage half-domain may be derived from a different endonuclease (or functional fragments thereof). In addition, the on-target site of the two fusion proteins is located in such a way that the cleavage half-domains are spatially oriented to each other by the binding of the two fusion proteins and their respective on-target sites. Thus, it is preferable to arrange the cleavage half-domains to be able to form a functional cleavage domain by dimerization. Accordingly, in one embodiment, neighboring edges of the on-target site are isolated by 3 to 8 nucleotides or 14 to 18 nucleotides. However, nucleotides or nucleotide pairs of any integer may be interposed between two on-target sites (e.g., 2 to 50 nucleotide pairs or more). Generally, the cleavage site lies between on-target sites.

Restriction endonucleases (restriction enzymes) are present in many species, may be sequence-specifically bound to DNA (at an on-target site), and cleave DNA directly at or near a binding site. Some restriction enzymes (e.g., Type IIS) cleave DNA at sites removed from a recognition site and have separable binding and cleavable domains. For example, the Type IIS enzyme FokI catalyzes double strand breaks of DNA at 9 nucleotides from a recognition site on one strand and 13 nucleotides from a recognition site on the other one strand. Accordingly, in one embodiment, the fusion protein comprises a cleavage domain (or cleavage half-domain) from at least one Type IIS restriction enzyme and one or more zinc-finger binding domains (which may or may not be engineered).

The term "TALEN" used in the present disclosure refers to a nuclease capable of recognizing and cleaving a target region of DNA. TALEN refers to a fusion protein comprising a TALE domain and a nucleotide cleavage domain. In the present disclosure, the terms "TAL effector nuclease" and "TALEN" are interchangeable. TAL effectors are known as proteins that are secreted by their type III secretion system when Xanthomonas bacteria are infected with a variety of plant species. The protein may be combined with a promoter sequence in a host plant to activate the expression of a plant gene that aids bacterial infection. The protein recognizes plant DNA sequences through a central repetitive domain consisting of various numbers of amino acid repeats of 34 or fewer. Accordingly, TALE is expected to be a novel platform for tools in genome engineering. However, in order to construct a functional TALEN with genomic-editing activity, a few key parameters that have not been known thus far should be defined as follows. i) The minimum DNA-binding domain of TALE, ii) the length of the spacer between the two half-digits constituting one target region, and iii) the linker or fusion junction that links the FokI nuclease domain with dTALE.

The TALE domain of the present disclosure refers to a protein domain that binds nucleotides in a sequence-specific manner via one or more TALE-repeat modules. The TALE domain includes, but is not limited to, at least one TALE-repeat module, and more specifically, 1 to 30 TALE-repeat modules. In the present disclosure, the terms "TAL effector domain" and "TALE domain" are interchangeable. The TALE domain may include half of the TALE-repeat module. The entire contents disclosed in International Patent Publication No. WO/2012/093833 or U.S. Patent Application Publication No. 2013-0217131 in relation to this TALEN are included in the present specification as reference.

The term "RGEN" used in the present disclosure means a nuclease comprising a target DNA-specific guide RNA and Cas protein as a component.

In the present disclosure, the RGEN may be, but is not limited to, applied to a genomic DNA isolated *in vitro* in the form of a target DNA-specific guide RNA and an isolated Cas protein.

The guide RNA may be transcribed *in vitro,* and in particular, it may be, but is not limited to, transcribed from an oligonucleotide double strand or a plasmid template.

In the present disclosure, the term "Cas protein" is a major protein component of the CRISPR/Cas system, and is a protein capable of forming an activated endonuclease or nickase.

The Cas protein may form a complex with crRNA (CRISPR RNA) and tracrRNA (trans-activating crRNA) to exhibit its activity.

Cas protein or gene information may be obtained from the known database such as GenBank of National Center for Biotechnology Information (NCBI). Specifically, the Cas protein may be a Cas9 protein. In addition, the Cas protein may be a *Streptococcus* genus, more specifically, a Cas protein derived from *Streptococcus pyojens*, and more specifically, a Cas9 protein. In addition, the Cas protein may be a *Neisseria* genus, more specifically, a Cas protein derived from *Neisseria meningitidis,* and more specifically, a Cas9 protein. In addition, the Cas protein may be a *Pasteurella* genus, more specifically, a Cas protein derived from *Pasteurella multocida,* and more specifically, a Cas9 protein. In addition, the Cas protein may be a *Francisella* genus, more specifically, a Cas protein derived from *Francisella novicida*, and more specifically, a Cas9 protein. In addition, the Cas protein may be a *Campylobacter* genus, more specifically, a Cas protein derived from *Campylobacter jejuni,* and more specifically, a Cas9 protein. However, the present disclosure is not limited to the examples described above.

In addition, the Cas protein is used in the present disclosure as a concept including both native proteins as well as variants capable of acting as an endonuclease or nickase activated in cooperation with a guide RNA. The variant of the Cas9 protein may be a mutated form of Cas9 in which a catalytic aspartate residue is changed to any other amino acid. Specifically, the other amino acids may, but is not limited to, be alanine.

In the present disclosure, the Cas protein may be a recombinant protein.

When used in reference to, for example, a cell, nucleic acid, protein or vector, etc., the term "recombinant" refers to the introduction of a heterologous nucleic acid or protein or a modification of a native nucleic acid or protein, or a cell, a nucleic acid, a protein, or a vector modified by a cell derived from a modified cell. Thus, for example, the recombinant Cas protein may be made by reconstructing a sequence encoding the Cas protein using a human codon table.

The Cas protein or a nucleic acid encoding it may be a form that allows the Cas protein to work in the nucleus.

The isolated Cas protein may also be a form that is easy to be introduced into cells. For example, Cas proteins may be linked to cell penetration peptides or protein transduction domains. The protein transduction domain may be, but is not limited to, poly-arginine or a TAT protein derived from HIV. In addition to the above-described examples, various types of cell penetrating peptide or protein transduction domain are well known in the pertinent art, so that a person skilled in the art may, but is not limited to, apply various examples to the present disclosure.

In addition, the nucleic acid encoding the Cas protein may further include a nuclear localization signal (NLS) sequence. Accordingly, the expression cassette containing the nucleic acid encoding the Cas protein may, but is not limited thereto, include an NLS sequence in addition to a regulatory sequence such as a promoter sequence, etc. for expressing the Cas protein.

The Cas protein may be linked to a tag advantageous for isolation and/or purification. For example, a small peptide tag such as a His tag, a Flag tag, or an S tag, etc., or a Glutathione S-transferase (GST) tag or a Maltose binding protein (MBP) tag may be, but is not limited to, linked depending on the purpose.

The term "guide RNA" used in the present disclosure means a target DNA-specific RNA, which may be bound to a Cas protein and guides a Cas protein to a target DNA.

In the present disclosure, the guide RNA is a dual RNA comprising two RNAs, that is, a crRNA (CRISPR RNA) and a tracrRNA (trans-activating crRNA) as components; or a form comprising a first site comprising a sequence complementary to a sequence in the target DNA and a second site comprising a sequence interacting with a Cas protein, and more specifically, a single chain guide RNA (sgRNA), which is a form of fusion of the major portions of crRNA and tracrRNA.

The sgRNA may include a portion having a sequence complementary to the sequence in the target DNA (also referred to as a Spacer region, a target DNA recognition sequence, a base pairing region, etc.) and a hairpin structure for Cas protein binding. More specifically, it may include a portion having a sequence complementary to a sequence in the target DNA, a hairpin structure for Cas protein binding, and a terminator sequence. The structures described above may, but is not limited to, be sequentially present in the order of 5' to 3'.

Any type of guide RNA can also be used in the present disclosure if the guide RNA comprises a major portion of the crRNA and tracrRNA and a complementary portion of the target DNA.

The crRNA may be hybridized with the target DNA.

RGEN may be composed of Cas protein and dual RNA, or may, but is not limited to, be composed of Cas protein and sgRNA.

The guide RNA, specifically, the crRNA or sgRNA, may comprise a sequence complementary to a sequence in the target DNA, and may comprise one or more additional nucleotides at the upstream region of crRNA or sgRNA, specifically, the 5' end of crRNA of sgRNA or dual RNA. The additional nucleotide may be, but is not limited to, guanine (G).

For the purposes of the present disclosure, the RGEN may have nuclease activity *in vivo* and *in vitro.* Accordingly, it can be used to detect the off-target site of genomic *DNA in vitro,* and when it is applied *in vivo,* it can be expected to have activity even at the same site as the detected off-target site.

The genomic DNA may be isolated from a transformed cell so that a non-transfomed cell or a target specific programmable nuclease has a nuclease activity, and may be used without limitation of its origin depending on the purpose of detecting the off-target sites of programmable nucleases.

In the present disclosure, the term "Cpfl" is a programmable nuclease of a new CRISPR system which is distinct from the CRISPR/Cas system, and the role of Cpfl as a programmable nuclease has recently been reported (Cell, 2015, 163 (3): 759-71). The Cpfl is a programmable nuclease driven by a single RNA, does not require tracrRNA and is relatively small in size compared to Cas9. In addition, it uses a thymine-rich protospacer-adjacent motif (PAM) sequence and cleaves the double chain of DNA to form a cohesive end. The Cpfl may be, but is not limited to, derived from *CandidatusPaceibacter, Lachnospira genus, Butyrivibrio genus, Peregrinibacteria, Acidominococcus genus, Porphyromonas genus, Prevotella genus, Francisella genus, Candidatus methanoplasma,* or *Eubacterium genus.*

In a specific embodiment of the present disclosure, on-target sites and some off-target predicted sites are cleaved as a result that the HBB gene-targeted RGEN is treated with genomic DNA isolated *in vitro. In vivo,* indels (insertion and deletion) were induced at the site (Fig. 1). However, not all off-target predicted positions were cleaved.

The step (b) is a step of performing a next generation sequencing (NGS) using the DNA cleaved through the step (a). Unlike the indirect method of finding a sequence that has a homology with a sequence at on-target sites and predicting it to be off-target sites, it is performed to detect off-target sites that are substantially cleaved by a programmable nuclease on the entire genomic scale.

In the present disclosure, the term "whole genome sequencing" means a method of reading the genome by many multiples in 10 X, 20 X, and 40 X formats for whole genome sequencing by next generation sequencing. "Next generation sequencing" means a technology that sculpts the whole genome or targeted region of genome in a chip-based and PCR-based paired end format and performs sequencing at a super high speed based on chemical reaction (hybridization) of the fragment.

The step (c) is a step of determining a site where the DNA is cleaved in the sequence reading obtained by the next generation sequencing (NGS), and on-target sites and off-target sites of a programmable nuclease may be easily detected by analyzing the sequencing data. Determining a specific site at which the DNA is cleaved from the sequence read may be performed in a variety of approaches, and the present disclosure provides many reasonable methods for determining the site. However, this is merely an example included in the technical idea of the present disclosure, and the scope of the present disclosure is not limited by these methods.

For example, as an example for determining a cleavage site, when the sequence read obtained through the whole genome sequencing is aligned according to the site in a genome using an analysis program (for example, BWA/GATK or ISAAC), the site where 5' end is vertically aligned may mean the site at which DNA is cleaved. In other words, in the present disclosure, the term "vertical alignment" means an arrangement in which the 5' end of two or more sequence reads starts at the same site (nucleotide position) of the genome when the whole genome sequencing results are analyzed with a program such as BWA/GATK or ISAAC, for each of the neighboring Watson strand and Crick strand. This is shown because each of the DNA fragments that are cleaved by programmable nucleases and thus have the same 5' end is sequenced.

That is, when the programmable nucleases have nuclease activity at on-target sites and off-target sites and cleave said sites, if the sequence read is aligned, the common cleaved sites are vertically aligned because each of their sites start at the 5' end. However, the 5' end is not present in the uncleaved sites, so that it can be arranged in a staggered manner in alignment. Accordingly, the vertically aligned site may be regarded as a site cleaved by programmable nucleases, which means on-target sites or off-target sites of the programmable nucleases.

The alignment means mapping the sequence read to the reference genome and then aligning the bases having the same site in a genome to fit for each site. Accordingly, any computer program may be used as long as the sequence read can be arranged in the same manner as described above, which may be a known program already known in the pertinent art, or a program tailored to the purpose. In one embodiment of the present disclosure, alignment is performed using ISAAC, but is not limited thereto.

As a result of the alignment, the site at which the DNA is cleaved by programmable nucleases may be determined by a method such as finding a site where the 5' end is vertically aligned as described above, and the cleaved site may be determined as an off-target site if it is not an on-target site. In other words, the sequence that is identical to the base sequence designed with an on-target site of programmable nucleases is an on-target site, and the sequence that is not identical to the base sequence is regarded as a off-target site. This is obvious according to the definition of an off-target site described above. The off-target site may, in particular, be composed of a sequence having a homology to the sequence of an on-target site, specifically, include a sequence having an on-target site and one or more nucleotide mismatches, and more specifically, an on-target site and 1 to 6 nucleotide mismatches, but is not particularly limited thereto. It may be included in the scope of the present disclosure if it is the site that programmable nucleases can cleave. At this time, the on-target site may be a 15-30 nucleotide sequences complementary to a guide RNA, and may further include a sequence recognized by a nuclease (for example, a PAM sequence recognized by Cas9 in the case of Cas9).

In addition to a method of finding the site where the 5' end is vertically aligned, the off-target site may be determined as an off-target site if the site is not an on-target site when the dual peak pattern is seen in the 5' end plot. When a graph is drawn by counting the number of nucleotides constituting the 5' end of the same base at each site in a genome, a dual peak pattern appears at a specific site. It is because that the dual peak is indicated by each of the double stands cleaved by programmable nucleases.

In a specific embodiment of the present disclosure, the genomic DNA was cleaved into RGEN, and after the whole genome analysis, it was aligned with ISAAC, and the patterns aligned vertically at the cleavage site and the staggered pattern at the uncleaved site were identified. It was identified that a unique pattern of double peaks appears at the cleavage site when represented by a 5 'end plot (Figs. 2 to 4).

Moreover, it is not limited thereto, but as a specific example, the site where two or more sequence reads corresponding to Watson strand and Crick strand are aligned vertically may be determined as an off-target site. In addition, the site where 20% or more of sequence reads is vertically aligned and the number of sequence reads having the same 5' end in each of the Watson and Creek strands is 10 or more is determined as an off-target site position, that is, a cleavage site.

In a specific embodiment of the present disclosure, the site where the number of sequence reads having the same 5' end at both strands is 10 or more, and at least 19% of the sequence reads are vertically aligned was searched. As a result, it was identified that Digenome-seq has a high reproducibility by detecting 125 sites including on-target and off-target sites that had been previously validated (Figs. 5 to 7).

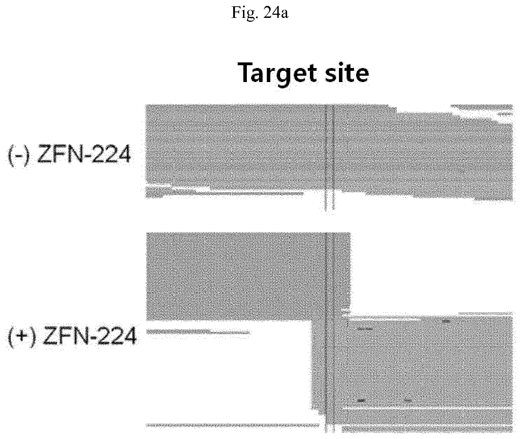

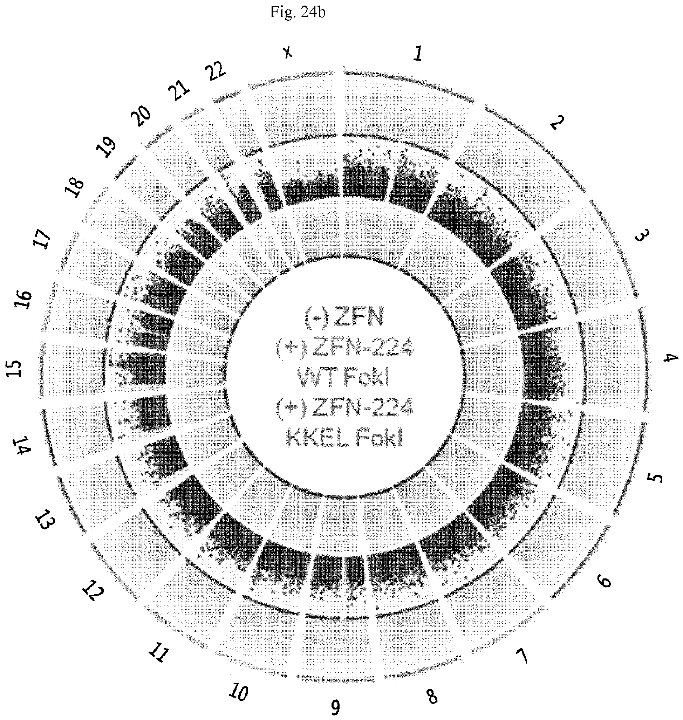

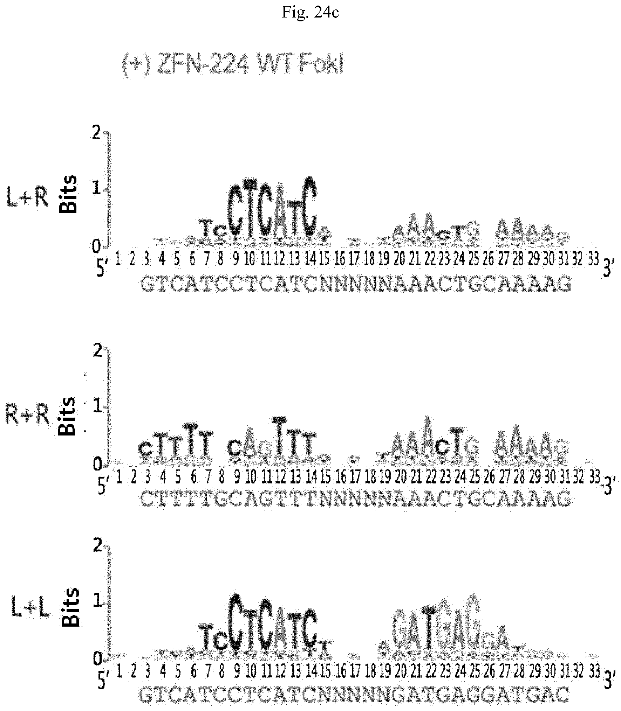

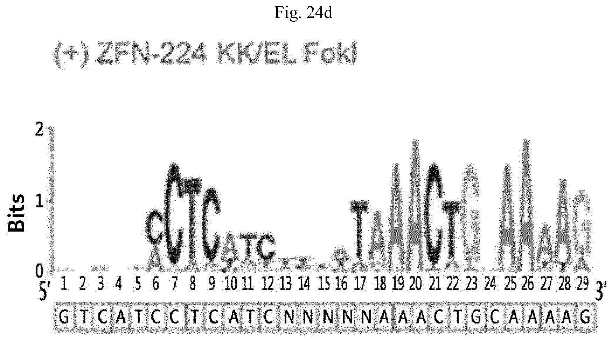

In another specific embodiment of the present disclosure, it was identified that off-target sites may be detected with Digenome-seq for another target gene, VEGF-A (Figs. 8 to 10). In another specific embodiment, it was identified that Digenome-seq may also detect off-target sites of ZFN other than RGEN (Fig. 24). In conclusion, it can be seen from these results that Digenome-seq of the present disclosure is a method for detecting off-target sites of programmable nucleases without being limited to the types of on-target sites and programmable nucleases.

The off-target site is performed *in vitro* by processing programmable nucleases in a genomic DNA. Thus, it can be identified whether off-target effects are actually produced also *in vivo* in the off-target site detected by this method. However, this is merely an additional verification process, and thus is not a step that is essentially accompanied by the scope of the present disclosure, and is merely a step that can be additionally performed according to the needs. In the present disclosure, the term "off-target effect" is a concept that is distinct from an off-target site. That is, as described above, in the present disclosure, the concept of an off-target site means a site other than the on-target sites among the sites where programmable nucleases can work, and is referenced as a site cleaved by nuclease. The off-target effect refers to an effect showing indels (insertion and deletion) by programmable nucleases at an off-target site in cells. In the present disclosure, the term "indel" is a generic term for a mutation in which some bases are inserted or deleted in the middle of a base sequence of DNA. In addition, the off-target site at which the indel caused by programmable nucleases is also referred to as an off-target indel site. In conclusion, the off-target site of the present disclosure is deemed as a concept of including an off-target indel site, and it is sufficient if it is a site where programmable nucleases have a possibility of having an activity, and indels do not necessarily have to be identified by programmable nucleases. Meanwhile, the off-target site in the present disclosure is referred to as a candidate off-target site, and the off-target indel site is also referred to as a validated off-target site.

Specifically, the verification process may include, but is not limited to, isolating genomic DNA from cells expressing the programmable nucleases for the off-target site, identifying indels at the off-target site of DNA, and identifying the off-target effect at the off-target site. The off-target effect may be identified by a method of analyzing a mutant detection using T7E1 analysis and Cel-I enzyme and identifying indels known in the pertinent art such as targeted deep sequencing. The step of identifying the off-target effect may be a direct confirmation on whether indels occur at an off-target site. However, even if indels do not occur during the *in vivo* verification process, it should be regarded as an auxiliary means because it does not identify the case that indels occur at a frequency below the detectable level.

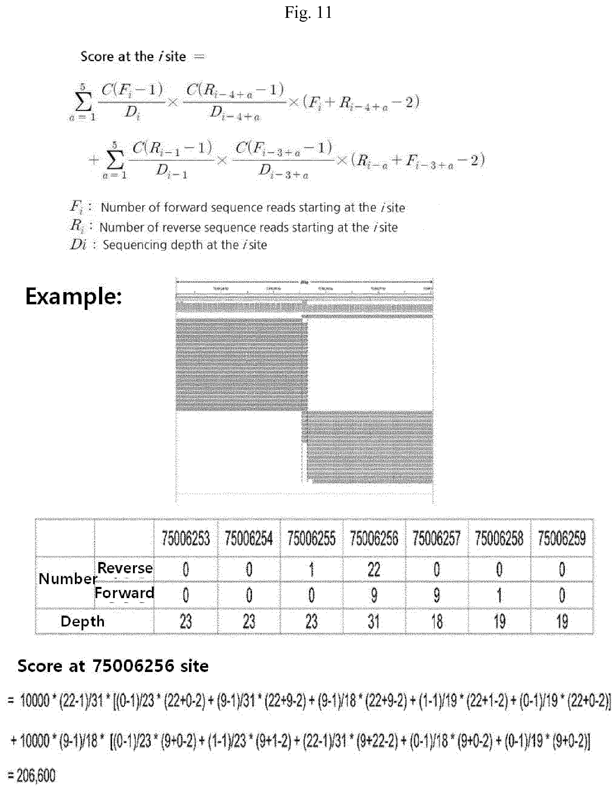

By identifying the vertically aligned site as described above, or by identifying the double peak in the 5' end plot, the off-target site may sufficiently be detected, which can be highly reproducible. However, there is a problem that some sites having a heterogeneous cleavage pattern or a low sequencing depth may be missing. Based on the alignment pattern of the sequence reads, the present inventors developed a formula for calculating the DNA cleavage score at each nucleotide site (Fig. 11) as follows:

$$\text{Score at the } i \text{ site} =$$

$$\sum_{a=1}^{5} \frac{C(F_i - 1)}{D_i} \times \frac{C(R_{i-4+a} - 1)}{D_{i-4+a}} \times (F_i + R_{i-4+a} - 2)$$

$$+ \sum_{a=1}^{5} \frac{C(R_{i-1} - 1)}{D_{i-1}} \times \frac{C(F_{i-3+a} - 1)}{D_{i-3+a}} \times (R_{i-a} + F_{i-3+a} - 2)$$

$F_i$ : Number of forward sequence reads starting at the $i$ site
$R_i$ : Number of reverse sequence reads starting at the $i$ site
$D_i$ : Sequencing depth at the /site
$C$ : Arbitrary constant

Through this formula, a plurality of additional sites that were not detected in the existing Digenome-seq could be detected, thereby allowing easy filtering of false-positive sites. The C value in this formula is not limited by the examples of the present disclosure, as a person skilled in the art can apply arbitrary constants. In particular, it is not limited thereto, but for example, when the C value is 100 and the calculated score is 25,000 or more, it may be determined as an off-target site. However, the criteria of the score may be appropriately adjusted or changed by a person skilled in the art depending on the purpose.

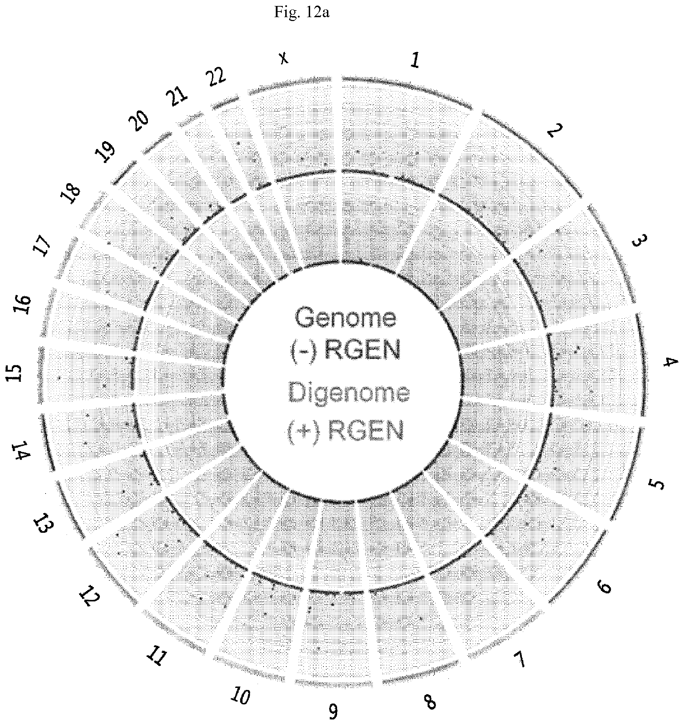

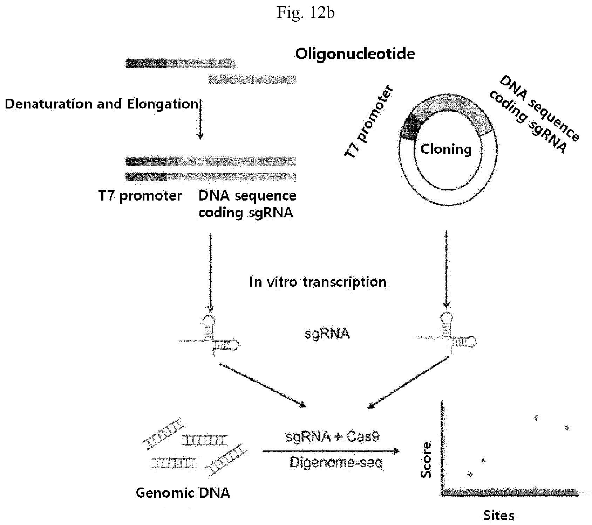

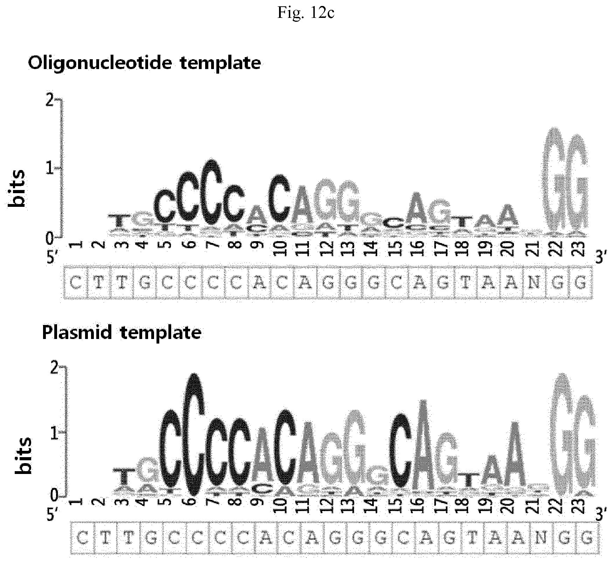

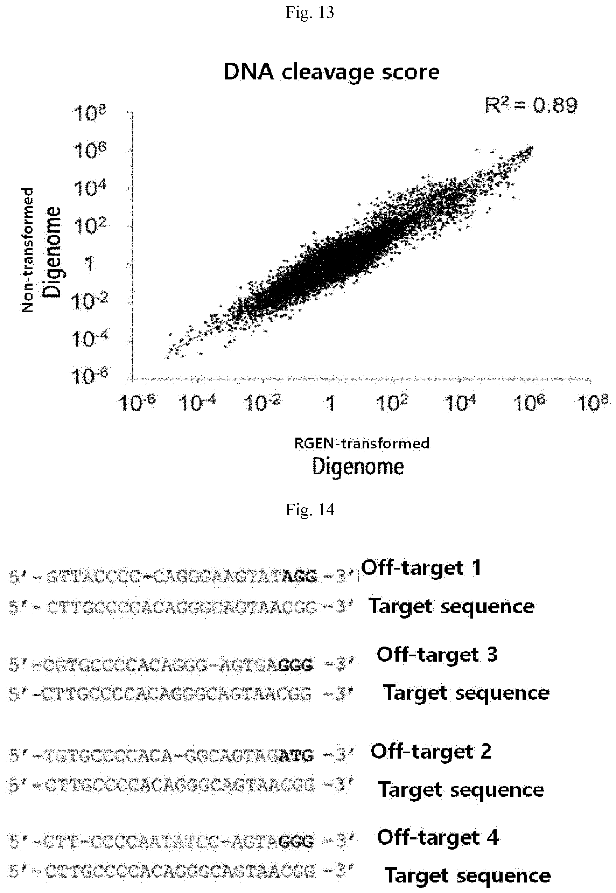

In a specific embodiment of the present disclosure, the off-target site was detected by introducing the DNA cleavage score into the existing Digenome-seq method. As a result, an additional position could be detected as compared with a method of merely finding a vertical alignment site, and it has a high reproducibility (Figs. 12 and 13). In another specific embodiment of the present disclosure, in the sgRNA of RGEN, the off-target site detected when the sgRNA transcribed from the plasmid template was used as compared with the one transcribed from a plasmid template and one transcribed from the oligonucleotide double strand has a high homology as compared with the on-target site (Fig. 14, Table 1 and Table 2).

Further, the Digenome-seq of the present disclosure may be performed using a plurality of programmable nucleases, and the present inventors have named this "multiplex digenome-seq". In this case, the programmable nucleases may be a mixture of programmable nucleases for 2 or more, specifically 2 to 100 targets, but is not limited thereto.

In the case of the multiplex Digenome-seq, it is important to check whether a cleavage site is cleaved by programmable nucleases because genomic DNA is cleaved by each of programmable nucleases. This can be achieved by classifying the off-target site according to the edit distance to the on-target site and is based on the assumption that the base sequence at the off-target site is homologous to the on-target site. This allows a clear distinction between on-target and off-target sites for each programmable nuclease.

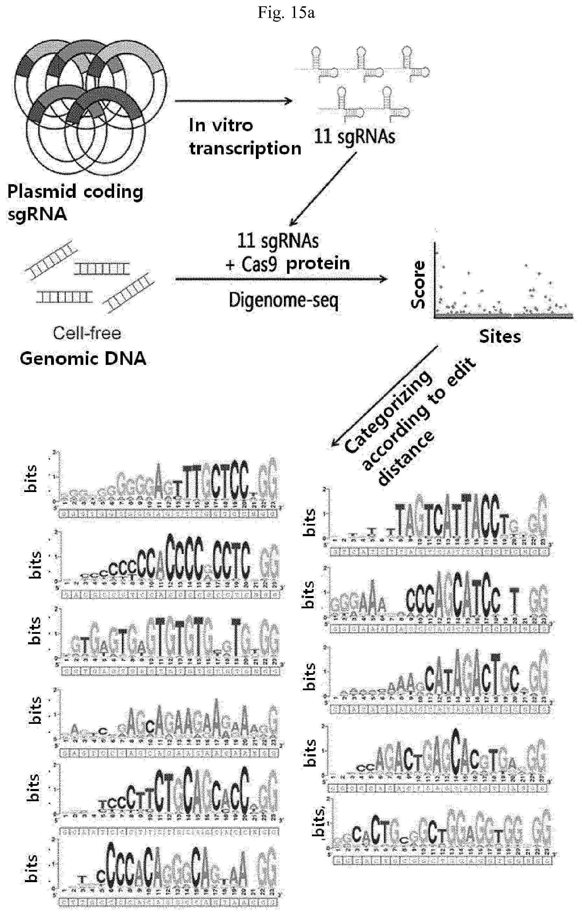

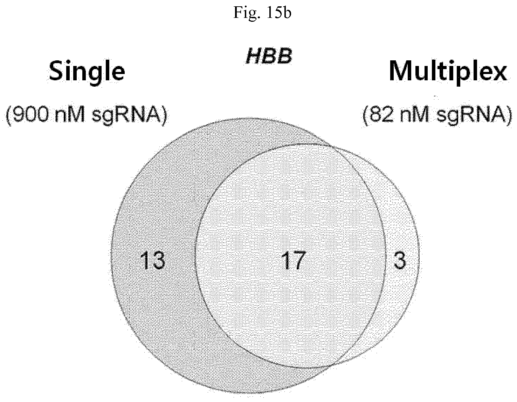

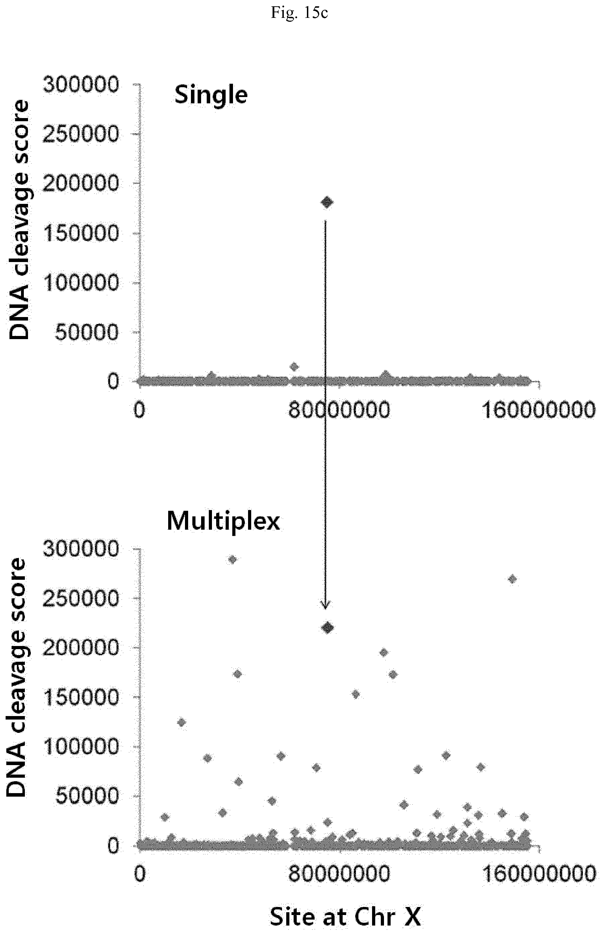

In a specific embodiment of the present disclosure, a multiplex Digenome-seq using sgRNA for 11 different on-target sites in Digenome-seq was performed, and 964 positions identified were classified according to edit distance with an on-target site to identify the off-target site for each on-target site (Figs. 15-19).

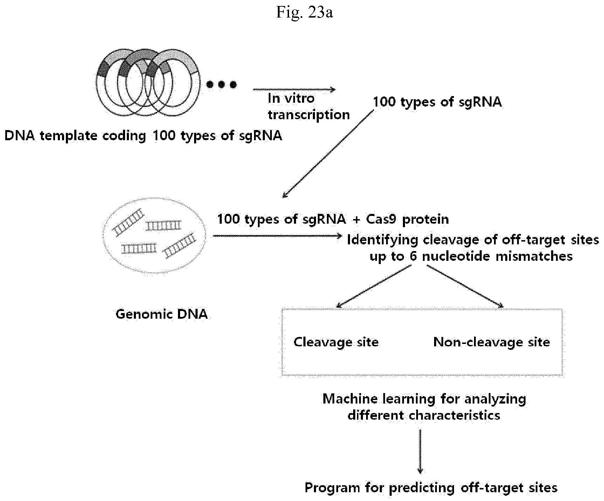

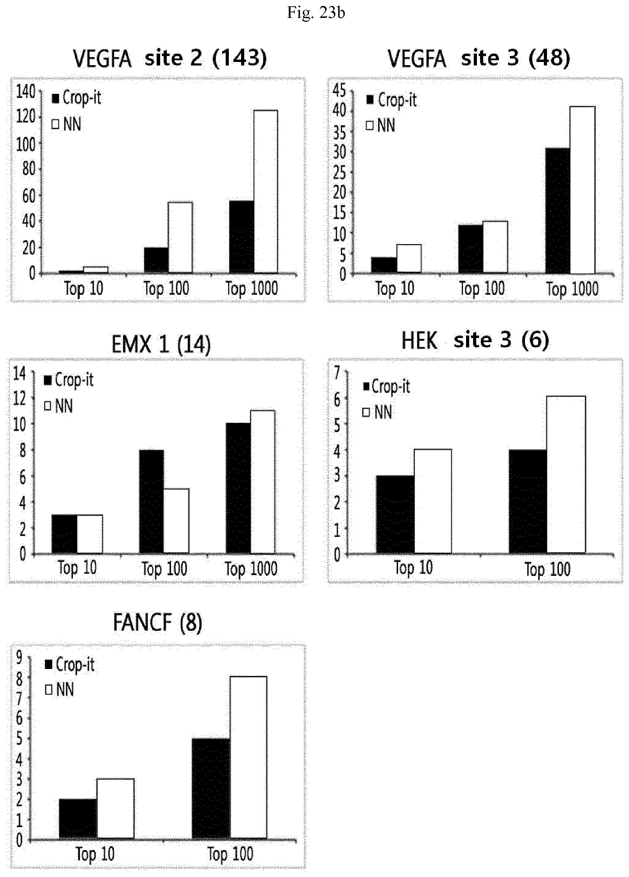

In another specific embodiment, a multiplex Digenome-seq was performed using sgRNA for 100 different on-target sites, and also in this case, off-target sites could be identified without particular limitation (Fig. 23). It was identified that the Digenome-seq of the present disclosure can be applied to any number of on-target sites without limitation.

In a specific embodiment of the present disclosure, for RNA-guided engineered nuclease (RGEN) targeting a specific site, among the off-target sites detected by Digenome-seq in the whole genome, when the homology site with a nucleotide mismatch to an on-target site of 6 or less is 13,000 or less and they do not have a homology site with a nucleotide mismatch of 2 or less, it was identified that the off-target effect can be minimized by selecting the specific site as the on-target site of the RGEN. This is an example showing a process of establishing a preferable criterion for selecting on-target sites using the Digenome-seq of the present disclosure, and it is expected that the off-target effect of programmable nucleases can be minimized through Digenome-seq.

In another specific embodiment of the present disclosure, it was identified that the number of sites having homology with the sequence at an on-target site was detected at a small rate by Digenome-seq as the nucleotide mismatch level increased (Fig. 16).

This is because the smaller the nucleotide sequence having homology in the target sequence and the genome in the selection of the on-target site of RGEN, the more specific the nucleotide sequence having a high homology. The on-target site of the selected RGEN through this may be that the of-target effect is minimized.

In another aspect, the present disclosure provides a method for reducing off-target effects in genome editing, comprising introducing *in vitro* transcribed guide RNA into cells having a plasmid as a template.

This off-target effect reduction is attributed to the prevention of indels at bulge-type off-target sites when the plasmid is used as a template. That is, when the guide RNA is prepared through *in vitro* transcription process, a large number of bulge-type off-target sites are detected when the oligonucleotide double strand is used as a template, but most of the bulge-type off-target sites disappear when the plasmid template is used. In addition to Digenome-seq, RGEN can be used to cleave genomic DNA and induce indels, which can use the plasmid as a template instead of an oligonucleotide double strand to reduce off-target effects. This is because oligonucleotides contain failed sequences, which are called (n-1)mer.

**[Best Mode]**

Hereinafter, the present disclosure will be described in detail with reference to examples. However, these examples of the present disclosure have been described herein for purposes of illustration only, and the scope of right of the present disclosure is not limited by these examples.

Example 1: Cas9 and *in vitro* sgRNA

Recombinant Cas9 protein was purified from E. coli or purchased from ToolGen (South Korea). sgRNAs were synthesized by *in vitro* transcription using T7 RNA polymerase. Specifically, sgRNA templates were mixed with T7 RNA polymerase in a reaction buffer (40 mM Tris-HCl, 6 mM $MgCl_2$, 10 mM DTT, 10 mM NaCl, 2 mM spermidine, NTP, and RNase inhibitor) at 37°C for 8 hours. Transcribed sgRNAs were purified using PCR purification kits (Macrogen) after being incubated with DNaseI to remove the template DNA.

Example 2: Cell culture and transformation conditions

HeLa cells were cultured in a DMEM medium containing 10% FBS. A Cas9 expression plasmid (500 ng) and a plasmid (500 ng) encoding sgRNA were introduced into $8 \times 10^4$ HeLa cells using lipofectamine 2000 (Life Technologies). After 48 hours, the genomic DNA was isolated with DNeasy Tissue kit (Qiagen) according to the manufacturer's instructions.

Example 3: *In vitro* cleavage of genomic DNA

Genomic DNA was purified from HAP1 cells using DNeasy Tissue kit (Qiagen). *In vitro* cleavage of the genomic DNA was performed for Digenome-seq. Specifically, Cas9 protein and sgRNA were incubated at room temperature for 10 minutes to form RNP (ribonucleoprotein). Next, the RNP complex and the genomic DNA were reacted in the reaction buffer (100 mM NaCl, 50 mM Tris-HCl, 10 mM $MgCl_2$, and 100 $\mu$g / ml BSA) for 8 hours at 37°C. The genomic DNA cleaved during this process to decompose sgRNA was treated with RNase A (50 ug / mL), and purified again with DNeasy Tissue kit (Qiagen).

Example 4: Whole genome sequencing and Digenome-seq (cleaved genome sequencing)

For whole genome sequencing (WGS), the cleaved DNA was disrupted with a sonicator and ligated with an adapter to make a library. WGS was performed on the Illumina HiSeq X Ten Sequencer from Macrogen (South Korea) using this library. Then, Isaac was used to align the sequence file for the human reference genome hg19. The cleavage scoring system was used to identify the DNA cleavage site.

For multiplex Digenome-seq, the detection site results were classified into 11 groups according to edit distance. The computer program used to detect the *in vitro* RGEN cleavage site and the computer program used for Digenome detection site classification were generated separately.

Example 5: Targeted deep sequencing

On-target sites and potential off-target sites were amplified using Phusion polymerase (New England biolabs). PCR amplification products were denatured with NaOH, paired-end sequencing was performed using Illumina MiSeq, and then the frequency of insertion and deletion (indels) was calculated.

Experimental Example 1: Cleavage of genomic DNA using RGEN *in vitro*

In order to develop a method for detecting off-target sites of programmable nucleases, the present inventors have conducted experiments using RGEN (RNA guided engineered nuclease) as a representative. However, this is only an example for explaining the technique of the present disclosure, and the kind of programmable nucleases that can be applied is not limited to RGEN. A method for detecting off-target sites of programmable nucleases in a genome of the present disclosure is characterized in that a genome is cleaved into programmable nucleases for a specific target *in vitro,* and then off-target sites of programmable nucleases was detected by performing and analyzing the whole genome sequencing (WGS). The present inventors named it Digenome-seq (nuclease-cleaved genomic DNA sequencing).

The present inventors reasoned that they could identify off-target mutations induced by programmable nucleases in a bulk population of cells by Digenome-seq.

It should be possible to cleave off-target DNA sequences efficiently at high RGEN concentration *in vitro,* producing many DNA fragments with identical 5' ends. These RGEN-cleaved DNA fragments would produce sequence reads that are vertically aligned at nuclease cleavage sites. In contrast, the sequence reads that were not cleaved by RGEN would be aligned in a staggered manner. A computer program was developed to search for sequence reads with vertical alignment that correspond to off-target sites.

First, the present inventors tested whether RGENs could cleave potential off-target DNA sequences efficiently in a genome *in vitro.* For this, a HBB gene-specific RGEN that had been shown to induce off-target mutations at an on-target site of RGEN and a highly homologous site (refereed to as OT1 site) was chosen. In addition to this site, three other potential off-target sites (referred to as OT3, OT7 and OT12 sites) that differed from the on-target site of the RGEN by three nucleotides were analyzed.

Genomic DHA isolated from wild-type HAP1 cells was cleaved using Cas9 protein pre-incubated with the HBB-specific sgRNA at concentrations that ranged from 0.03 nM to 300 nM (Fig. 1a). Then, quantitative PCR was used to measure DNA cleavage at these sites. Both the HBB on-target and OT1 sites were cleaved almost completely even at a very low RGEN concentration (Fig. 1b). By contrast, the OT3 site was cleaved completely only at high RGEN concentrations. The other two sites, OT7 and OT12, were cleaved poorly even at the highest concentration.

Next, this RGEN was transformed into HAP1 cells and used T7 endonuclease I (T7E1) and targeted deep sequencing were used to detect indels (insertion and deletion) induced at these sites.

For T7E1 assay, genomic DNA was isolated using DNeasy Tissue kit (Qiagen) according to the manufacturer's instructions. The on-target site was amplified by PCR. Next, amplified PCR products were denatured by heating and cooled slowly using a thermocycler. The cooled products were incubated with T7 endonuclease I (ToolGen) for 20 minutes at 37°C, and size-separated by agarose gel electrophoresis.

For targeted deep sequencing, genomic DNA segments spanning the on-target and off-target sites were amplified using Phusion polymerase (New England biolabs). The PCR amplicons were subjected to paired-end sequencing using Illumina MiSeq.

In interpreting the results, indels located 3-bp upstream of the PAM (protospacer-adjacent motif) were considered to be the mutations induced by RGENs. As expected, the HBB RGEN was highly active at both the HBB on-target and the OT1 off-target sites, producing indels at frequencies of 71% and 55% (T7E1), respectively (Fig. 1c). Off-target indels were also induced at the OT3 site with a frequency of 3.2% (T7E1) or 4.3% (deep sequencing) (Figs. 1c, d). Meanwhile, at the other two potential off-target sites that were poorly cleaved *in vitro,* no indels were detected using T7E1 (detection limit, ~1%) and deep sequencing (detection limit, ~0.1%). Note that the OT7 site had no nucleotide mismatches in the seed region (10- to 12-nt sequence upstream of the PAM) but was not cleaved either *in vitro* or in cells, identifying the importance of the PAM-distal region.

These results are consistent with our previous finding that RGENs can cleave off-target DNA sequences *in vitro* but often cannot induce indels at the same sties in cells. Accordingly, RGENs appear much more promiscuous *in vitro* than in cells in terms of target specialty. Perhaps, most DNA double strand breaks (DSBs) generated by RGENs are repaired in cells by non-homologous end-joining (NHEJ) or homologous recombination (HR).

Experimental Example 2: Sequence read analysis

Four different sets of genomic DNA were subjected to whole genome sequencing (WGS) to investigate whether *in vitro* cleavage of genomic DNA using RGENs can produce sequence reads with vertical alignment at cleavage sites.

Genomic DNA isolated from RGEN- and non-transformed HAP1 cells was completely cleaved *in vitro* with 300 nM Cas9 and 900nM sgRNA targeting HBB genes. In parallel, WSG was performed without RGEN cleavage *in vitro* by using the genomic DNA isolated from these cells (Fig. 2a). After mapping sequence reads into the reference genome, IGV (intergrative genomics viewer) was used to observe patterns of sequence alignments at the on-target and the four homologous sites.

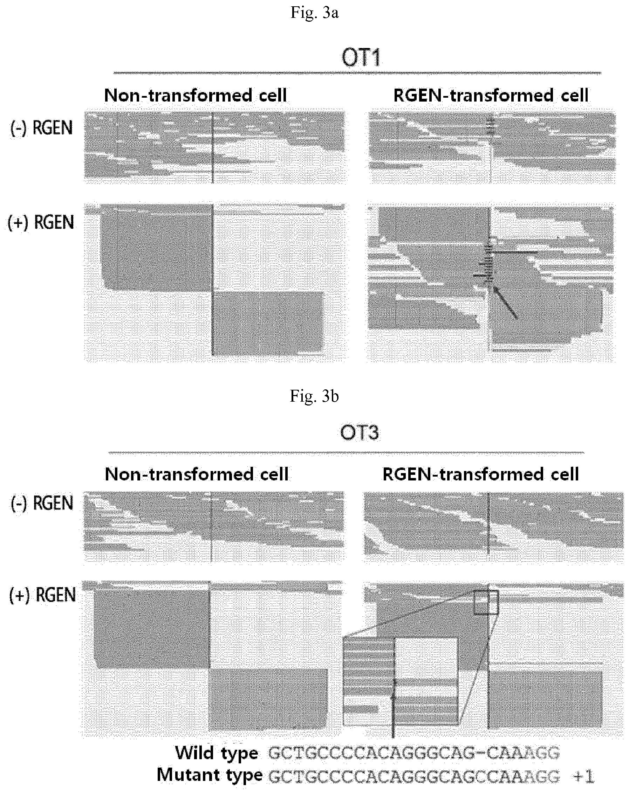

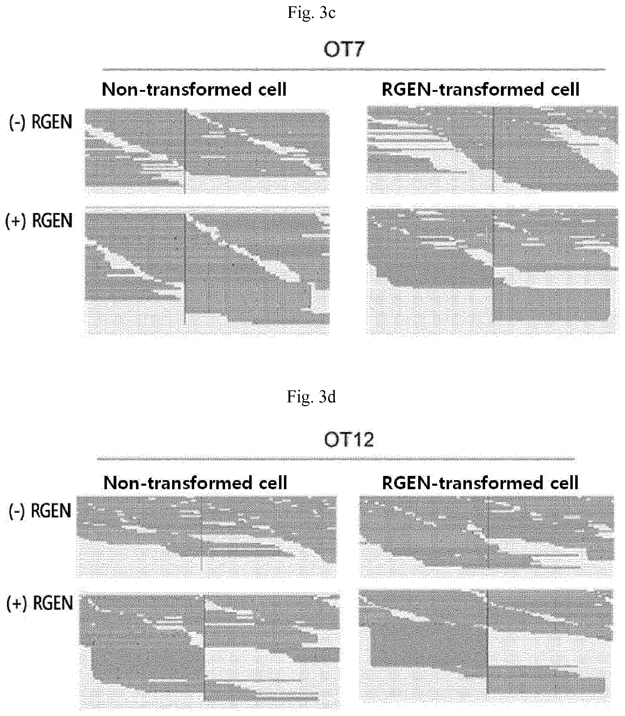

First, the Digenome (cleaved genome) isolated from control group HAP1 cells were examined. At the on-target, OT1, and OT3 sites, unusual patterns of vertical alignments were observed (Fig. 2b and Fig. 3a, b). Sequence reads that spanned the cleavage sites were very rare. In contrast, no such vertical alignments were observed at these sites when the intact genome that had not been treated with the RGEN was analyzed. At the OT7 and OT12 sites, most sequence reads spanned the potential cleavage site (3-bp upstream of the PAM), resulting in a staggered alignment (Fig. 3c, d).

Second, the Digenome isolated from RGEN-transformed cells was compared with the corresponding intact genome. At all five sites, the intact genome gave rise to typical patterns of staggered alignments (Fig. 2b and Fig. 3). In contrast, the Digenome showed both vertical and staggered alignments at the on-target and OT1 sites. At these two sites, almost all sequence reads corresponding to staggered alignments contained indels (Fig. 2b and Fig. 3a and 3b). That is, note that RGENs cannot cleave indel sequences induced by themselves. Meanwhile, no indels were found with sequence reads that spanned the OT7 and OT12 cleavage sites, in line with the T7E1 and deep sequencing results. At the OT3 site, the Digenome showed a straight alignment pattern with a few sequence reads that spanned the cleavage sites. In particular, one sequence read contained an indel, induced by the RGEN (Fig. 3b).

These results suggest that Digenome-Seq is sensitive enough to allow identification of rear off-target mutations and that a vertical alignment of sequence reads is a unique signature of RGEN cleavage *in vitro.*

Experimental Example 3: 5' End plot at signal nucleotide scale

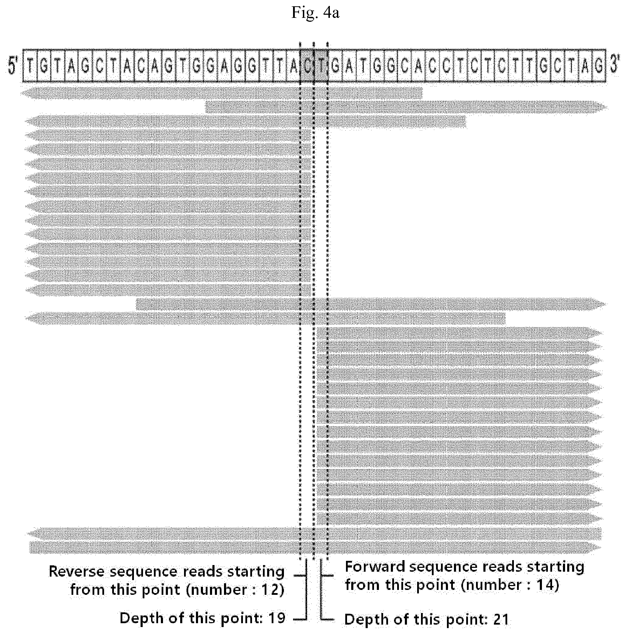

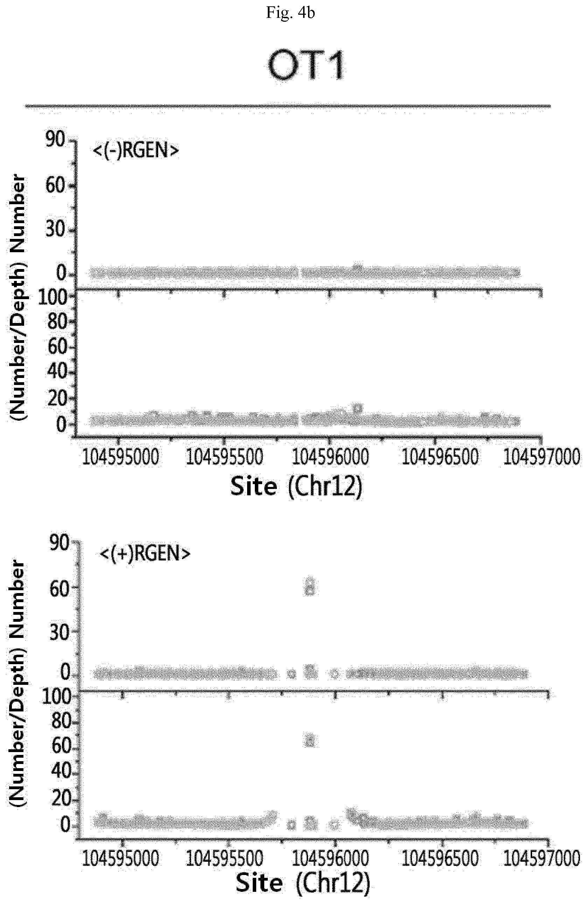

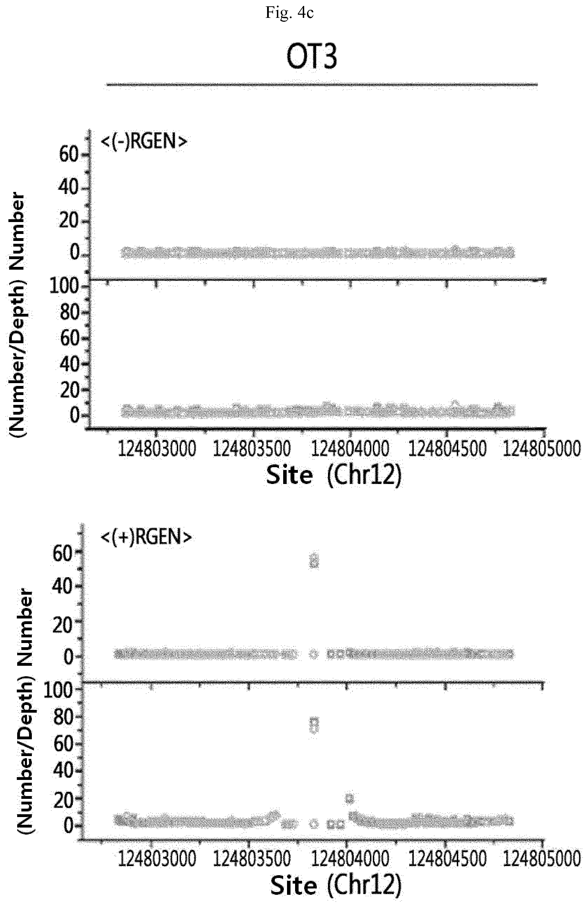

To identify potential RGEN off-target sites on a genomic scale, a computer program that searched for straight alignments of sequence reads was developed. First, the count of sequence reads whose 5' ends started at the nucleotide position near the HBB on-target and two validated off-target sites (OT1 and OT3) at single nucleotide scale (Fig. 4a) was plotted. Because both Watson and Crick strands were sequenced, it was assumed that almost an equal number of sequence reads, corresponding to each strand, should be observed right next to each other at a cleavage site, producing double peaks. As expected, the digenome gave rise to double peaks at the three cleavage sites (on-target site, OT1 and OT3) (Fig. 2c and Fig. 4b, c). The intact genome that had been undergone RGEN treatment *in vitro* did not produce such double-peak patterns at these sites.

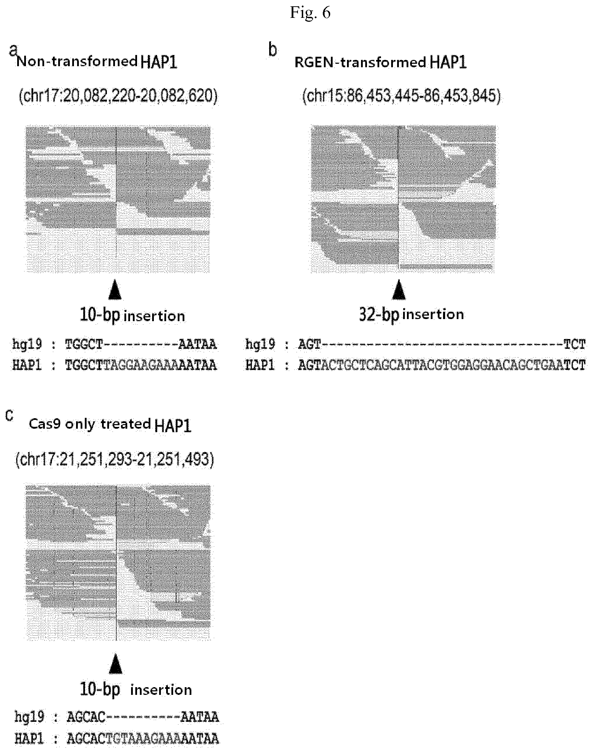

Next, this approach was applied to the entire RGEN-transformed Digenome, non-transformed Digenome, intact RGEN-transformed genome, and intact non-transformed genome. In addition, non-transformed genomic DNA was treated with Cas9 protein *in vitro* in the absence of sgRNA or with a 100-fold lower concentration of RGEN (3 nM Cas9) and subjected to WGS and Digenome analysis. The search was conducted for sites where the count of sequence reads with the same 5' end was greater than 10 in both strands and where at least 19% of sequence reads were aligned vertically. A total of 17 and 78 sites, including the on-target and two validated off-target sites, were identified in the non-transformed digenome treated with 3nM and 300 nM RGEN (Fig. 5a), which showed double-peak patterns in a 5' end plot and straight alignments in a nIGV image. Among these sites, one and two sites in the digenomes treated with 3nM and 300 nM RGEN were false positives that resulted from naturally-occurring indels. In addition, such patterns were observed at a total of 125 sites, including the three validated on- and off-target sites in the RGEN-transformed Digenome. Meanwhile, the invalidated OT7 and OT12 sites did not show double-peak patterns in these three digenomes. Moreover, most sites were commonly identified in the three Digenomes, demonstrating the high reproducibility of Digenome-seq. Specifically, 15 (94%) of the 16 candidate sites (excluding the one false positive site) found in the non-transformed Digenome (3nM RGEN) were also identified in the other two Digenomes. 74 (97%) of 76 candidate sites found in the non-transformed Digenome (300 nM) were also identified in the RGEN-transformed digenome (Fig. 5a). Other than the three validated cleavage sites, none of the other 122 sites were accompanied by indels in the RGEN-transformed Digenome, suggesting that mutations at these candidate sites occurred rarely. Meanwhile, such double-peak patterns were observed at only two positions in the intact genome, three positions in the intact RGEN-transformed genome, and one position in the Cas9 (300 nM) alone-treated, non-transformed genome. All of these positions identified in the three intact genomes were false positive that resulted from naturally-occurring indels in the HAP1 genome relative to the reference genome (Fig. 6a to 6c). Accordingly, double-peak patterns or vertical alignments of sequence reads were unique features found in the Digenomes.

Next, DNA sequences at the 74 common sites identified in the RGEN-transformed and non-transformed Digenomes were compared with the 20 bp on-target site and it was found that of the 20 nucleotides, all but the one at the 5' end were conserved (Fig. 5b). Furthermore, the sequence logo or de novo motif obtained by comparing the DNA sequences at the 74 sites with one another rather than with the on-target sequence clearly showed matches with the on-target sequence at all positions other than the first two nucleotides (Fig. 5c). In addition, 70 (95%) of these double-peak positions were accompanied by the 5'-NAG-3'PAM exactly 3 nucleotides downstream from the expected cleavage position. Only 6.25% (=1/16) of sites are expected to be accompanied by a PAM by chance. Two sites contained the 5'-NAG-3'PAM. Some sites were matched to the on-target site by allowing a DNA or RNA bulge or assuming 5'-NGA-3' as a non-canonical PAM. It is questionable whether 5'-NGA-3' can function as a PAM in cells, but, under our extreme in vitro cleavage conditions, RGENs may cleave these sites. The other sites had no sequence homology with the on-target sequence, suggesting that they could be false positives.

In addition, the fewer nucleotide mismatches there were in homologous sites, the more likely they were to be detected by Digenome-seq. That is, 7 out of 15 (47%) and 14 out of 142 (10%) homologous sites that differed by 3 and 4 nucleotides from the on-target site were detected, but only 15 out of 1,191 sites (1.2%) and one out of 7,896 sites (0.013%) that differed by 5 and 6 nucleotides were detected (Fig. 5d).

Taken together, these results indicate that most of the double-peak patterns are caused by RGEN cleavage in vitro and that Digenome-seq can find nuclease cleavage sites on a genomic scale.

Experimental Example 4: Deep sequencing to identify off-target effects at candidate sites

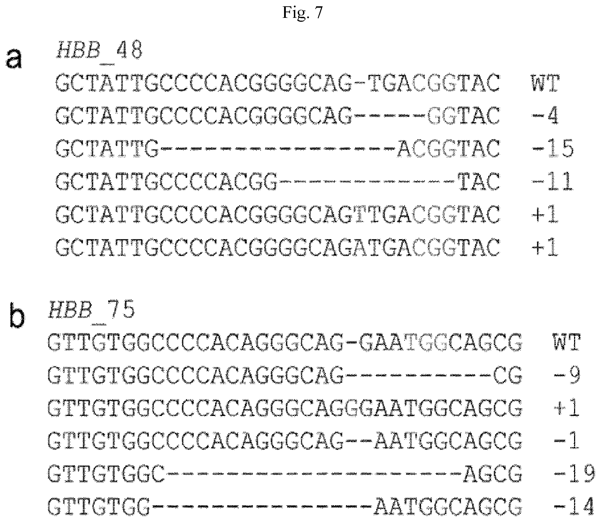

Deep sequencing was performed to validate off-target effects at the 74 common sites identified in the two Digenomes (Fig. 5e). Moreover, the other 8 sites that differed from the on-target site by three nucleotides but were not detected by Digenome-seq was also tested. No off-target indels were detected at these 8 sites with a frequency of at least 0.1% and greater than that of negative control group (Fisher exact test, p < 0.01) (Fig. 5d). Indels were observed at a total of 5 sites including already-validated on-target, OT1, and OT3 sites, among the 74 sites, with frequencies ranging from 0.11% to 87% (Fig. 5e and Fig. 7a, b). At the other two newly-validated off-target sites, termed HBB_48 and HBB-75, indels were detected with a frequency of 0.11% and 2.2%. These two sites differed from the on-target site by three nucleotides. There were three nucleotide mismatches at the HBB_48 site and two mismatches at the HBB_75 site, relative to the 20-nt sgRNA sequence, which differed from the on-target site by one nucleotide at the 5' end. None of these validated off-target sites harbored a DNA or RNA bulge compared to the 20-nt sgRNA sequence, nor were they accompanied by a non-canonical PAM such as 5'-NGA-3' or 5'-NAG-3'. Note that these two new off-target sites and the other three sites were identified independently in each of the three Digenomes. These results show that Digenome-seq is a sensitive and reproducible method to identify nuclease off-target effects on a genomic scale.

Experimental Example 5: Digenome sequencing for VEGF-A specific RGEN

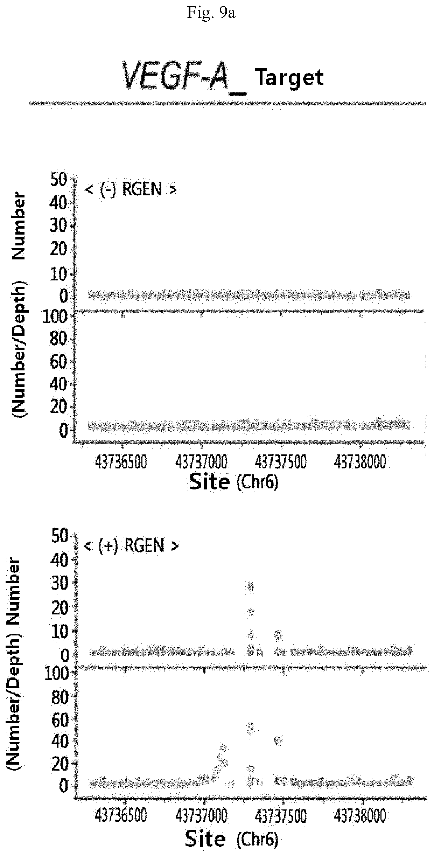

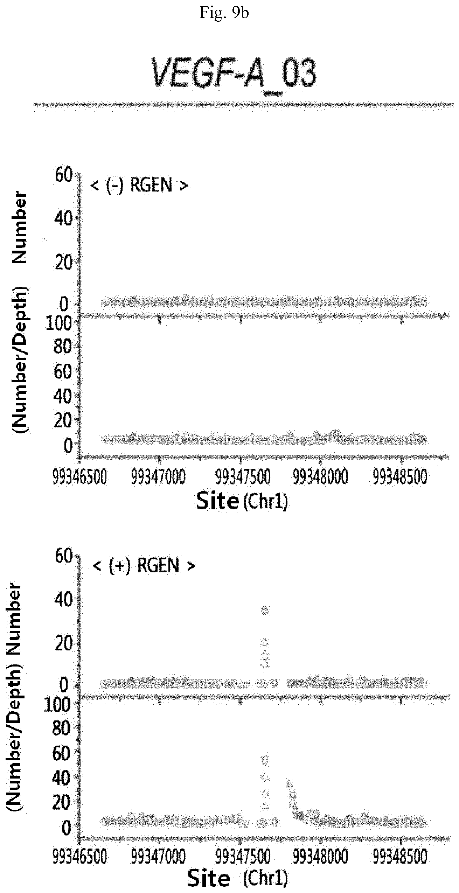

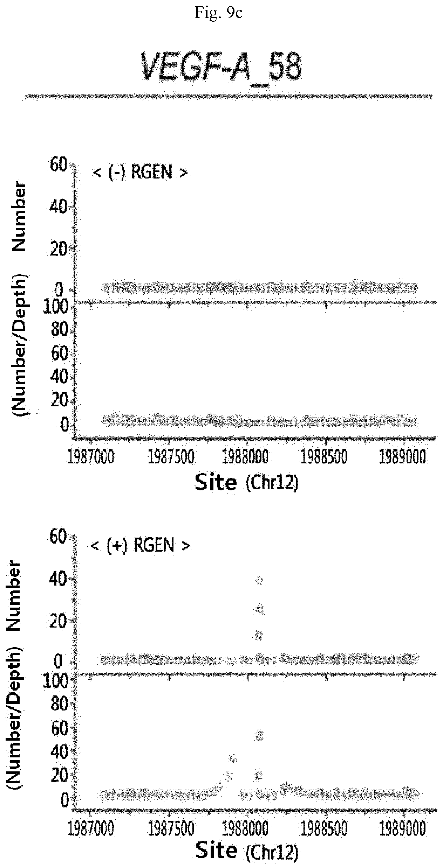

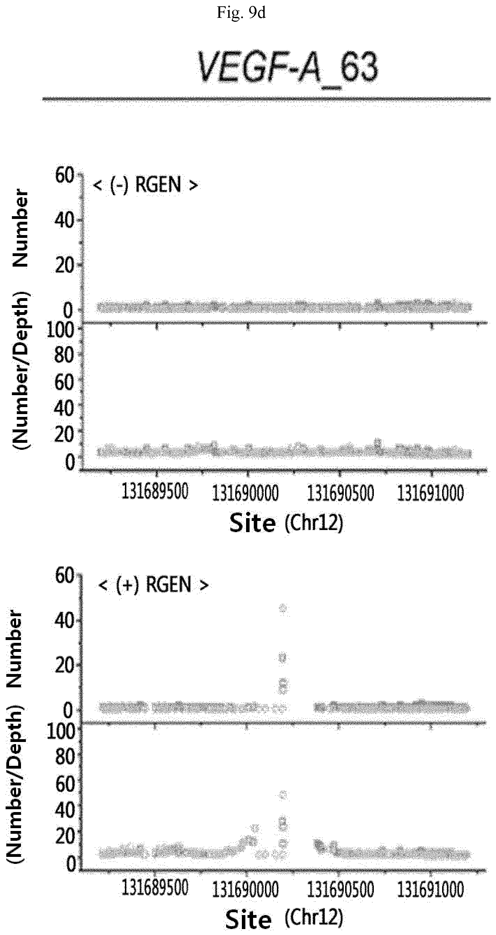

Next, the present inventors tried to identify whether Digenome-seq is applicable to the other genes other than the HBB genes. Digenome-seq was performed with another RGEN that had been shown to induce on-target mutations at a VEGF-A locus and additionally, off-target mutations at four homologous sites. A total of 81 sites, including the on-target and four already validated off-target sites, were identified that showed double-peak patterns (Fig. 8a and Fig. 9). All of the DNA sequences at these 81 sites contained the canonical 5'-NGG-3' PAM sequences. Comparison of these sequences with the on-target sequences showed matches at every nucleotide site. Moreover, these sequences were also compared with one another to obtain a de novo motif: The resulting sequence logo also showed matches with the target sequence at almost every nucleotide position, suggesting that every nucleotide in the 20-nt sgRNA sequence contributed to the specificity of RGEN (Fig. 8b and 8c).

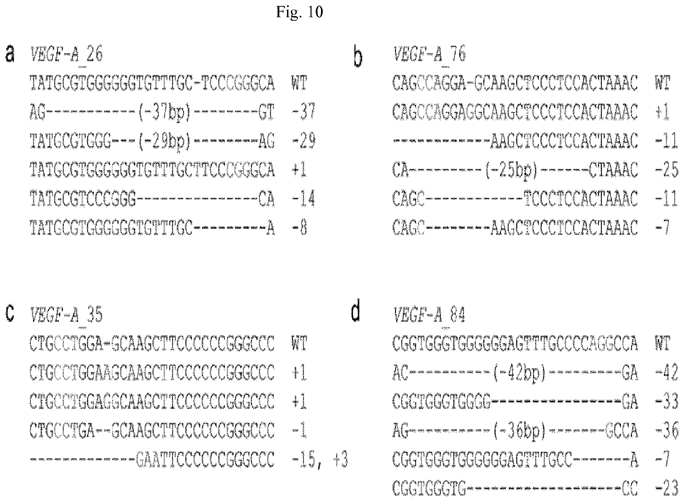

Next, targeted deep sequencing was used to identify on-target and off-target effects at the 81 sites identified by Digenome-seq and 28 sites that differed by 3 or fewer nucleotides from the on-target site but were not identified by Digenome-seq. This RGEN was highly active in HAP1 cells, producing indels at the on-target site with a frequency of 87% and at the four previously-validated off-target sites with frequencies that ranged from 0.32% to 79%. In addition, four off-target sites were additionally identified at which indels were induced with frequencies that ranged from $0.065 \pm 0.021\%$ to $6.4 \pm 1.2\%$ (Fig. 8e and Fig. 10). The indel frequency at these sites obtained using the RGEN was significantly greater than that obtained using an empty vector control group (Fisher exact test, p < 0.01). These off-target sites contained one to six nucleotide mismatches with the 20-nt target sequence and at least one mismatch in the PAM-proximal seed region. There are 13,892 sites with 6-nt mismatches in the human genome but only 6 sites (0.043%) were identified by Digenome-seq and, among them, only one site was validated by deep sequencing (Fig. 8d and 8e). Thus far, an RGEN off-target site with 6-nt nucleotide mismatches with on-target sites had never previously been identified. None of these off-target sites contained a DNA or RNA bulge, although 40 out of 81 sites identified by Digenome-seq contained a missing or extra nucleotide compared to the 20-nt target sequence. At all the other sites, including those not identified by Digenome-seq, indel frequencies obtained using the RGEN were 0.05% or less, or were smaller than or not statistically different from those obtained using an empty vector control group.

It can be seen from these Experimental examples 1 to 5 that the Digenome-seq of the present disclosure is a very highly reproducible method for detecting off-target sites of programmable nucleases.

Experimental Example 6: Improved Digenome-seq

First, the present inventors developed a scoring system capable of identifying an *in vitro* cleavage site using the whole genome sequencing (WGS) data on a human genome. The Digenome-seq analysis identified in these Experimental examples 1 to 5 has a high reproducibility, but there is a problem that some sites having a heterogeneous cleavage pattern or a low sequencing depth may be missing. The present inventors have found that these sites can be identified by estimating the case where the Cas9 protein makes one or two nucleotide overhangs at the blunt end. Based on the alignment pattern of the sequence read, a DNA cleavage score was assigned to each nucleotide site (Fig. 11). Through this program, a number of additional sites that were not detected in the existing Digenome-seq were detected. A genomic scale plot of the cleavage score shows that few false positive sites are found in the uncleaved genomic DNA (Fig. 12a):

A small number of false positive sites identified in the whole genome include indels (insertion and deletion), which occurs naturally in genomic DNA, which can be easily screened. As can be seen in two independent Digenome-seq analyses, the cleavage score for the human genome has a high reproducibility (R2 = 0.89) (Fig. 13).

The present inventors also found that the sgRNA transcribed through the plasmid template in the Digenome-seq analysis does not cleave even a bulge-type off-target site of any nucleotide-deficient false positive at an on-target site where it was detected with transcribed one using oligonucleotide double strand (Fig. 12b and Fig. 14).

This is because sgRNA transcribed from the oligonucleotide double strand is not a homogeneous component, including incomplete molecules transcribed from oligonucleotides that failed to synthesize. As a result, the cleavage sites identified using the sgRNA transcribed from the plasmid template are more highly homologous to the on-target site than those identified using the sgRNA transcribed from the oligonucleotide template (Table 1 and Table 2). The DNA sequences surrounding the cleavage site can be identified from a sequence logo obtained by comparing them (Fig. 12c).

[Table 1]

| Oligonucleotide template | | | |
| --- | --- | --- | --- |
| Chromosome | location | DNA sequence at cleavage site | Bulge |
| chr11 | 5248215 | CTTGCCCCACAGGGCAGTAACGG | x |
| chr1 | 38230668 | CTCTGTCTCGCGCTGCTTTTGGG | x |
| chr1 | 177593980 | TCTACCCCACATGGCAGTAATGG | x |
| chr2 | 112686732 | GGTCCCGGGAATAGCGGGTAAGG | x |
| chr2 | 240591539 | ACAGCCCCACAGGGCACTAGAGG | x |
| chr3 | 3662556 | AAAGCCCCACAGGGTAGTAGAGG | x |
| chr3 | 19957834 | GCTACCCCACAGGGCATTAGGGG | x |
| chr4 | 45763604 | GCTGCCCCACATGACAGAAATGG | x |
| chr4 | 48091817 | ACTCGTCTCCGATATCCAGTTGG | x |
| chr4 | 55979545 | GGTGTAACCCGGAGTGACCAAGG | x |
| chr4 | 55979546 | GGTGTAACCCGGAGTGACCAAGG | x |
| chr4 | 148531374 | GTTACCTCACAGAGCAGAAAGGG | x |
| chr4 | 166593737 | TATGCTCCAGAGGGTAGTAATGA | x |
| chr5 | 14347051 | CATACCCCACAGGTCAGTAAGGA | x |
| chr5 | 131423385 | TCTGCCCCACAGGCCAGGAAGGG | x |
| chr6 | 50041372 | TCTGCCCCACATGGCAGTAATGA | x |
| chr6 | 80093919 | TGAGTTCTCCAATATCCAGTTGG | x |
| chr6 | 85738203 | ACTGCCCCACAGGGAAGTAATAG | x |
| chr8 | 41296585 | TCAGCCCCACAGGTCAGCAATGG | x |
| chr9 | 24439672 | GGACTCCTCCAATATCCTGTTGG | x |
| chr9 | 78341070 | GTTACCCC-CAGGGAAGTATAGG | RNA Bulge |
| chr9 | 104595883 | TCAGCCCCACAGGGCAGTAAGGG | x |
| chr9 | 134809673 | TTTGCCCCTCAGGGCAGCTAAGG | x |
| chr9 | 134994964 | CCTGCCCCACAGGGCAATTATGG | x |
| chr10 | 71943328 | CATGGCCAGGAAGAGAAGGCTGG | x |
| chr10 | 72286450 | CAAGCCCCACAGGGCAGACAGGG | x |
| chr10 | 73555691 | CAGGCCCCACAGGACAGGAAGGG | x |
| chr11 | 3125346 | AGCCCCCACAGGGCAGGTAGGGG | x |
| chr11 | 59611432 | CGGCCAGATTCATGGCAATCAGG | x |
| chr11 | 76387498 | CTGCCCCTCAGGGACAGTATGGG | x |
| chr12 | 27234755 | GATGCCTCACAGGACAGGAAGGG | x |
| chr12 | 40327469 | GCTATGGTTCCTGAACGGCCTGG | x |
| chr12 | 93549202 | ATTGCCCCACGGGGCAGTGACGG | x |
| chr12 | 124803834 | GCTGCCCCACAGGGCAGCAAAGG | x |
| chr13 | 29005426 | TTGGTCAATTCGTCGCCTTACGG | x |
| chr13 | 44886376 | GGAGCCCCACAGGGCAGAGAGGG | x |
| chr14 | 36889538 | GTTATCCCACAGGACAGTGAGGG | x |
| chr14 | 59445901 | CTT-CCCCAATATCCAGT-AGGG | RNA Bulge |
| chr14 | 94585327 | ATGGCCCCACAAGGCAGAAATGG | x |
| chr15 | 29983547 | CCAGCCCCACAGGGCAGTAAAGC | x |
| chr15 | 46598129 | GTTGCCCCTCAGGACAGTACAGG | x |
| chr15 | 99709337 | TGTGCCCCACAGGG-AGTGAGGG | RNA Bulge |
| chr16 | 49082904 | GCAGCCCCACAGGTCAGTGAGGG | x |
| chr17 | 8370253 | TGCTCCCACAGGGCAGTAAACGG | x |
| chr18 | 745994 | AAAATACCTCGTTGATTTCCAGG | x |
| chr18 | 6663844 | GTTGCCCCACTGGGGAGAAAAGG | x |
| chr19 | 29880768 | TGTGCCCCACAGG-CAGTAGATG | RNA Bulge |
| chr19 | 34262013 | CTGCTCCACAGGGCAGGTATGGG | x |
| chr19 | 37539042 | CTTGCACCACAGAGCACTAAGGG | x |

| chr20 | 39992928 | AGTGGCCCCAGGGCAGTGAGGG | X |
| chr22 | 17230623 | TGTGCCCCACAGAGCACTAAGGG | X |
| chr22 | 35537395 | AGTGCCCCACAGGGGAGAAATGG | X |
| chrX | 75006257 | GTGGCCCCACAGGGCAGGAATGG | X |
| chrX | 132429379 | GCATCCCCACAGGGCAGTATGTG | X |

[Table 2]

| Plasmid template | | | |
| Chromosome | location | DNA sequence at cleavage site | Bulge |
|---|---|---|---|
| chr11 | 5248215 | CTTGCCCCACAGGGCAGTAACGG | X |
| chr1 | 17346702 | GGTCCCCACAGGGTCAGTAAGGG | X |
| chr1 | 177593980 | TCTACCCCACATGGCAGTAATGG | X |
| chr3 | 3662556 | AAAGCCCCACAGGGTAGTAGAGG | X |
| chr3 | 19957634 | GCTACCCCACAGGGCATTAGGGG | X |
| chr4 | 148531374 | GTTACCTCACAGAGCAGAAAGGG | X |
| chr5 | 14347051 | CATACCCCACAGGTCAGTAAGGA | X |
| chr5 | 131423385 | TCTGCCCCACAGGCCAGGAAGGG | X |
| chr6 | 23709579 | GAAGCCCTACAGGGCAGCAATGG | X |
| chr6 | 50041372 | TCTGCCCCACATGGCAGTAATGA | X |
| chr8 | 24931381 | AGTGCCACACACAGCAGTAAGGG | X |
| chr9 | 104595883 | TCAGCCCCACAGGGCAGTAAGGG | X |
| chr9 | 134994964 | CCTGCCCCACAGGGCAATTATGG | X |
| chr10 | 72286450 | CAAGCCCCACAGGGCAGACAGGG | X |
| chr10 | 73555691 | CAGGCCCCACAGGACAGGAAGGG | X |
| chr11 | 76387498 | CTGCCCCTCAGGGACAGTATGGG | X |
| chr12 | 27234755 | GATGCCTCACAGGACAGGAAGGG | X |
| chr12 | 93549202 | ATTGCCCCACGGGGCAGTGACGG | X |
| chr12 | 124803834 | GCTGCCCCACAGGGCAGCAAAGG | X |
| chr13 | 44886376 | GGAGCCCCACAGGGCAGAGAGGG | X |
| chr14 | 36889538 | GTTATCCCACAGGACAGTGAGGG | X |
| chr14 | 94585327 | ATGGCCCCACAAGGCAGAAATGG | X |
| chr15 | 34059408 | GTTACCACACAGAGCAGTTAAGG | X |
| chr15 | 46598129 | GTTGCCCCTCAGGACAGTACAGG | X |
| chr16 | 49082904 | GCAGCCCCACAGGTCAGTGAGGG | X |
| chr17 | 8370253 | TTGCTCCCACAGGGCAGTAAACG | X |
| chr19 | 8560462 | AAATCCCCACAGGGCAGTAAGGC | X |
| chr20 | 39992928 | AGTGGCCCCAGGGCAGTGAGGG | X |
| chr22 | 17230623 | TGTGCCCCACAGAGCACTAAGGG | X |
| chrX | 75006257 | GTGGCCCCACAGGGCAGGAATGG | X |

Accordingly, the number of false negative sites can be significantly reduced using the cleavage scoring system of the present disclosure, and the number of false positive sites can be significantly reduced using the sgRNA transcribed in the plasmid template.

Experimental Example 7: Multiplex Digenome-seq

Unlike the other methods, Digenome-seq can be used in combination without increasing sequencing depth proportional to the number of nuclease. The present inventors selected 10 sgRNAs that were individually analyzed using GUIDE-seq, which is more sensitive than IDLV detection and other methods. The present inventors cleaved human genomic DNA with a mixture of one additional sgRNA targeting Cas9 protein, 10 sgRNA, and HBB gene, and performed two independent WGS analyses (Fig. 15a). Next, the scoring system was used to investigate *in vitro* cleavage sites on a genomic scale. As a result, a total of 964 sites were identified in the human genome (Tables 3 to 12). Next, the site was then classified according to the edit distance to the on-target site (Fig. 15a and Tables 3 to 12).

[Table 3]

| VEGFA1 | | | |
|---|---|---|---|
| Chr | Position | DNA cleavage Score | DNA seq at a cleavage sites |
| Chr6 | 43737290 | 389070 | GGGTGGGGGGAGTTTGCTCCAGG |
| Chr15 | 65637537 | 255675 | GGATGGAGGGAGTTTGCTCCTGG |
| Chr5 | 7067159 | 221853 | GAGGGTGGGGAGTTTACTCCTGG |
| Chr1 | 99347651 | 212884 | GGGGAGGGGAAGTTTGCTCCTGG |
| Chr12 | 1988077 | 206789 | CGGGGGAGGGAGTTTGCTCCTGG |
| Chr22 | 37215276 | 204286 | GGGTGGGGGGAGTTTGCCCCAGG |
| Chr17 | 32986325 | 177694 | GGGGGTGGGGACTTTGCTCCAGG |
| Chr1 | 82627648 | 165975 | GGGTGCTGGCACAGTGCTCCTGG |
| Chr12 | 26641302 | 164500 | AGTTTGGGGGAGTTTGCCCCAGG |
| Chr1 | 233157354 | 156007 | GGAGGAGGGGAGTCTGCTCCAGG |
| Chr10 | 124731416 | 153228 | AGCTGGAGGGAGTTTGCCCCAGG |
| Chr12 | 131690199 | 143751 | GGGAGGGTGGAGTTTGCTCCTGG |
| Chr11 | 71497119 | 143413 | AGGAAGGAGGAGTTAGCTCCTGG |
| Chr20 | 7836107 | 142045 | CAGGTGGGAGAGTTTGCTCCCAG |
| Chr17 | 39796328 | 140863 | TAGTGGAGGGAGCTTGCTCCTGG |
| Chr4 | 8453803 | 140625 | GAGTGGGTGGAGTTTGCTACAGG |
| Chr9 | 88657759 | 140587 | GGATGGAGGTAGTTTGTTCCTGG |
| Chr9 | 93925190 | 140509 | GGGGGTGGGGAGCATGCTCCAGG |
| Chr3 | 125633992 | 137819 | AGGAAGGAGGAGTTAGCTCCTGG |
| Chr16 | 8763213 | 134448 | AAGTAAGGGAAGTTTGCTCCTGG |
| Chr8 | 140714327 | 131288 | GGGAGGAGAGAGTTTGCTCTCTG |
| Chr20 | 56175356 | 130037 | AGGGAGGAGGAATTTGCTCCAGG |
| Chr15 | 93140401 | 126800 | GGGGGAGGGAAGTTTCCTCCAGG |
| Chr2 | 209437600 | 115754 | AGGGAGGGAGAATTTGCTCCTGG |
| Chr3 | 128284321 | 115556 | AGGTGGTGGGAGCTTGTTCCTGG |
| Chr5 | 32945275 | 115513 | GCGTGGGGGGTGTTTGCTCCCGG |
| Chr6 | 14316373 | 114987 | GTGGGGGTAGAGTTTGCTCCAGG |
| Chr13 | 26202812 | 113722 | GGTTGAGGGGAGTCTGCTCCAGG |
| Chr5 | 156390 | 112826 | TGCTCGGGGGAGTTTGCACCAGG |
| Chr21 | 43689878 | 106684 | GGCCCAGGGGAGTTTGCTCCCAG |
| Chr19 | 51310920 | 106639 | GTGCAGGGGGAATTTGCTTCCGG |
| Chr5 | 139263024 | 106310 | TTGGGGGGGCAGTTTGCTCCTGG |
| ChrX | 82127748 | 104937 | AGAGGGGGAGAGTTTGCCCCTGG |
| Chr7 | 17819097 | 101772 | ACAACTGGGGAGTTTGCTCCTGG |
| Chr22 | 41676762 | 100633 | AGTGCAGGGAGCTTGCTCCTGG |
| Chr2 | 96056645 | 98836 | GGGTGGGGAGAGTTTCTTCCTGG |
| Chr3 | 195871264 | 97500 | GGTGGGGGAGAGCTAGCTCCGGG |
| Chr11 | 3445204 | 97085 | AGGAAGGAGGAGTTAGCTCCTGG |
| Chr6 | 45554056 | 96928 | GGGGTGGGAGAGTTTGCTCTCTG |
| Chr18 | 366714 | 94490 | GGGGGCAGGGAGATTGCTCCTGG |
| Chr3 | 13580170 | 91496 | ATGGGGGAGAAACTTGCTCCTGG |
| ChrX | 19185601 | 89375 | GGGAGGGGAGAGTTTGTTCCAGG |
| Chr11 | 67574262 | 66762 | AGGAAGGAGGAGTTAGCTCCTGG |
| Chr17 | 47317539 | 85047 | CTGGTGGGGGAGCTTGCTCCAGG |
| Chr6 | 91365255 | 83954 | CCCGGGGGGAAGCTTGCTCCAGG |
| Chr22 | 18454623 | 83642 | GGAAAGGAGGAGCTTGCTCCAGG |
| Chr22 | 19698463 | 83277 | GAGGGGGAGCAGTTTGCTCCAGG |

| | | | |
|---|---|---|---|
| Chr3 | 36358934 | 82931 | AGTGGGGGAGAGTATGCTCCGGG |
| Chr21 | 37116659 | 77154 | AAGTGGGAAGAGTTTGTTCCAGG |
| Chr11 | 117481208 | 75392 | GGGCAAGGGGAGGTTGCTCCTGG |
| Chr7 | 29081029 | 74507 | GGAGTGGGTGAGCTTGCTCCTGG |
| Chr17 | 63035708 | 73840 | AGGAGGGGGAAGAATGCTCCAGG |
| Chr2 | 181170961 | 67144 | TGGGGAGGGGAAATTGCTCCTGG |
| Chr6 | 109284989 | 66994 | TGGAGAGGGGAGTTGGCTCCTGG |
| Chr11 | 122583511 | 66565 | AGAAGAGGGGATTTTGCTCCTGG |
| Chr5 | 56172079 | 66003 | GGTGGGGGTGGGTTTGCTCCTGG |
| Chr1 | 33643288 | 64800 | GGGTGGGTGGAGTTTGCTACTGG |
| Chr8 | 28483353 | 63725 | AAGTGGGAGGAGACTGCTCCAGG |
| Chr22 | 38219333 | 60450 | AGGTCGGGGGAGTTAGATCCCGG |
| Chr15 | 29263777 | 59556 | GGGATGGGAGAGTCTGCTCCTGG |
| Chr2 | 30430777 | 57143 | AGGGAGAGGGAGCTTGCTCCCAG |
| Chr12 | 107832636 | 54149 | TCTTGGGGGGAAGTTGCTCCAGG |
| Chr4 | 165246171 | 53056 | GGAGGGGGGGCTTTTGCTCCAGG |
| Chr8 | 10804669 | 48246 | GAGTGAGGAGAGCTTGCTCCATG |
| Chr5 | 95220670 | 46459 | GGGAGCAGGGAATTTGCTCCAGG |
| Chr2 | 129199817 | 44575 | TCCTGAGGGCAGTTTGCTCCAGG |
| Chr13 | 31251013 | 43669 | TGTAGAGGGAGTTTTGCTCCCGG |
| Chr16 | 89679839 | 43503 | GGAGGAGGGAACTTTGCTCCAGG |
| Chr1 | 20166440 | 42581 | GTGGGAGGATAGCTTGCTCCTGG |
| Chr18 | 13983474 | 37242 | GGGTGAAAGAAGTTTACTCCTGG |
| Chr6 | 50485682 | 36345 | ATGTGTGGGGAATTTGCTCCAGG |
| Chr1 | 205484156 | 35692 | GTGTGAGTGGAGTTTGCTCTGGG |
| Chr6 | 109070771 | 35169 | GGTGGGGGAAAGTTTGCTCCTGA |
| Chr15 | 101813024 | 34008 | AAGGAGGCGGAGCTTGCTCCTGG |
| Chr11 | 11823598 | 31395 | GGCTGGAGGGGATTTGCTCCTGG |
| Chr9 | 5336085 | 31120 | TCGTGGTGGGAATTTACTCCTGG |
| Chr4 | 116853325 | 29172 | AAAGGGGGGAACTTTGCTCCAGG |
| Chr11 | 86695108 | 28100 | AGGGAAGGGGAATTTGCACCTGG |
| Chr5 | 57030871 | 27679 | CTCTGAGGGGAGTTTGCTCTGGG |
| Chr15 | 84047385 | 26663 | GGAGTCAGGGAATTTGCTCCTGG |

[Table 4]

| | | VEGFA2 | |
|---|---|---|---|
| Chr | Position | DNA cleavage Score | DNA seq at a cleavage sites |
| Chr2 | 242214607 | 1670405 | ATTCCCCCCACCCCGCCTCAGG |
| Chr9 | 103599649 | 1051618 | ACACCCCCCCACCCCGCCTCAGG |
| Chr14 | 75098723 | 1009605 | CCTCACCCCCACCCCACCTCTGG |
| Chr11 | 31817483 | 952389 | GGGCCCCTCCACCCCGCCTCTGG |
| Chr17 | 4358752 | 726896 | TACCCCCCACACCCCGCCTCTGG |
| Chr16 | 56963429 | 579579 | TGCCCCCCCCACCCCACCTCTGG |
| Chr12 | 26025095 | 561897 | CATTCCCCCCACCCCACCTCAGG |
| Chr1 | 111860603 | 445046 | TAAATCCTCCACCCCACCTCAGG |
| Chr18 | 21359559 | 407413 | GCCCCCACCCACCCCGCCTCTGG |
| Chr6 | 43738562 | 362166 | GACCCCCTCCACCCCGCCTCCGG |
| Chr10 | 116294256 | 353588 | CCCCACCCCCACCCCGCCTCAGG |
| Chr22 | 32532901 | 351783 | GAGCCACTGCGCCCGGCCCCCGG |
| Chr9 | 27338875 | 339351 | GACCCCTCCCACCCCGACTCCGG |
| Chr17 | 40044757 | 334353 | TGCCCCTCCCACCCCGCCTCTGG |
| Chr12 | 31812350 | 318535 | GATCGACTCCACCCCGCCTCTGG |
| Chr13 | 100546989 | 300000 | CCCCCCCCCCCCCCGCCTCAGG |
| Chr19 | 13122189 | 299926 | GCCCCCACCACCCCACCTCGGG |
| Chr5 | 6715119 | 294250 | CTACCCCTCCACCCCGCCTCCGG |
| Chr10 | 72538218 | 293269 | CAGTCCCCCCACCCCACCTCTGG |
| Chr16 | 13492458 | 286462 | TCCGCCCCCCACCCCACCTCCGG |
| Chr4 | 38537628 | 280706 | CTCCCCACCCACCCCGCCTCAGG |
| Chr6 | 160552566 | 278803 | TCAGACCTCCACCCCGCCTCAGG |
| Chr16 | 81442194 | 261364 | TTCACCATCAACCCCCACTTCAG |
| Chr4 | 182638032 | 250540 | TCCTTTCTCCACCCCACCTCTGG |
| Chr10 | 135149948 | 247222 | CGCCCTCCCCACCCCGCCTCCGG |
| Chr11 | 2686249 | 231975 | CTCACCCCCCACCCCACCTCTGG |
| Chr11 | 83433600 | 193501 | GTCACTCCCCACCCCGCCTCTGG |
| Chr4 | 148977716 | 187619 | TCCGCCCCCACCCCACCTCCGG |
| Chr1 | 198124848 | 187500 | TGCAACCTCCTCCCCGCCTCGGG |
| Chr9 | 131706582 | 185503 | AGCCAACCCCACCCCGCCTCTGG |
| Chr17 | 29983010 | 158558 | CATCTTCCCCACCCCGCCTCTGG |
| ChrX | 70597642 | 142798 | CTACGCTCCACCACCACCTCCAG |
| Chr16 | 69166711 | 130116 | AGTAGCCCCCACCCCGCCTCGGG |
| Chr4 | 1496258 | 121825 | AGGCCCCCACACCCCGCCTCAGG |
| Chr4 | 160033153 | 121760 | TCACTCCCCCACCCCACCTCTGG |
| Chr11 | 71948805 | 113590 | GCTTCCCTCCACCCCGCATCCGG |
| Chr18 | 19751064 | 106648 | CGTCTCCCCCACCCCACCTCAGG |
| Chr11 | 374667 | 92770 | AGGCCCCCCCGCCCCGCCTCAGG |
| Chr14 | 19361511 | 87124 | GTCGAGGTCCACCCCGCCTCAGG |
| Chr5 | 139028257 | 86248 | CTCCCCCCCCTCCCCGCCTCGGG |
| Chr9 | 140428961 | 86077 | CTCCAGACTCCTCCCCCTCCTC |
| Chr3 | 140398801 | 81457 | CAACCCCCCCACCCCGCTTCAGG |
| Chr20 | 25240252 | 80973 | CCCACACCCCACCCCACCTCCGG |
| Chr8 | 122367964 | 70587 | CCACCATCCCACCCCGCCTCTGG |
| ChrX | 118865483 | 60675 | GTCCTCCACCACCCCGCCTCTGG |
| Chr1 | 5477153 | 60344 | CTGCCTCCTCACCCCGCCTCAGG |
| Chr6 | 10882454 | 56969 | CCCTCTCCACCCCCACCCTCTGG |
| Chr13 | 107367839 | 55772 | TCTCCCCTGTACCCCGCCTCTGG |

| | | | |
|---|---|---|---|
| Chr11 | 14596970 | 44608 | CCCTACCCCCACCCCACCTCAGG |
| Chr17 | 48624779 | 38894 | CCCTTCCCCCACCCCACCTCCGG |
| Chr19 | 42806801 | 38547 | TTCTCCCTCCTCCCCGCCTCGGG |
| Chr2 | 225762279 | 38133 | CTCCCCTCCACCCCAGCCTCCGG |
| Chr12 | 101603788 | 37584 | GCCAGCCCTCACCCCGCCTCGGG |
| Chr2 | 12744776 | 36920 | GACACACCCCACCCCACCTCAGG |
| Chr11 | 45402251 | 33163 | CGATCCTCTTACCCCGCCTCCGG |
| Chr6 | 167929803 | 32514 | GCTGTCTCCCACCCCGCCTCAGG |
| Chr21 | 37111654 | 31086 | TCTTCTTTCCACCCCGCCTCAGG |
| Chr17 | 41797972 | 29279 | TCCCCTTCCCACCCCACCTCCGG |
| Chr9 | 13973961 | 29086 | CAAGTAATCCACCCCACCTCAGG |
| Chr1 | 112708281 | 28448 | GCCACCTTCCACCCCACCTCAGG |
| Chr5 | 58336894 | 27731 | CTTCCTCCACCCCGCAGTCTATG |
| Chr17 | 58404869 | 26399 | CGCCCACCCCACCCCACCTCAGG |
| Chr4 | 84744222 | 25794 | CCAGCTCCCCACCCCACCTCAGG |

[Table 5]

| | | VEGFA3 | |
|---|---|---|---|
| Chr | Position | DNA cleavage Score | DNA seq at a cleavage sites |
| Chr20 | 2650069 | 500934 | GGTGTATGAGTGTGTGCGTCGGA |
| Chr2 | 177463426 | 450296 | GGTGAGTGTGTGTGTGCATGTGG |
| Chr5 | 89440969 | 437216 | AGAGAGTGAGTGTGTGCATGAGG |
| Chr5 | 98946319 | 431533 | GGTGTAGTGGTGTGTGCTTGTGG |
| Chr6 | 39028642 | 412319 | GGTGTGTGAGTGTGTGCATTGGG |
| Chr4 | 58326608 | 395166 | AGTGAGTGAGTGAGTGAGTGAGG |
| Chr19 | 1716792 | 367812 | CATGAGTGAGTGTGTGGGTGGGG |
| Chr16 | 74898121 | 311776 | GGTGAGAGAGTGTGTGCGTAGGA |
| Chr7 | 152671378 | 309713 | AGTGAGTGAGTGAGTGAGTGAGG |
| Chr4 | 89935133 | 298318 | TCTGAGTGAGTGTGGGCATGGGG |
| Chr16 | 84032646 | 287579 | GGTGAATGAGTGTGTGCTCTGGG |
| Chr22 | 37662824 | 277795 | GCTGAGTGAGTGTATGCGTGTGG |
| Chr20 | 50724405 | 270841 | CGTGAGTGAGTGTGTACCTGGGG |
| Chr6 | 157078327 | 269512 | GATGAGTGAGTGAGTGAGTGGGG |
| Chr11 | 79178523 | 268949 | AGTGAGTGAGTGAGTGGGGTTGG |
| Chr14 | 65569159 | 247298 | AGTGAGTGAGTGTGTGTGTGGGG |
| Chr20 | 20178284 | 240641 | AGTGTGTGAGTGTGTGCGTGTGG |
| Chr17 | 33323269 | 238213 | TGTGAGTGAGTATGTACATGTGG |
| Chr7 | 23792987 | 227214 | TATGAGTGAGTGTGTGGATGAGG |
| Chr5 | 34452076 | 220662 | TGTGTGAGTGTGTGTGTGCGTGG |
| Chr5 | 29367379 | 213110 | TGTGAGTGAGTGTGTGTATGGGG |
| Chr14 | 98442534 | 205743 | GGTGAGTGTGTGTGTGAGTGTGG |
| Chr15 | 29699015 | 204548 | GGAGAGCGAGTGTGTGCATTTGG |
| Chr8 | 143890827 | 204401 | GGTGTATGAGTGTGTGTGTGAGG |
| Chr3 | 10723167 | 203640 | AGCGAGTGAGTGAGTGCATTGGG |
| Chr2 | 230506241 | 196805 | GGTGAGCAAGTGTGTGTGTGTGG |
| Chr2 | 199628306 | 188735 | TGTGAGTGAGTGTGTGCAGAAGG |
| Chr10 | 109378067 | 180328 | GGTGAGTGAGTGAGTGAGTGAGG |
| Chr18 | 43287997 | 178553 | TGAGAGTGAGTGTGTGTATATGG |
| Chr2 | 183092036 | 176699 | GATGTGTGAGTGTGTGCCTGTGG |
| Chr15 | 92864212 | 168436 | TGTGAGTGAGTGTGTGTGTGTGA |
| Chr5 | 115434676 | 161900 | TGTGGGTGAGTGTGTGCGTGAGG |
| Chr9 | 18733635 | 156191 | AGCGAGTGAGTGTGTGTGTGGGG |
| Chr17 | 79111961 | 153074 | GGTAAGTGTGTGTGTGCATGTGG |
| Chr3 | 10403702 | 150578 | CATGAGTGGGTGTGTGCATTGGG |
| Chr8 | 48997806 | 147492 | GTAGAGTGAGTGTGTGTGTGTGG |
| Chr20 | 21927847 | 145142 | GAAGAATGAGTGTGTGCTTGTGG |
| Chr10 | 87387984 | 141970 | GGTGTGTGAGTGTGTGCATGTTG |
| Chr10 | 1684972 | 140632 | TGTGAGTGGGTGTGTGAGTGAGG |
| Chr11 | 7625795 | 134588 | GGTGAGTAGGTGTGTGTGTGGGG |
| Chr18 | 75912617 | 134342 | GGAGAGTGTGTGTGTGAGTGTGG |
| Chr6 | 24224744 | 129788 | GGTGAGCGTGTGTGTGCATGTGG |
| Chr2 | 18696225 | 129667 | AGTGAGAAAGTGTGTGCATGCGG |
| Chr1 | 203434970 | 129446 | CATAAGTGAGTGTGTGCGAGTGG |
| Chr10 | 130228354 | 127783 | AGGGAGTGACTGTGTGCGTGTGG |
| Chr1 | 152925734 | 124308 | TGTGAGTGTGTGTGTGCATCTGG |
| Chr3 | 14430297 | 124127 | GGTGAAGTGGTGTGTGCCTGTGG |
| Chr1 | 116485644 | 124043 | AATGAGTGAGTGTGTGAGTGAAG |

| | | | |
|---|---|---|---|
| Chr6 | 144458291 | 122623 | AGGGAGTGAGTGTGAGAGTGCGG |
| Chr1 | 32738764 | 120061 | GGGGTGAGTGTGTGTGTGGGGGG |
| Chr6 | 145090503 | 119609 | TGTGAGTGAATGTGTGCATATGG |
| Chr21 | 26653015 | 119496 | GGTGTGTGTGTGTGTGCATGTGG |
| Chr22 | 49740001 | 118564 | GGTGTGTGAGTGTGTGTGTGTGG |
| Chr19 | 47732492 | 116403 | CTGGAGTGAGTGTGTGTGTGTGG |
| Chr1 | 181204797 | 115862 | GGAGAGTGAGTGTGTTTGTGTGG |
| Chr16 | 49384711 | 114011 | TGTGTATGAGTGTGTGCGTTGGG |
| Chr17 | 47051410 | 113965 | AATGGGTGAGTGTGTGGGTGGGG |
| Chr15 | 71796660 | 113213 | AATGAATGAATGTGTGCATGTGG |
| Chr7 | 158305228 | 112748 | TGTGTGTGAGTGTGTGCATGTGG |
| Chr1 | 47690894 | 111112 | TGTGAGAGAGAGTGTGCGTGTGG |
| Chr8 | 128556646 | 109297 | TGTGAGTATGTGTGTGCATGTGG |
| Chr6 | 1587476 | 107804 | TGTGCATGAGGGTGTGTGTTGGG |
| Chr2 | 74655959 | 107266 | GGTAAGTATGTGTGTGCATGGGG |
| Chr7 | 51294279 | 106266 | AGTGAGTAAGTGAGTGAGTGAGG |
| Chr2 | 10373473 | 105950 | TGTGAGTGAATGAGTGCATGTGG |
| Chr11 | 63366342 | 105655 | AGTGAGTATGTGTGTGAGGGTGG |
| Chr21 | 44179977 | 104795 | TGTGAGTGGGTGTGTGCATGTGG |
| Chr4 | 168168030 | 104058 | GGTGTGTGTGTGTGTGTGTGTGG |
| Chr19 | 16569487 | 103866 | TGTGTGAGTGAGTGTGTGTGTGG |
| Chr16 | 87047314 | 103772 | AGTGAATGAGTGAGTGAGTGAGG |
| Chr3 | 193993884 | 103526 | AGTGAATGAGTGTGTGTGTGTGG |
| Chr8 | 92645411 | 103384 | GATGTGTGAGTGTGTACATGAGG |
| Chr11 | 78871125 | 103076 | AATGAGTGAGTGAGTGCATGGAG |
| Chr17 | 64940809 | 102789 | AGTGAATGAGGCTGTGCTTCGGG |
| ChrX | 56327306 | 101167 | TGTGAGTGTGTGTGTGCATGTGG |
| Chr22 | 43939297 | 100509 | GGTGAGAGAGTGTGTGCACGGGG |
| Chr4 | 154005628 | 99910 | TGTGAGTGTGTGTGTGCATGCAG |
| Chr21 | 43375271 | 98094 | GTGATGTGAGCGTGTGTGTGTGG |
| Chr16 | 46642109 | 98037 | AGAGAGTGAGTGAGTGAGTGTGG |
| Chr3 | 55318919 | 97636 | AGTGAGTGAATGAGTGCATAGTG |
| Chr3 | 10207131 | 96875 | GGTGTGTGTGTGTGTGTGTGTGG |
| Chr11 | 68651139 | 95585 | GGTGAGTGAGTGCGTGCGGGTGG |
| Chr1 | 212639778 | 95559 | GGGGAATGAGTGTGTGCATGGAG |
| Chr3 | 43415186 | 95395 | TCAGAATGAGTGTGTGCCTGGGG |
| Chr6 | 140710487 | 92344 | GGGAGGTGAGTGCATGCGTGTGG |
| Chr12 | 133361327 | 90593 | GGGGTGTGAGCATGTGCGTGTGG |
| Chr17 | 74046702 | 89136 | CGTGAGTGAGTGTGTGGTTGGGG |
| Chr18 | 6130265 | 88536 | TGTGAGTGAATGTGTGTGTGTGG |
| Chr14 | 106029032 | 87987 | GGTGAGTGAGTGTGTGTGTGAGG |
| Chr19 | 47787100 | 86825 | GATGAGTGTGTCTGTGCATGAGG |
| Chr3 | 1831002 | 86791 | ACTGAGTGGGTGTGTGCCTGAGG |
| Chr14 | 62078773 | 86236 | TGTGAGTAAGTGTGTGTGTGTGG |
| Chr1 | 48691305 | 85819 | AGTGTGTGAGAGTGTGCATGTGG |
| Chr19 | 40561867 | 83975 | ACTGTGTGAGTGTGTGCGTGAGG |
| Chr20 | 39096994 | 83171 | TGTATGTGAGTGTGTGCGTGTGG |
| Chr10 | 45209676 | 82764 | AGGTAGTGAGTGTGTGCATGGGT |
| Chr14 | 76750082 | 79666 | TGTGAGTGCGTGTCTGTGTGTGG |
| Chr16 | 84532 | 79700 | TATGAGTGTGTGTGTGAGTGTGG |

| | | | |
|---|---|---|---|
| Chr19 | 6660674 | 79444 | TGTGAGTGAGTGAGTGAATGTGG |
| Chr22 | 29329724 | 79139 | AGTGTGTGTGTGTGTGTGGGG |
| Chr4 | 5844313 | 78441 | TGTGAGAGAGTGTGTGAGTGTGG |
| Chr1 | 22117219 | 78182 | AGTGATGGAGTGTGTGCCTGTGG |
| Chr12 | 5100948 | 77679 | TGCATGTGAGTGTGTGTGCGTGG |
| Chr11 | 115758116 | 76545 | AGAGAGTGTGTGTGTGCTTGGGG |
| Chr18 | 73286082 | 76468 | CATGAGTGGGTGTGTGCGTGGAG |
| Chr1 | 236264583 | 76389 | TATGAGTGTGTGTGTGAATGTGG |
| Chr6 | 101025624 | 73050 | AGAGAGTGTGTGTGTGTGTGTGG |
| Chr7 | 101077901 | 71834 | TGTGAGTGAGTGTGTTGGTGAGG |
| ChrX | 38624688 | 71296 | TATGAGTGTATGTGTGCATAGGG |
| Chr5 | 22787253 | 70950 | GGTGTGTGTGTGTGTGTGTGTGG |
| Chr17 | 66592348 | 70915 | GGTGTGTGTGTGTGTGTGTGTGG |
| Chr10 | 5749657 | 70553 | AGTGAGTATGTGTGTGTGTGGGG |
| Chr2 | 217617270 | 70535 | AGGGAGTGAGTGTGTAAGTGTGG |
| Chr7 | 20263523 | 69959 | TGTGAGTGTATGTGTGTGTGTGG |
| Chr9 | 96679964 | 69839 | TGTGAGTGTGTGTGTGCATGTGA |
| Chr3 | 30904559 | 69551 | AGAGAGTGAGTGTGTGAGTGTGA |
| Chr4 | 62067619 | 69092 | GATGAGTGTGTGTGTGTGTGAGG |
| Chr17 | 72614843 | 68998 | GGGTGAGGAAGGTGTGCGTGGTG |
| Chr13 | 30280840 | 68632 | GATAAGTGAGTATGTGTGTGTGG |
| Chr20 | 62468987 | 67982 | AGTGAGTGAGTGAGTGAATGAGG |
| Chr11 | 63585151 | 67687 | AGAGAGAGAGTGTGTGCGTGTGA |
| Chr14 | 74353497 | 67524 | AGCGAGTGGGTGTGTGCGTGGGG |
| Chr3 | 150919004 | 67276 | AGAGAGAGAGTGTGTGCACGTGG |
| Chr3 | 38182513 | 66357 | TGTGAGTGAATGTGTGCCAGGGG |
| Chr16 | 23981202 | 66336 | GGTGTGTGTGTGTGTACGTGGGG |
| Chr11 | 12159168 | 66034 | TGTGTGAGTGTGTGTGTGGGGGG |
| Chr12 | 113240368 | 65974 | TGTGCGTGAGTGTGTGTATGTGG |
| Chr12 | 57612417 | 65969 | CTTGAGTGAGAGTGAGCGTGAGG |
| Chr3 | 80057064 | 65928 | GGTGTGTGTGTGTGTGTGTGTGG |
| Chr10 | 107867379 | 65724 | AGAGAGTGAGTGTGTGTGTTGGG |
| Chr21 | 39875948 | 65333 | AGTGTGTGAGTGTGTGTATGAGG |
| Chr10 | 105307473 | 65196 | TGAGTGTGAGTGTGTGCGTGGGG |
| Chr2 | 126931490 | 64648 | TGTGTGTGAGTGTGTGTGTGTGG |
| Chr9 | 23824554 | 64347 | TGTGGGTGAGTGTGTGCGTGAGA |
| Chr1 | 48305038 | 63571 | TGTGGGTGAGTGTGTGTGTGTGG |
| Chr22 | 33161120 | 61767 | AGCGAGAGAGTGTGTGAGTGTGG |
| Chr10 | 130236827 | 61760 | GGTGTGTGTGTGTGTGCGTGCGG |
| Chr6 | 54584099 | 61560 | GGTGTGTGTGTGTGTGTGTGTGG |
| Chr1 | 59647610 | 61476 | ACAGAGTGAGTGTATGTGTGGGG |
| Chr3 | 58727139 | 61458 | TGGTGATGAGTGTGTGTGTGTGG |
| Chr2 | 765652 | 61000 | TATGAATGTGTGTGTGCATGTGG |
| Chr18 | 50274481 | 60745 | GGTGTGTGAGTGAGTGAGTGCGG |
| Chr11 | 41554134 | 60452 | GGTGTGTGTGTGTGTGTGTGTGG |
| Chr5 | 21934229 | 59877 | TGTGTGTGAGTGTGTGTGTGTGG |
| Chr1 | 208239410 | 59583 | TGTGTGAGTGAGTGTGTGTGTGG |
| Chr5 | 150224721 | 59559 | AGTGAGAGTGTGTGTGTGGGGGG |
| Chr10 | 99685339 | 59057 | TGAGAGTGAGTGTGAGAGTGGGG |
| Chr6 | 89076647 | 58986 | TGTGAGTGTGTATGTGTGTGGGG |

| Chr12 | 592739 | 58963 | GGTGTAGTGGTGTGTGCCTGTGG |
|---|---|---|---|
| Chr13 | 90418142 | 58280 | TGTGTGTGTGAGTGTGTGTATGG |
| Chr11 | 40324874 | 58268 | TGTGTGTGAGTGTGTGCATGTGA |
| Chr18 | 23286405 | 57990 | CGTGAGTGTGTGTATGCGTGTGG |
| Chr4 | 7430877 | 57892 | CATGCGTGAGTGTGTGCATGGGG |
| Chr12 | 114752937 | 57500 | TGTGAGTGAGTGTGTGCATGTGA |
| Chr17 | 1176800 | 57499 | AGTGTGTGAGTGTGTGTGTGAGG |
| Chr3 | 194073652 | 57337 | ACTGAGTGAGTGTGAGTGTGAGG |
| Chr14 | 84459815 | 56913 | GGGTGAGATGTGTGTGCATGTGG |
| Chr20 | 25612568 | 56877 | GGTGTAGTGGTGTGTGCCTGTGG |
| Chr15 | 85046541 | 56714 | TGTGTGTGAGTGTGTGCATGTGG |
| Chr3 | 169379105 | 56450 | AGAGAGAGAGAGTGTGTGTGTGG |
| Chr2 | 238409544 | 56372 | GAAGAGTAAGTGTGTGTGTGGGG |
| Chr2 | 236425371 | 56133 | AGTGGATGAGTGAGTGCATGCGG |
| Chr11 | 8794152 | 55741 | GTAGAGTGAGTGTGAGAGTGTGG |
| ChrX | 42430834 | 55717 | AGTGAGTGAGTGTGAGCGTGAAG |
| Chr2 | 121087513 | 55543 | AGAGTGTGAGTGTGTGTGTGGGG |
| Chr16 | 86214365 | 55396 | GGGGTGTGAGTGTGGTGCGTGTG |
| Chr6 | 4373471 | 54966 | GGTGAGTGAGTGTGTGCGTGTGG |
| Chr1 | 238722563 | 54429 | GGTGTGTGTGTGTGTGCGTGTGG |
| Chr17 | 78954441 | 54363 | TGTGAGTGAGTGTGTGTGTGTGA |
| Chr8 | 20109735 | 53794 | AGTGTGTAAGTGTGTGAGTGGGG |
| Chr7 | 23276732 | 53659 | AGTGAGTGTGTGTGTGTGTTGGG |
| Chr2 | 124275984 | 53635 | AGTGAATGTGTGTGTGCATGTGG |
| Chr11 | 131931836 | 53621 | TGTGAGTGCGTGTGTGTGTGTGG |
| Chr5 | 139122257 | 53371 | TGTGAGTGCGTGTGTGAGTGTGG |
| Chr5 | 155118304 | 53029 | TGTGAGAGAGTGTGTGCATGTGA |
| Chr5 | 159012346 | 52995 | TGTGTGTGAGTGTGTGTATGTGG |
| Chr11 | 78932523 | 52603 | GGTGAGTTTGTGTGTGGGTGTGG |
| Chr13 | 60101377 | 52367 | GGTAAATGAGTGTGAGGCATGGG |
| Chr1 | 18868939 | 52267 | GGAGTGTGAGTGTGTGAGTGCGG |
| Chr5 | 178746539 | 51744 | TGTGAGTGAGTGCATGTGTGTGG |
| Chr6 | 129056545 | 51217 | TGTGTGTGAGTGTGTGTGTGTGG |
| ChrX | 99664994 | 50909 | GAAGAGTGAGTGTGTGGTGTGGG |
| Chr11 | 61485469 | 50776 | GCAGAGTGAGTGTGTGTGTTGGG |
| Chr4 | 187327499 | 50405 | CATGAGTGTGTGTGTGAGTGTGG |
| Chr9 | 95974277 | 50327 | TGTGAATGAGTGCGTGAATGGGG |
| Chr13 | 91706956 | 50092 | AGTGTGTGAGTGTGTGAGTGAGG |
| Chr2 | 73317050 | 49126 | GGTGAGTCAGTGTGTGAGTGAGG |
| Chr12 | 6937056 | 48851 | GGTGGATGAGTGTGTGTGTGGGG |
| Chr5 | 132802864 | 48840 | TGAGAGTGAGTGTATGAGTGTGG |
| Chr1 | 229141145 | 48503 | TGTGTGTGAGTGTGTACATGAGG |
| Chr2 | 123052110 | 48485 | GGTATTTGAGTGTGTACATGTGG |
| Chr2 | 171597348 | 48413 | ACTGACTGAGTGTGAGCATGTGG |
| Chr5 | 56989649 | 48026 | AATGAGTGTGTGTGTGTATGGGG |
| Chr7 | 117741100 | 47875 | GGTGTGTGTGTGTGTGCGTGTGG |
| Chr1 | 207097588 | 47546 | GTAGAGTGTGTGAGTGTGTGGCG |
| Chr10 | 28251664 | 46622 | GATGAGTGTGCGTGTGCATGAGG |
| Chr11 | 74904632 | 46613 | ACCAGGTGAGTGTGTGCGTGGGC |
| Chr14 | 28518304 | 46479 | TGTGAGTATGTGTGTGTGTGTGG |

27

| | | | |
|---|---|---|---|
| Chr10 | 95051225 | 45827 | CCTGAGCGAGTATGTGCATGTGG |
| Chr1 | 181557204 | 45772 | GGAGAGTGAGTGTGTGCATGTGC |
| Chr10 | 120245284 | 45770 | GGTGTGTGAATGTGTGTGTGTGG |
| Chr7 | 87667089 | 44986 | AGAAAGTGAGTGTGTGTATAAGG |
| Chr3 | 155092668 | 44566 | AGTGCATGAGTGTGTATGTGAGG |
| Chr12 | 31106567 | 43922 | GCTGAGTGTGTGTGTGCGTGTAG |
| Chr20 | 2780911 | 43695 | GGTGAGTGAGCGAAGGAGTAGGG |
| Chr6 | 107510883 | 43442 | TGTGAGTGTGTGTGTGAGTGTGG |
| Chr2 | 81220097 | 43319 | TGTGAGTGTATGTGTGTGTGTGG |
| Chr20 | 36039815 | 43235 | TATGAGTGTGTGTGTGCACGTGG |
| Chr1 | 4770493 | 43006 | TGGGTGTGAGTGTGTGCGTGTGG |
| Chr14 | 102953779 | 42717 | TGTGAGTGTGTGTGTGCGTGCGC |
| Chr5 | 23562308 | 42040 | AGAGAGAGAGTGTGTGTGTGTGG |
| Chr11 | 62781473 | 41650 | CATGAGTGACTGTGTGTGTGTGG |
| Chr21 | 30993730 | 41270 | GGTGTGTGTGTGTGTGTGTGGGG |
| Chr19 | 56497640 | 41146 | TGTGAGTGTGAGTGTGTGTTGGG |
| Chr15 | 37202049 | 41005 | TGTGTGTGGGGGTGGGGGTGGGG |
| Chr19 | 41713254 | 40809 | AGTGAGTGTGTATGTGTGTGTGG |
| Chr3 | 184590078 | 40193 | AATGAGTGTGTATGTGTGTGTGG |
| Chr13 | 101257208 | 40117 | TTTGAGTGTGTGTGTGCATGAGG |
| Chr11 | 133611177 | 39673 | TGCGTGTGAGTGTGTGCGTAGGT |
| Chr10 | 99306651 | 39637 | AGAGAGAGAGTGTGTGTGTGAGG |
| Chr10 | 61044507 | 39573 | GGGGTAAGGGTGTGTGTGTGTGG |
| Chr17 | 10029642 | 39200 | TGTGTGTGAGCGTGTGTGTGTGG |
| Chr5 | 149501694 | 39132 | GATGAGTGAGTGTGTGAGTGAGA |
| Chr2 | 174931405 | 39132 | GGTGTGAGAGTGTGTGCGGAGGC |
| Chr4 | 168057437 | 39128 | TGTGTGTGAGTGTGTGTGTGTGG |
| Chr2 | 88996016 | 39077 | GATGAGTTTGTGTGTGTGTGGGG |
| Chr11 | 44999873 | 38823 | TGTGAGAGAATGTGTGCGTGTGA |
| Chr8 | 135523492 | 38820 | TGAGAGTGAGAGTGTGTGTGGGG |
| Chr19 | 40596585 | 38681 | GGACTGTGAGTGTGTGCGTGAGG |
| Chr18 | 60759565 | 38462 | TGTGAGTGGGTGTGTGTGTGTGG |
| Chr19 | 48782757 | 38450 | TGTGAGTGTGTGTGTGGGTGGGG |
| ChrX | 41726218 | 38335 | GGTGAGTGAGTGAGTGAGTGAGG |
| Chr11 | 1004348 | 38204 | GGTGTAGTGGTGTGTGCCTGTGG |
| ChrX | 105614415 | 37642 | AGTGAATGAGTGTGTGCATGTGA |
| Chr7 | 77128126 | 37477 | TGTGTATGAGTGTGTGTATGCGG |
| Chr2 | 16837556 | 37405 | TGTGAGTGGGTGTGTGGGTGTGG |
| Chr8 | 121823447 | 37394 | TGAGTGTGAGTGTGAGCGTGCGG |
| Chr7 | 31100113 | 37187 | TGTGAAGGAGTGTGTGTGTGTGG |
| Chr16 | 88218507 | 37056 | ATTGTGTGAGTGTGTGCATGTGG |
| Chr4 | 7132480 | 36475 | TGTGGGTGTGGATGTGTGTGTGG |
| Chr12 | 129149692 | 36397 | TATGTGTGAGTGTGTGCATATGG |
| Chr4 | 183729842 | 36229 | TGTGGGTGGGTGTGTGCGTGTGG |
| Chr10 | 98760588 | 36228 | GTTGAGTGAATGTGTGCGTGAGG |
| Chr3 | 172121469 | 36168 | GGGAAGGGAGTGTGTGCATGGGG |
| Chr2 | 4734730 | 36144 | GGGGAATGAGTGTGTATGTGAGG |
| Chr5 | 31640966 | 35357 | AGTGAGTGTGTGTGTTGCGGGGG |
| Chr10 | 107228008 | 35025 | GGTGTGTGTGTGTGTGTGTGTGG |
| Chr16 | 23869051 | 34306 | AGAGAGTGTGTGTGTGTGTGTGG |

| Chr19 | 54524100 | 34299 | TGAGTGTGTGTGTGTGCGTGTGG |
|-------|----------|-------|-------------------------|
| Chr5 | 134817941 | 34058 | CATGAGTGTGTGTGTGCTTGTGG |
| Chr17 | 50130332 | 33753 | GTGAGTGATGTGTGTGTGTGTGG |
| Chr11 | 75330150 | 33458 | TGTGTGTGAGTGTGTGCATGAGG |
| Chr13 | 110882529 | 33303 | TGTGTGTGAGTGTGTGCCCGTGG |
| Chr5 | 84905674 | 32861 | TGTGTGTGAGTGTGAGTGTGTGG |
| Chr8 | 9768212 | 32615 | AGAGAGAGAGTGTGTGTGTGTGG |
| Chr12 | 124763151 | 32224 | TGTGAGTGTGTGTGTACCTGGGG |
| Chr6 | 43905520 | 32218 | GGTGTAGGAGTGTGTGTGTGGGG |
| Chr20 | 31382040 | 31490 | GGTGAGGTGGTGTGTGCCTGTGG |
| Chr16 | 73585926 | 31285 | AATGAGTGAGTGTGTGTGTGTGA |
| Chr11 | 69518904 | 31172 | GGGGTGTGAGTGGGTGTGTGCGG |
| Chr12 | 131196667 | 31067 | GGTGGGTGAGTGAGTGAGTGAGG |
| Chr4 | 158621598 | 31029 | AGTGTATGAGTGTTTGCATGGGG |
| Chr7 | 134234248 | 30738 | AGTGAGTGAGTGAGTGAATGTGG |
| ChrX | 30439128 | 30450 | TGTGAGTGTGTGTGTGTATGTGG |
| Chr5 | 73855632 | 30379 | GGTGTGTGAGAGTGTGTATGTGG |
| Chr5 | 146520400 | 30071 | GGTGTGTGGGTGTGTGTGTGGGG |
| Chr12 | 125156261 | 29909 | GATGAGTGTGTGTGTGTGTGCGG |
| Chr15 | 80907957 | 29859 | TGTGAGTGTGTATGTGTGTGTGG |
| Chr14 | 78443706 | 29808 | TGTGTGTGTGTGTGTGTGTGTGG |
| Chr1 | 18837923 | 29595 | GGTGTGTGTGTGTGTGTGTGTGG |
| Chr1 | 35189392 | 29530 | TGTGTGTGAGTGTGTGTGTGGGG |
| Chr18 | 6110703 | 29521 | AGGATGTGAGTGTGTGCATGTGG |
| Chr12 | 33270686 | 29418 | GGAGAATAGGTGTGTGCGTGGGG |
| Chr8 | 141037928 | 29408 | AGTGAGTGTGTGTGTGAAGGAGG |
| Chr16 | 26809933 | 29366 | GATGAGTAAGTGTCTGAGTGGGG |
| Chr8 | 21494840 | 29292 | TGTGAGTGTGTGTATGCGTGTGA |
| Chr7 | 121687676 | 29255 | TGTGTGTGAGTGTGTGTGTGTGG |
| Chr9 | 29602720 | 29089 | GGGGTGTGTGTGTGTGTGTGTGG |
| Chr6 | 105265269 | 29056 | AGAGAGAGAGTGTGTGCAAGGGG |
| Chr10 | 43251651 | 29026 | GTAGGGTGGGAGTGTGTGTGTGG |
| Chr8 | 139883090 | 28455 | TGTGAGTGGGTGTGTATGTGAGG |
| Chr16 | 10276764 | 28379 | GGCGAGTGTGTGTGTGAGTGTGG |
| Chr14 | 90885641 | 28211 | GATGTGTGTGTGTGTGCGTGTGG |
| Chr6 | 33999846 | 27544 | TGTTAGTGAGTGTGTGCAGGTGG |
| ChrX | 39606149 | 27511 | GATGAGCGAGTGTGTGTGTATGG |
| Chr17 | 6891149 | 27499 | GGTGAAAGAGTATGTGTGTGTGG |
| Chr2 | 240564198 | 27202 | GGTGTGTATGTGTGGGGGTGTGG |
| Chr1 | 3325807 | 27195 | GGTGTGAGAGTGTGTGAGTGGGG |
| Chr12 | 2469347 | 27066 | GGGGTGTGTGTGTGTGTGTGTGG |
| Chr6 | 24574540 | 27056 | GGTGTAGTGGTGTGTGCCTGTGG |
| Chr1 | 175049116 | 26933 | TGTGAGTGTGTGTGTGTGTGTGG |
| Chr3 | 3697106 | 26689 | GGTGTGTGTGTGTGTGTGTGTGG |
| Chr7 | 39341125 | 26138 | GGTGTGTGAGTGTGTGTGTGTGA |
| Chr20 | 23960933 | 26077 | GGTATGTGAGTGTGAGTGTGGGG |
| Chr19 | 54375904 | 26077 | GGTGTGGTGGTGTGTGCGTGTGG |
| Chr7 | 31353825 | 25742 | CCAGAATGAGTGTGTGTGTGTGG |
| Chr3 | 79455732 | 25729 | TGTGTGTGAGTATGTGTGTGTGG |
| Chr2 | 126515435 | 25686 | TGTGAGTGAATATGTGTATGTGG |

| Chr4 | 82574191 | 25545 | GGTATGTGAGTGTGTGTATATGG |
| Chr1 | 3002774 | 25443 | GGTGAGCTCGTGAGTGCGTGAGG |
| Chr17 | 43132890 | 25361 | AAGTGAGGAGTGTGTGCCTGTGG |
| Chr18 | 74103175 | 25153 | GGTGAGTAAGTGTGAGCGTAAGG |

[Table 6]

| | | EMX1 | |
|---|---|---|---|
| Chr | Position | DNA cleavage Score | DNA seq at a cleavage sites |
| Chr11 | 62365273 | 772387 | GAATCCAAGCAGAAGAAGAGAAG |
| Chr19 | 1438827 | 597313 | GAAGTAGAGCAGAAGAAGAAGCG |
| Chr2 | 73160999 | 589285 | GAGTCCGAGCAGAAGAAGAAGGG |
| Chr1 | 234492864 | 494830 | GAAGTAGAGCAGAAGAAGAAGCG |
| Chr2 | 172374203 | 403017 | GAAGTAGAGCAGAAGAAGAAGCG |
| Chr2 | 219845073 | 382588 | GAGGCCGAGCAGAAGAAAGACGG |
| Chr1 | 23720618 | 374603 | AAGTCCGAGGAGAGGAAGAAAGG |
| Chr12 | 106646091 | 294995 | AAGTCCATGCAGAAGAGGAAGGG |
| Chr15 | 44109764 | 284237 | GAGTCTAAGCAGAAGAAGAAGAG |
| Chr8 | 128801260 | 279063 | GAGTCCTAGCAGGAGAAGAAGAG |
| Chr15 | 22366622 | 233100 | GGAGTAGAGCAGAGGAAGAAGGG |
| Chr9 | 127309505 | 222741 | AAGCCCAAGCAAATGAAGAATGG |
| Chr11 | 43747949 | 217179 | AAGCCCGAGCAAAGGAAGAAAGG |
| Chr5 | 9227163 | 213293 | AAGTCTGAGCACAAGAAGAATGG |
| Chr10 | 128080196 | 196235 | GAGTACAAGCAGATGAAAAACGG |
| Chr1 | 33608480 | 192057 | GAGCCTGAGCAGAAGGAGAAGGG |
| Chr3 | 34042974 | 178213 | GAGTTCAAGCAGAGAAGAAAGGG |
| Chr21 | 24667742 | 166991 | CCCTCCAAGCAGAAGAAGATGAG |
| Chr14 | 38654666 | 164832 | AAGTCTGAGAAGAAGAAGACATG |
| Chr5 | 45359067 | 163490 | GAGTTAGAGCAGAAGAAGAAAGG |
| Chr18 | 68861321 | 160118 | AGAGTAGAACAGAAGAAGGAAAG |
| Chr4 | 87256692 | 159096 | GAGTAAGAGAAGAAGAAGAAGGG |
| Chr10 | 58848729 | 156786 | GAGCACGAGCAAGAGAAGAAGGG |
| Chr8 | 74634212 | 155436 | AAGTCCAAAAAGAAGAAAAAAGG |
| Chr19 | 24250503 | 146744 | GAGTCCAAGCAGTAGAGGAAGGG |
| Chr8 | 105164126 | 143429 | GAGCCCAAGAAGAAGAAGAAGGA |
| Chr14 | 43156807 | 141435 | GAGGCCAAGCAGAAAAAAAATGG |
| Chr11 | 30301809 | 140265 | CAGTCTGAGTAGAAGAAAAAGGG |
| Chr4 | 58838593 | 138462 | AAGTCCCAGAAGAAGAAATATGG |
| Chr15 | 65614525 | 136049 | GAGAGGGAGCAGGGAGAAGAAGG |
| ChrX | 149010707 | 135051 | GGAGTCAAGGAGAAGAAGAAGAG |
| Chr7 | 111475449 | 130150 | GAGCCCAAGCACAAAAAGAATGG |
| Chr14 | 48932120 | 129466 | GAGTCCCAGCAAAAGAAGAAAAG |
| Chr10 | 91416162 | 127813 | ATGTCCAAGCAGAAGAAGTCTGG |
| Chr12 | 102743729 | 121776 | TAGTCAGAGCAGATGAAGGAAAG |
| Chr3 | 95690186 | 117619 | TCATCCAAGCAGAAGAAGAAGAG |
| Chr3 | 16077518 | 117265 | GAGGCAGAGAGAAAGAAGAAAGG |
| Chr12 | 73504675 | 105719 | GAGTTAGAGCAGAAAAAAAATGG |
| Chr6 | 9118799 | 104220 | ACGTCTGAGCAGAAGAAGAATGG |
| Chr5 | 141129457 | 103516 | AAGTCACAGGAGCAGAAGAAGA |
| Chr7 | 31901077 | 103321 | GAGGCCAAGCAGAAAGAAAAAGG |
| Chr1 | 209931600 | 102834 | TTATCCGAGAAGAAGAAGTAAGG |
| Chr6 | 106901799 | 102469 | GAGCCGGAGCAGGGAGAAGAAGG |
| Chr22 | 34858917 | 102348 | GAGCCTGAGCAGGAAGAAGAAGA |
| Chr6 | 29806752 | 102267 | TGTCCAAGGCAGGAGAAGAAGGG |
| Chr15 | 36839471 | 99856 | CAGAGAGAGGAGCAGAAAAAAGA |
| Chr5 | 134375867 | 96143 | CAGGCTGAGCAGAAAGAAGAAAG |
| Chr2 | 199919487 | 95742 | GAGTCAGAGCAGAACTAGAAGGG |

| Chr20 | 6653999 | 95723 | AAGTCCAGACAGAAGAAGAAGGA |
|---|---|---|---|
| Chr8 | 135098073 | 94515 | CAGTCCAGCAGGAAGAAGAGAGG |
| Chr11 | 131106371 | 90172 | GCCTCCAAGCAGAAGGAGAAATG |
| Chr9 | 2513258 | 90018 | GAGAGAGAGCAAAAGGAAGAATG |
| Chr17 | 72057114 | 89855 | GAGGAGAGCAGAAAGAAGAAGGG |
| Chr16 | 56184077 | 88757 | AAGTCAGAGAAGGAAGAAGAAAG |
| Chr5 | 146833190 | 88608 | GAGCCGGAGCAGAAGAAGGAGGG |
| Chr5 | 120294736 | 88489 | ATGTCCAAGCACAAGAGGAATGG |
| Chr1 | 113741471 | 87189 | GAGGTAGAGCAGAAGAAGAAGCG |
| ChrX | 38971206 | 86924 | GAGTCCCAGAAGAAGAAAGAAAG |
| Chr4 | 2181662 | 86342 | CCTCTCGAGCAAAAGGAAGAAGG |
| Chr14 | 75723908 | 78355 | AGTTCCAAGCAGAGGAAGAAGGG |
| Chr4 | 155734338 | 77475 | TGCTTTGAGCAGAAAGAAGAAAG |
| Chr4 | 122686219 | 76915 | AAGTAAGAAGAGCAGGAAGAAGA |
| Chr12 | 4927416 | 75200 | TAGTCCTAGCAAGAATAAGAATG |
| Chr3 | 5031614 | 73504 | GAATCCAAGCAGGAGAAGAAGGA |
| Chr2 | 106719739 | 73041 | TAATGAGAGCAGAAAGAAGAATG |
| Chr7 | 142597224 | 72663 | GACAGAGAAGAGAAGAAGGAAGA |
| Chr1 | 27913391 | 72320 | AGGTCAGAGCAGAAGAAAAGAGG |
| Chr7 | 73602675 | 71804 | GCAAAGAGCAGGAAGAAGAAGGG |
| Chr18 | 34906762 | 71062 | GAGCCTGAGCGGAAGAGGAAAGG |
| Chr2 | 45607957 | 69584 | TAATCCCAGAGCAGGAAGAAGAA |
| Chr18 | 1677040 | 69087 | AGTCCAGAGCAAAATAAGAAGGG |
| Chr4 | 44622977 | 68873 | AAGTCTGAGAAGAAGAAGAAAGA |
| Chr12 | 2873991 | 68800 | GCTAAAGAGCAGAAGGAAGAAGG |
| Chr2 | 239393515 | 68020 | CAGTACGAGCAGAGGAAGGAAGA |
| Chr8 | 102244552 | 66479 | AGTTCCAAGCAGAAGAAGCATGG |
| Chr2 | 66582071 | 66179 | ATGGCAGAGCAGAAAGAAGAAAG |
| Chr11 | 69660352 | 62977 | CAGTCCATGCAGAGGGAAGAAGG |
| Chr11 | 130764292 | 62968 | GCATTAGAGCAGAAGGAAGAAGG |
| Chr1 | 231750743 | 61748 | GAGTCAGAGCAAAAGAAGTAGTG |
| Chr6 | 36604882 | 60741 | GGCAGAGAGCAGAAGGAAGAAAG |
| Chr15 | 61646878 | 60004 | AAGTCAGAGGAGAAGAAGAAGGG |
| Chr7 | 141972562 | 58917 | AAGTCCGGGCAAAAGAGGAAAGG |
| Chr12 | 111418051 | 58806 | GAGAGGGAGCAAAAGAAGGAAGG |
| Chr9 | 72899757 | 57967 | CAGAATGAGCAGGAAGAAGAACA |
| Chr17 | 8640231 | 56884 | GAGACTGAGAAGAAGAAGAAAGG |
| Chr1 | 84869216 | 56816 | GAGTCAGCTGAGCAGAAGGAAGA |
| Chr4 | 41187173 | 56700 | GAAGGAGAGCAGAAAGAAGAAAG |
| Chr9 | 130107853 | 53625 | GTTTGAGAGCAGAAGGAAGAAGA |
| Chr11 | 118816273 | 53228 | ATTTCCAAGCAGAGAGAAGAATG |
| Chr8 | 72462455 | 52761 | GAGTCCGAGAAGAAGAAAGAAAA |
| Chr1 | 221522625 | 50986 | GAGTTTGAGTAGAAGAAGAAGAG |
| Chr21 | 37132446 | 49332 | TGGCCAGAGCAGAAGGAAGAAGG |
| Chr2 | 217972073 | 49031 | TGTCCGAGGCAGTAGAAAGAACG |
| Chr5 | 35927682 | 48391 | AAGCCCGAGCTAGAAGAAATAGG |
| Chr3 | 157623637 | 46601 | AAGGGGAGCAGGAAGAAGAAAGG |
| Chr20 | 14924870 | 46219 | AAGAAGGAGCAGGAAGAAGAAAG |
| Chr4 | 48639408 | 44366 | CACTCCAAGTAGAAGAAGAAAAG |
| Chr9 | 91487902 | 43847 | GAGGCAGAGAGAAGAAAGAAGGG |

| | | | |
|---|---|---|---|
| Chr2 | 105425353 | 43348 | AGATCCAAACAGAAGGAAGAATG |
| Chr7 | 100895242 | 43128 | CGCTCCGAGCAGAAGAAAAGTGG |
| Chr7 | 93390477 | 42514 | AGTCCTGAGCAGAGGAAGGAATG |
| Chr1 | 179024805 | 42398 | GAGTCCAAGAAGAAGAAGCCAGG |
| Chr17 | 54421043 | 42361 | GAGTCCCAGGAGAAGAAGAGAGG |
| Chr8 | 108409228 | 42088 | TGTTGAGAGCAGAAAGAAGAAAG |
| Chr15 | 68455211 | 42027 | GTCCAAAGGCAGGAGAAGAAGGG |
| Chr14 | 88550473 | 41703 | GAGGGAGAGAGCAGGAAGAAGAA |
| Chr12 | 124551806 | 41457 | TTGTTGAGCAGGAAGAAGAATGG |
| Chr18 | 32722290 | 41419 | TGTCCAGAGCAGATGAAGAATGG |
| Chr7 | 97319990 | 41090 | GAATCCAAGCAGAAGAAAATGGA |
| Chr7 | 3812781 | 40762 | GAGTCCTAGAAAAAGAAGAGAGG |
| Chr11 | 36270410 | 39031 | GAGAGAGAGCAGAAGAAGTAGAG |
| Chr18 | 25950253 | 38508 | AGGCCTGAGCAGAAGGAAGAAGG |
| Chr15 | 100292479 | 38402 | AAGTCCCGGCAGAGGAAGAAGGG |
| Chr3 | 169381222 | 38279 | GAGGGAGAGCAAAAGAAGGAAAG |
| Chr5 | 74513307 | 37749 | GTCCATAGCAAGAAAAAGAAGGG |
| Chr2 | 238373187 | 37583 | AGTGCAGAGCAGAAGAAGGAAAG |
| Chr7 | 70109967 | 37116 | GAATCAGAGCAAAAGGAGAAAGG |
| Chr6 | 110491414 | 36961 | AAGTCAGAGCAGAAAAAGAGAGG |
| Chr1 | 151027598 | 36487 | TTCTCCAAGCAGAAGAAGAAGAG |
| Chr9 | 135663404 | 35979 | CAGTCCAAACAGAAGAGGAATGG |
| Chr6 | 147955462 | 35474 | TGGCCAGAGCAGAAGGAAGAAAG |
| Chr9 | 140936012 | 34365 | GAGTCAAAGCAGAAGAAAGAACG |
| Chr14 | 35092801 | 33826 | TATCCAAGCAGGAAGAAGCAAGG |
| Chr17 | 73339913 | 33391 | TGCACGAGCAGGGAGAAGAAAGG |
| Chr4 | 82567700 | 33038 | TATTTACAGAGCAGGAAGAAGAG |
| Chr14 | 98020018 | 32807 | CATTCCAAGCAGAAGGAAGAGAG |
| Chr9 | 119853407 | 32546 | TACCAGGAGCAGGAAAAAGAAGG |
| Chr7 | 29268537 | 31836 | GAGCGGGAGCAAAAGGAAGAATG |
| Chr3 | 9802191 | 30997 | GTACCCAAGCAGAAGGAAGAAGG |
| Chr18 | 24570836 | 30752 | CCTGAAGAGCAGAAGGAGGAAGG |
| Chr13 | 101018849 | 27972 | GTCTGAGCAGAAAGGAAGAAGGG |
| Chr10 | 8337281 | 27943 | GAAGTCAGACAGAAGAAGAAGAG |
| Chr15 | 68619369 | 27871 | GAGAAAGAGCAGAAGGAAGAAGT |
| Chr2 | 218378108 | 27737 | GAGTCTAAGCAGGAGAATAAAGG |
| Chr1 | 2744291 | 27717 | GGTCCAGAGAGAAAGAAGAAAGG |
| Chr16 | 78848850 | 27402 | AAATCCAACCAGAAGAAGAAAGG |
| Chr10 | 5401788 | 27266 | TAATCCAATCAGAAGAAGAAGGG |
| Chr11 | 30490142 | 26821 | GAGAGAAGCAGAAAGAAGAAAGG |
| Chr17 | 21133222 | 26641 | GAATCCCAGCAGAAAGGAAGAAA |
| Chr6 | 12210833 | 26330 | ATGAATGAGCAGAAGGAGGAAAG |
| Chr7 | 43259054 | 26202 | GATACCGAGCTAAAGAAGGAAGG |
| Chr22 | 47725583 | 25746 | GAAGAGGAGCAGAAGGAGGAAGG |
| Chr11 | 56910170 | 25694 | ACCTGGGAGCAGGAAAAAGAAGG |

[Table 7]

| FANCF | | | |
|---|---|---|---|
| Chr | Position | DNA cleavage Score | DNA seq at a cleavage sites |
| Chr7 | 102131659 | 1143904 | GTCTCCCCTTCTGCAGCACCAGG |
| Chr11 | 22647336 | 880988 | GGAATCCCTTCTGCAGCACCTGG |
| Chr10 | 73463136 | 820158 | TGAATCCCATCTCCAGCACCAGG |
| Chr10 | 37953200 | 740976 | GGAGTCCCTCCTACAGCACCAGG |
| Chr10 | 43410031 | 602472 | GGAGTCCCTCCTACAGCACCAGG |
| Chr7 | 44076497 | 564535 | GTCTCCCCTTCTGCAGCACCAGG |
| Chr18 | 8707528 | 471495 | GGAACCCCGTCTGCAGCACCAGG |
| Chr16 | 49671025 | 438282 | GGAGTCCCTCCTGCAGCACCTGA |
| Chr3 | 35113165 | 408220 | TGAATCCTAACTGCAGCACCAGG |
| Chr7 | 102230832 | 340386 | GTCTCCCCTTCTGCAGCACCAGG |
| Chr20 | 44903573 | 337872 | TCCATCCCTACTGCCAGCACCAG |
| Chr11 | 66475045 | 330843 | GGAACACCTTCTGCAGCTCCAGG |
| Chr3 | 10885901 | 316520 | ACCCTCCCTTCTGCAGGCACCGG |
| Chr10 | 3151994 | 278757 | CTCTGTCCTTCTGCAGCACCTGG |
| Chr17 | 39675789 | 267600 | GGGAGTCCATCTGCAGCACCAGG |
| Chr11 | 47554037 | 192902 | GGAATCCCTTCTACAGCATCCTG |
| Chr2 | 54853314 | 174124 | GGAATATCTTCTGCAGCCCCAGG |
| Chr12 | 115467808 | 171967 | AGGGTCCCTTCTGCAGCCCCTGG |
| ChrX | 86355180 | 161944 | ACCATCCCTCCTGCAGCACCAGG |
| Chr2 | 35692938 | 130816 | ACTTTATCTTCTGCAGCACCTGG |
| Chr2 | 133174786 | 114095 | ATCCTTTCTTCTGCAGCACCTGG |
| Chr12 | 2719895 | 106450 | ACACTCCCTTCTGCAGCACCATG |
| Chr16 | 28615201 | 99927 | GGCTTCCCTTCTGCAGCCCCAGG |
| ChrX | 97566910 | 97653 | TGTATTCCTTCTGCAGGCACCAG |
| Chr17 | 30452575 | 89120 | CCTGCTGCTTCTGCAGCACCTGG |
| Chr9 | 113162294 | 88941 | AAAATCCCTTCCGCAGCACCTAG |
| Chr4 | 53092937 | 71120 | AATATTCCCTCTGCAGCACCAGG |
| Chr3 | 97725864 | 69246 | ACCATTTCTTCTGCAGCACCTGG |
| Chr17 | 34965068 | 67667 | GGGTCCGCTTCTGCAGCACCTGG |
| Chr9 | 111467809 | 52903 | AGGAATCCTACTGCAGCACCCAG |
| Chr4 | 76159966 | 48719 | TTACTCACTTCTGCAGGCACCTG |
| Chr5 | 68338523 | 47347 | CCACTCCTTTCTGCAGCACCCGG |
| Chr17 | 3980376 | 46215 | GGAACCCCCTCTGCAGCTTCTGG |
| Chr20 | 19790626 | 41979 | CATTTTCTTTCTGCAGCACCTGG |
| Chr17 | 78923978 | 41804 | AGAGGCCCCTCTGCAGCACCAGG |
| Chr8 | 71384404 | 40000 | TTTCCTGCTTCTGCAGCACCAGG |
| Chr17 | 39655735 | 37576 | GCCCCCTCCTCTGCAGCACCTGG |
| Chr6 | 41457565 | 33918 | CTCCTCCCTCCTGCAGCACCTGG |
| Chr13 | 109802140 | 32087 | AAAATACCTTCTGCAGTACCAGG |
| Chr5 | 85696042 | 30501 | CTACTGACTTCTGCAGCACCTGG |
| Chr4 | 7336836 | 30249 | AGCTCCCATTCTGCAGCACCCGG |
| Chr9 | 139891539 | 29763 | AGTTCCCCATCTGCAGCACCAGG |
| Chr10 | 13436789 | 28672 | CTCATCCCTTCTGCAGCCCCAGG |
| Chr2 | 176121765 | 26781 | GCCCCCTGCTCTGCAGCACCCGG |
| Chr13 | 100011450 | 26724 | ACCTGCCTTCTGGCAGCACCAGG |
| Chr17 | 8432606 | 25254 | ACTGTCATTTCTGCAGCACCTGG |

[Table 8]

| RNF2 | | | |
|---|---|---|---|
| Chr | Position | DNA cleavage Score | DNA seq at a cleavage sites |
| Chr1 | 185056773 | 695836 | GTCATCTTAGTCATTACCTGAGG |
| Chr12 | 131619805 | 123134 | ATCACCTTAGCCATTACCAGGGG |
| Chr18 | 62061226 | 98156 | ATTATTTTAGTCATTACCTTTGG |
| Chr8 | 99451745 | 82293 | CGTGCATTAGTCATTACCTGAGG |
| Chr7 | 43967267 | 70140 | ACTTATTTAGTCATTACCTGTAG |
| Chr6 | 143212079 | 67376 | GTAATATTAGTCATTACCGGTGG |
| Chr4 | 171989625 | 59218 | TAACACATAGTCACTACCTGGTG |
| Chr8 | 5745882 | 57546 | AATATGTTAGTCATTACCTGAGG |
| Chr4 | 189725512 | 34869 | ATGCTTCTTGTCATTACCTTGGG |
| Chr9 | 138721895 | 31931 | ACTTCAGTAGTCATTACCTAGGG |
| Chr5 | 92036966 | 29732 | GGTATCTAAGTCATTACCTGTGG |
| Chr15 | 25135467 | 35423 | CATCTAATAGTAATTACCTGGGG |
| Chr17 | 53928586 | 32362 | GTCATCTTAGTCATTAC-TGAGG |

[Table 9]

| HEK293-1 | | | |
|---|---|---|---|
| Chr | Position | DNA cleavage Score | DNA seq at a cleavage sites |
| Chr1 | 201992441 | 982832 | GGGAAAGTCCCAGCATCCTTTGG |
| Chr9 | 110103705 | 840188 | GGGAAAGACCCAGCATCCGTGGG |
| Chr22 | 47970525 | 773256 | GGAAAAGACCAAGCATCAGTGGG |
| Chr8 | 21121524 | 663039 | GGGAAGGACCCAGCATCCTGGGG |
| Chr9 | 129621088 | 618782 | GGGAAATACCCAGCATCCAATGG |
| Chr10 | 123094947 | 317069 | GGGAAAAGCCCAGCATCCCTTGG |
| Chr10 | 86303625 | 261270 | TGGAAAGAAACAGCATCCGTACG |
| Chr11 | 75956264 | 204471 | TTATAAGACCCAGCATCCGTAAG |
| Chr12 | 5555206 | 197869 | GGAGAAAGACCAGCATCCATAGG |
| Chr4 | 8541711 | 115848 | GGGAAATCCCCAGCATCTCTAGG |
| Chr5 | 65217522 | 89183 | GGAAAGTACACAGCATCCATCGG |
| Chr1 | 3488120 | 79221 | GGGGAAGACCCAGCACCCTTGGG |
| Chr13 | 31633478 | 74016 | ATGAAAGACCCAGCATCCATTGA |
| Chr19 | 30027761 | 30738 | AGGATTCCCCAAGCATCCGTGGG |
| Chr8 | 48879627 | 28382 | GAGAAAAGCCCAGCATCCTTAGG |
| Chr2 | 85388185 | 25657 | GGGAATACACCAGCATCCGTAGA |

[Table 10]

| | | HEK293-2 | |
|---|---|---|---|
| Chr | Position | DNA cleavage Score | DNA seq at a cleavage sites |
| Chr5 | 87240614 | 486955 | GAACACAAAGCATAGACTGCGGG |
| Chr4 | 90522184 | 377451 | GAACACAATGCATAGATTGCCGG |
| Chr10 | 43159340 | 336031 | AACACAAAGACATAGACCACTGG |
| Chr1 | 40461425 | 300007 | TACACACAAGCACAGACTGCAGG |
| Chr2 | 19844956 | 252268 | AACTCCAAAGCATATACTGCTGG |
| Chr15 | 93557679 | 248075 | AGAACACATGCATAGACTGCTAG |
| Chr11 | 128508577 | 243706 | GAATTCAAAGCATAGATTGCAGG |
| Chr5 | 131174461 | 212455 | AAATACAATGCATAGACTGCTAG |
| Chr19 | 30112719 | 209645 | TACACAAAACATAAGACTGCTGG |
| Chr20 | 97641 | 192351 | GAATTCAAAGCATAGATTGCAGG |
| Chr4 | 53536210 | 179748 | GAATACTAAGCATAGACTCCAGG |
| Chr13 | 113428467 | 171254 | CAATACAAAGGATAGACTGCAGG |
| Chr18 | 22360702 | 167163 | GGAATCAAAGCACAGACTGCAGG |
| ChrX | 36949815 | 154618 | GAAAACAAAACATAGAGTGCTGG |
| Chr7 | 83764327 | 113259 | ACTATATAAGCATAGACTGTTGG |
| Chr5 | 126385455 | 107283 | CCACACCAAGCATAGACTTCTGG |
| Chr5 | 142142325 | 106923 | TAAACACTAACATAGACTGCAGG |
| Chr18 | 56307003 | 85167 | AAGAACAAAACATAGACTGCAGG |
| Chr1 | 36097073 | 85043 | GTAAACAAAGCATAGACTGAGGG |
| Chr3 | 81710854 | 85027 | CTCCTAAAGCATTAGACTGCAGG |
| Chr5 | 7625837 | 63687 | GGTACACAATAATAGACTGCAGG |
| Chr18 | 5596266 | 58941 | CCTACAGAAGCATAGACTGCAGG |
| Chr6 | 139353018 | 58275 | CCAAACAAAACATAGACTGCTGG |
| Chr22 | 28895718 | 51098 | ATTAAGATAGCATAGACTGCAGG |
| Chr1 | 77190607 | 50766 | TCACACAAACCATAGACTGAGGG |
| Chr6 | 40925010 | 46749 | AACAAGTATGCATAGACTGCTGG |
| Chr9 | 97332609 | 39286 | GTAATTAAAGCACAGACTGCTGG |
| Chr18 | 68431104 | 35875 | TGTGTAAGAGCATAGACTGCTGG |
| Chr20 | 23101380 | 34207 | ATACACAGAGCAAAGACTGCAGG |
| Chr8 | 97317605 | 30476 | GAACACAGTACATAGACTGGCAG |
| Chr9 | 290168 | 30365 | AAACATAAAGAATAGACTGCAAG |
| Chr6 | 152710071 | 30053 | TACTCTATATCATAGACTGCTGG |
| Chr4 | 31157435 | 28353 | TGATTGAGTGCATAGACTGCTGG |
| Chr1 | 108869935 | 28244 | AGTATAGCAGCATAGACTGCAGG |
| Chr15 | 65377019 | 26457 | GAGCGATAAGCACAGACTGCTGG |

[Table 11]

| | | HEK293-3 | |
| --- | --- | --- | --- |
| Chr | Position | DNA cleavage Score | DNA seq at a cleavage sites |
| Chr15 | 79749931 | 658647 | CACCCAGACTGAGCACGTGCTGG |
| Chr10 | 131593121 | 497117 | GAGCCAGAATGAGCACGTGAGGG |
| Chr1 | 47005705 | 473980 | AGCTCAGACTGAGCAAGTGAGGG |
| Chr7 | 66968042 | 471010 | GACACAGACCGGGCACGTGAGGG |
| Chr9 | 110184637 | 465474 | GGCCCAGACTGAGCACGTGATGG |
| Chr11 | 134562415 | 445120 | GGCGCAGACAGAGCACGTGACGA |
| ChrX | 114764149 | 271791 | AGACCAGACTGAGCAAGAGAGGG |
| Chr4 | 72203005 | 230364 | ATATCAGACTGAGCACCGTGAGG |
| Chr6 | 103918240 | 230024 | AAATAAGACTGAGCACGTGGTGG |
| Chr17 | 7594610 | 229698 | AGCCAGACTGAGGCAAGTGAGGG |
| Chr3 | 160129761 | 183487 | AGGCCAGACTGAACACGATGAGG |
| Chr15 | 35402774 | 163023 | CCTAAAGACTGAGCAAGTGAAGG |
| Chr9 | 137039236 | 159048 | CAGCCAGACAGAGCACGTGGAGG |
| Chr6 | 79958440 | 147188 | AACAAAGACTGAGCACGTTAGGG |
| Chr2 | 130402896 | 104759 | GACCCAGAATGAGCACAAAAGGG |
| Chr1 | 34163192 | 99903 | ATTCTAGACTGAGCACGTGCAAG |
| Chr2 | 97163211 | 92744 | CCCATGGACTGAGCACATGAAGG |
| Chr17 | 79043523 | 71767 | TGGCCAGACTGAGCTCGTGAGTG |
| Chr10 | 22896606 | 64963 | GAAGGAGACTGAGCATGTGAGGG |
| Chr8 | 20947876 | 57273 | TCTCCAGACTGAGCCCATGAGGG |
| Chr19 | 19169358 | 51573 | GGACCAGGCTGAGCACATGGAGG |
| Chr6 | 112273496 | 51305 | AAGCCAGACTGAGCACGTTCAGG |
| Chr1 | 229294224 | 45946 | GGTCATCACTGAGCACGTGAGGT |
| Chr2 | 240026760 | 43234 | GGCTCAGACTGAGCACCTGAGAG |
| Chr15 | 94188821 | 42943 | ATTCCAGAATGAGCACATGAAGG |
| Chr2 | 131090691 | 41142 | ACCCAAGACAGAGCACGTGGAGG |
| Chr11 | 60830962 | 38724 | TCCCAGAACTAAGCACGTGAATG |
| Chr14 | 102917106 | 37462 | CTCGGAGACTGACCACGTGAGGG |
| Chr10 | 23135503 | 32499 | ACTCCAGACTGAGCAACTGAGGG |
| ChrX | 16606309 | 31195 | TTCCCAGACAAAGCACGCGAAGG |
| Chr14 | 78070357 | 25732 | AACCAGACTGGAGCACGTGGTGG |

[Table 12]

| | HEK293-4 | | |
|---|---|---|---|
| Chr | Position | DNA cleavage Score | DNA seq at a cleavage sites |
| Chr15 | 75632142 | 795491 | GCACCTGCGGCTGGAGGTGGCAG |
| Chr1 | 201067378 | 656701 | GGCACTGCTGCTAGAGGTGCAGG |
| Chr16 | 50300347 | 649176 | AGCACTGTGGCTGGGGGAGGGGG |
| Chr4 | 56815199 | 648870 | GGCAATGCGGCTGGAGGCGGAGG |
| Chr11 | 528949 | 647145 | GGCTCTGCGTCCGGAGGTGAGAG |
| Chr9 | 139522901 | 644731 | GCCACAGCGGCCGGAGGTGGCAG |
| Chr20 | 60895671 | 643148 | GGCACAGCAGCTGGAGGTGCTGG |
| Chr16 | 28266968 | 617861 | GGCTCTTCGGCTGGAGGTAGCGG |
| Chr15 | 41044242 | 612537 | GGCGCTGCGGCGGGAGGTGGAGG |
| Chr7 | 134872032 | 592761 | AGCACTGTGGCTGGGGGAGGCGG |
| Chr14 | 21993455 | 579921 | GGTACAGCGGCTGGGGGAGGCGG |
| Chr5 | 141232854 | 577115 | GGCACTGCGGCAGGGAGGAGGGG |
| Chr12 | 113935461 | 571487 | GGCCCTGCGGCTGGAGATATGGG |
| Chr7 | 1397399 | 562494 | AGCACTGCAGCTGGGAGTGGAGG |
| Chr18 | 37194556 | 558246 | GGCACTGCGGGTGGAGGCGGGGG |
| Chr10 | 126694875 | 557909 | GGCACGACGGCTGGAGGTGGGGG |
| Chr3 | 51725452 | 545918 | GGCTCTGTGGCTGGAGGAGGTGG |
| Chr22 | 23284036 | 517249 | GGCCCTGCTGCTGGAGGTGCTGG |
| Chr15 | 80601340 | 503910 | GACACCTCGGCTGGAGGTGCAGG |
| Chr13 | 39262929 | 502808 | AGCAGTGCGGCTAGAGGTGGTGG |
| Chr19 | 33382081 | 502713 | GGCTCTGCGGCTGGAGGGGGTGG |
| Chr20 | 1151854 | 496956 | GGCACTGTGGCTGCAGGTGGAGG |
| Chr17 | 16982386 | 494752 | CAGACTGCGGCAGGGGGTGGCGG |
| Chr7 | 139244407 | 493992 | GCCACTGCGACTGGAGGAGGGGG |
| Chr7 | 54561438 | 490603 | AGGACTGCGGCTGGGGGTGGTGG |
| Chr1 | 32471660 | 484475 | GGCACTTCAGCTGGAGGCAGAGG |
| Chr19 | 1295087 | 474612 | GACACTGAGGCAGGAGGTGGGGG |
| Chr20 | 31349773 | 464930 | GGCACTGCGGCTGGAGGTGGGGG |
| Chr1 | 171018460 | 459439 | GCCACTGGGGCTGGGGGTGGGGG |
| Chr10 | 52054245 | 457305 | GGCCCTGCTACTGGGGGTGGTGG |
| Chr3 | 123863990 | 454429 | TGCCCAGCAGCTGGAGGTGAGGG |
| Chr20 | 60080554 | 452874 | AGCACTGCAGATGGAGGAGGCGG |
| Chr9 | 133039176 | 451926 | GTCACTGCAGCTGGAGGAGGGGG |
| Chr20 | 60010563 | 449013 | TGCACTGCGGCCGGAGGAGGTGG |
| Chr3 | 130508525 | 447541 | GACACTGTGGCTGGAAGTGGAAG |
| Chr19 | 41220525 | 439919 | GGCAATGTGGCTGAAGGTGGGGG |
| Chr22 | 35746283 | 439510 | GACACTAGGGCAGGAGGTGGAGG |
| Chr6 | 51500473 | 434313 | GACACAGTGGCTGGAGGTGTGGG |
| Chr15 | 60790562 | 425666 | GGCACTGCAACTGGAAGTGATGG |
| Chr13 | 27629410 | 423491 | GGCACTGGGGTTGGAGGTGGGGG |
| Chr9 | 5020591 | 417491 | TGCACTGCAGCTGCAGGTGGAGG |
| Chr19 | 38616186 | 415646 | GGCACTGAGACTGGGGGTGGGGG |
| Chr22 | 30130865 | 413438 | GGCTGTGCGGCCAGAGGTGGAGG |
| Chr8 | 144997514 | 411333 | AACACTGCGGCTGCAGCTGGAGG |
| Chr10 | 45317434 | 409344 | GGCTCTGCAGCTGTAGGAGGAGG |
| Chr2 | 241640854 | 407399 | GGGGCTGCGGCCGGAGGTGGTGG |
| Chr20 | 37471343 | 403424 | AGCACTGTGCCTGGGGGTGGGGG |
| Chr10 | 127971444 | 401650 | GGAACTGGGGCTGGGGGTGGGGG |

39

| | | | |
|---|---|---|---|
| Chr8 | 11479079 | 399039 | GGCCCTGCAGCTGGAGATGGAAG |
| Chr15 | 71686928 | 397419 | TGCTCTGCGGCAGGAGGAGGAGG |
| Chr12 | 54977735 | 395702 | GACACTGCCTCTGGGGGTGGGGG |
| Chr20 | 24376057 | 393677 | GGCACTGAGACCAGAGGTGGTGG |
| Chr5 | 177676326 | 392871 | GCCACTGTGGCTGGAGGTGGGGA |
| Chr3 | 23651530 | 387632 | GGCACAGCAGGTGGAGGTGGAGG |
| Chr7 | 110143151 | 387129 | GCCACTGCAGCTAGAGGTGGAGG |
| Chr2 | 25348467 | 384216 | GGAACTGTGGCTGGAGGTGGCAG |
| Chr19 | 56125854 | 376148 | GGCCCAGCGGCGGGAGGTGGGGG |
| Chr10 | 1285239 | 374554 | GGCCCTTCGGCTGGAGGTGGCAG |
| Chr6 | 119227146 | 370348 | GGCACAATGGCTGGAGGTGAAGG |
| Chr20 | 45343011 | 363311 | GGCACTGAGGGTGGAGGTGGGGG |
| Chr5 | 3606830 | 361575 | GACACAACGGCAGGAGGTGGCGG |
| Chr10 | 126752487 | 353759 | GGCACTGCAGCCTGGGGGTGGGG |
| Chr20 | 61810739 | 352160 | GTCACTGCGGCTGCAGATGGCGG |
| Chr22 | 41620073 | 346404 | GGGCATGCGGCTGGAAGTGGTGG |
| Chr8 | 20854500 | 341030 | GGCACTGGGGCTGGAGACGGGGG |
| Chr22 | 49132903 | 339625 | AGCACAGCAGCTGCAGGTGGGGG |
| Chr1 | 230193260 | 336680 | GACTCTGCAGCTGAAGGTGGGGG |
| Chr11 | 118950338 | 326013 | GTCACTGAGGCTGGAGTGGAGGG |
| Chr20 | 22805414 | 318568 | AGCACTGTTACAGGAGGTGGGGG |
| Chr6 | 158452389 | 317681 | AGCTCTGTGGCTGGAGGTGTGAG |
| Chr19 | 46887174 | 316408 | GAGGCTGCGGCTGGGGGGTGGAGG |
| Chr22 | 43768275 | 308603 | AGCACTGCGCTTGGGGGTGGGGG |
| Chr15 | 34061546 | 306434 | AGCACTGTAGCAAGAGGTGGAGG |
| Chr3 | 53375995 | 305643 | GGCTCTGAGGCCAGAGGTGGTGG |
| Chr10 | 77103120 | 304242 | GGCATCACGGCTGGAGGTGGAGG |
| Chr10 | 73435248 | 302892 | GTAACTGCGGCTGGCGGTGGTGG |
| Chr5 | 96338759 | 300204 | AGCACTGGGGATGGAGGTGTAGG |
| Chr1 | 44397932 | 298786 | AGAACTGCTGCTGGAGGTGGTGG |
| Chr5 | 1832938 | 286492 | GGCTCTGTGGCCGGAGGAGGCGG |
| Chr6 | 160517861 | 263538 | GGCACTGCTGCTGGGGGTGGTGG |
| Chr9 | 140205577 | 281021 | GGCCCTGGGGCTGGAGGTGTTGG |
| Chr6 | 33950129 | 273481 | GGCTCTGAGGCTGGTGGTGGGGG |
| Chr1 | 53336192 | 264545 | GGCACGCGGCTGGGAGGTGGAGG |
| Chr3 | 128301954 | 259163 | TGCACTGCAGCTGGGGCTGGAGG |
| Chr12 | 104739609 | 258159 | CCTTCTGCGGCTGGAAGTGGTGG |
| Chr10 | 60003488 | 256317 | GGCACGCGGCTGGGAGGTGGAGG |
| Chr17 | 69519133 | 253054 | AGCAATACGGATGGAGGTGGAGG |
| Chr2 | 152827915 | 251661 | GGCACTTCGGTTGGGGGTGGGGG |
| Chr15 | 41803379 | 250222 | TGCACTGCGGGCGGAGGCGGCGG |
| Chr3 | 10418956 | 250189 | GGCTCCGCAGCTGGAGGTGGGGG |
| Chr7 | 139631 | 249296 | TGCACCGCGGCTGGGGCTGGAGG |
| Chr16 | 22690928 | 242892 | TCCACTGAGGCTGGGGGTGGTGG |
| Chr11 | 65326667 | 242757 | CTGGCAGCGGCTGGGGGTGGGGG |
| ChrX | 70836550 | 231845 | GGCCATGCGGCTGGTGGTGGTGG |
| Chr13 | 88900992 | 229015 | CACACTGCAGCTGGAGGTGGTGG |
| Chr12 | 104234592 | 228650 | CTGCCTGCGGCTGGGGGTGTGGG |
| Chr17 | 75429280 | 226119 | GACACCACGGCTGGAGATGGTGG |
| Chr14 | 101945036 | 224127 | GGGACTGCAACTGGAGGTGGGGG |

| | | | |
|---|---|---|---|
| Chr9 | 74103955 | 220510 | GGCACTGCAGCAGGGGATGGGGG |
| Chr3 | 9039864 | 218073 | GGCTCTGTAGCTGGGGGTGGTGG |
| Chr1 | 204463911 | 208882 | GGCGCTGCGGCTGGAGCCGGCGG |
| Chr2 | 8817154 | 207325 | TGCACAGCGGATGGAGGGGGGGG |
| Chr17 | 40693639 | 204010 | GGCACTGCAGGCAGGAGGTGAGT |
| ChrX | 152805653 | 201320 | GCCACTGAGGCCGGAGGTGGAGA |
| Chr6 | 41374185 | 201307 | GGGCACGCGGCTGGAGGAGGGGG |
| Chr2 | 6961256 | 200536 | AGCTCTGCGGCAGGAGTTGGAGG |
| Chr10 | 13692637 | 199091 | GGCACTGGGGCTGGGGGAGGGGG |
| Chr17 | 75325331 | 196964 | GGCCCTGCAGCTGGAGAGGGAGG |
| Chr7 | 43256545 | 196365 | TACACTGCAGCTGGGAGTGGTGG |
| Chr14 | 88773031 | 195053 | AGCACTGGGGCTGGGGGAGGGGG |
| Chr14 | 63796588 | 194350 | GACACTAAGGCTGGAGGTGGGGA |
| Chr17 | 42152617 | 190730 | TGCACTGCAGCTGGGGGTCGGGG |
| Chr7 | 29233956 | 187308 | GCCACTGGGGCTGGAGGGGGAGG |
| ChrX | 104846030 | 178315 | CAGCTCTGCGCTGGAGGAGGGGG |
| Chr4 | 19769425 | 177335 | AGCTCTGCTGCTGGAGGAGGTGG |
| Chr3 | 52035832 | 174753 | GGCACTGAATCTGGAGGTGGGGG |
| Chr7 | 55344186 | 172714 | ATCACTGCGCCTGGTGGTGGGGG |
| Chr17 | 73501168 | 169547 | GCACCTGCGGCCAGGGGTGGGGG |
| Chr9 | 136602370 | 168438 | GGCACTGGGGCAGGAGATGGGGG |
| Chr16 | 88716134 | 167431 | AGCACGGCAGCTGGAGGAGGGGG |
| Chr14 | 95761249 | 163668 | GGCACTCTGGCTGGAGCTGGGGG |
| Chr6 | 151886068 | 161687 | GGCCCTGCTGCTGGAGAAGGTGG |
| Chr10 | 36109441 | 159071 | GGCATTGCTGCTGGTGGTGGTGG |
| Chr1 | 228559256 | 158331 | GCACCGCGTGCTGGAGGAGGAGG |
| Chr21 | 36453434 | 155062 | AGCTCTGCTATTGGAGGTGGAGG |
| Chr9 | 19933045 | 151459 | AGCCCTGGGGCAGGAGGTGGGGG |
| Chr7 | 150498859 | 149636 | GCTGCTGCGGCTGGAGGTGGGGA |
| Chr16 | 1072626 | 147810 | GGCCCTGCAGCAGGGGGTGGAGG |
| Chr5 | 41968123 | 147631 | GGAAGTGCGGCAGGAGGTGGAGG |
| Chr2 | 45247404 | 143408 | GACACCGTGACTGGAGGTGGAGG |
| Chr18 | 60646595 | 142546 | GCAGCTGCGGCTGGAGCTGAGGG |
| Chr1 | 18954894 | 141715 | GGAACTGTGGCTGGGGATGGGGG |
| Chr2 | 231467380 | 141358 | GGCACTGCAGCTGGGGGTTGGTG |
| Chr4 | 7686554 | 132791 | AACACTGGGGCTGGTGGTGGTGG |
| Chr17 | 25735157 | 130579 | TGCACTCCGACTGGAAGTGGTGG |
| Chr2 | 149402504 | 130567 | TGCACTGAGGAAGGAGGTGGAGG |
| Chr12 | 53453557 | 128079 | TGGACTGCGGCTGGAGAGGGAGG |
| Chr17 | 29815563 | 126311 | GGCGCTGCGGCCGGAGGTGGGGC |
| Chr8 | 145730111 | 126139 | GGCACATGGGCTGGGGGTGGGGG |
| Chr12 | 55427953 | 124563 | GGCACTGAGAAAGGAGGTGGAGG |
| Chr19 | 32836900 | 123779 | TGCCCTGCAGCTGGGGGTGGGGG |
| Chr20 | 49771524 | 121173 | TGCACTGCAGATGGTAGGTGGGG |
| Chr17 | 38478448 | 121131 | GGCACCTTGGCTGAAGGTGGGGG |
| Chr3 | 128169624 | 120130 | ACCACTGTGGCTGGCAGGTGGTG |
| Chr1 | 12259808 | 117998 | AGCACTGCAGCGGGAGGTGAGAG |
| Chr7 | 157443393 | 117892 | GGCACTGGGTCTGAAGGTGGAGG |
| Chr17 | 31790791 | 112013 | TGCACTGCAGCTGGGGGCAGAGG |
| Chr12 | 101718339 | 106833 | GGCACTCTGGCTGGACGTGGTGG |

| Chr8 | 1241128 | 105778 | GGCACTGTTGCTGGAGGAGGCAG |
|---|---|---|---|
| Chr13 | 27530813 | 105452 | GGCACTGCTGACTAGGGGTGGTG |
| Chr16 | 49777696 | 102520 | TGCACTGCGACTGGAGGGAGAGG |
| Chr3 | 193847797 | 101152 | GCACTGCAAACTGGAGGTGGGGG |
| Chr20 | 60174571 | 98694 | CCCACTGTGGCTGGAGGTGTGGG |
| Chr8 | 145543672 | 97195 | AGCCCTGCGGCCGGGGGAGGCGG |
| Chr3 | 49055364 | 96343 | GGGACTGCGGCTGGAGGTGGGAA |
| Chr4 | 156491955 | 94045 | TTCACTGTGGCTGGAGGTGGGGA |
| Chr2 | 3610377 | 86281 | AGCACTATGGATAGAGGTGGAGG |
| Chr9 | 138465751 | 86247 | TACACTGCGGCCGGGAGTGGTGG |
| Chr16 | 26710067 | 84876 | TGCACTGAAGCTGGAGGTGGAGA |
| Chr9 | 35349204 | 81775 | AGTACTGCGGCTGGGCGTGGTGG |
| Chr22 | 18663160 | 81182 | AGCACTAGGGCAGGAGATGGGGG |
| Chr18 | 75260893 | 81143 | GACACTGAGGCTGGAAGAGGTGG |
| Chr12 | 90804707 | 79601 | GGCATGCGGCTGGGAGGTGGAGG |
| Chr6 | 167276293 | 78532 | CGTTCTGCGGCGGGAGGTGGCGG |
| Chr7 | 17979718 | 76594 | GCACTGGCAGCCGGAGGTGGTGG |
| Chr17 | 64544877 | 76045 | GGCAGGGCGGCTGGAGGAGGTGG |
| Chr10 | 132972512 | 75938 | AGCACTGGGGCAGGAGGGTGGTG |
| Chr1 | 229619193 | 73977 | TTGCATGCGGCTGGAAGTGGTGG |
| Chr6 | 36761680 | 73537 | CCCACTGGGGCTGGAGGTGGGGG |
| Chr14 | 77678312 | 73330 | CAGACTGCAGCTGGTAGGTGGTG |
| Chr11 | 3159715 | 69407 | GGCAGTGCAGCTGGAGGCAGGGG |
| ChrX | 26910569 | 68725 | GGCTCTGCCACTGGAGGGGGTGG |
| Chr20 | 61989531 | 68404 | GACACTGAGGCTGGAGGTCTGGG |
| Chr1 | 2933843 | 66266 | GGCCCTGAGACTGCAGCTGGAGG |
| Chr15 | 77121510 | 65960 | AGCACTGTGGATGGAGTTGGAGG |
| Chr9 | 11158273 | 65661 | CTTCCTACGGCAGGAGGTGGGGG |
| Chr3 | 16815640 | 63432 | CGCACTGGGGCTGCAGGTGGAGG |
| Chr6 | 159190938 | 59673 | GGCCCTGCAGCTGGAGGAGGAGA |
| Chr2 | 71766040 | 58033 | AGCACTGCAGTGAGAGGTGGAGG |
| Chr10 | 128864484 | 56269 | GACACCGCAGCTGGGGGCGGCGG |
| Chr7 | 48144881 | 56266 | AGCACTGGGGCTGGAGCTAGAGG |
| Chr16 | 50334859 | 51736 | GGTTCTGCGGTTGGGGGTGGGGG |
| Chr15 | 25425088 | 51134 | GGCTCTGCATTTGGAGGTGTGCG |
| Chr17 | 176302 | 50056 | TGCACTGTGGCTGGAGATGGGGG |
| Chr16 | 1029978 | 49426 | GGCACTGCAGACGGAGGTGTGGG |
| Chr13 | 29913424 | 47868 | GACACTGCTGCTGGAGAGTGGAG |
| Chr16 | 89469252 | 46847 | GGCACTGCGGGAGGAGGTGGGCG |
| Chr6 | 157547859 | 45175 | AGAACTGGGGCTGGGGGTGGGGG |
| Chr20 | 56668028 | 44304 | GGGCCTGCAGCTGGGGGTGGGGG |
| Chr16 | 78413349 | 43989 | GGTACAGTGGCTGGAGGTGGAAG |
| Chr5 | 177928896 | 43690 | CCCACTGCGGGTGGAGGTGGAAG |
| ChrX | 101411055 | 43362 | CGCAGTGCGGCAGGAGGGTGGGG |
| Chr11 | 20409041 | 42605 | AACCCTGCGGCAGGAGGAGGCGG |
| Chr14 | 99266477 | 42026 | GATACTGGGGCTGGGGGTGGAGG |
| Chr11 | 78127585 | 41787 | TGCACTGCAGCTGGAGGCAACGG |
| Chr1 | 183596713 | 40667 | GCACTTGCTGCTGGAGGAGTAGG |
| Chr11 | 17538892 | 40520 | TGCACTGCGGTCAGGAGGAGGCG |
| Chr22 | 18854922 | 35903 | AGCACTAGGGCAGGAGATGGGGG |

42

| Chr12 | 21742959 | 33984 | AGCCCTGCTACTGGGGGTGGGGG |
| Chr8 | 144781302 | 33431 | GACACTGCAGCTGGAGGTGGGGT |
| Chr2 | 59012462 | 33083 | TGCACTGCAACTGGGGGTGGCAG |
| Chr1 | 908980 | 33024 | GACCCTGCGGTGGGAGGTGGCGG |
| Chr15 | 43601412 | 31873 | GGCCCTGAGGCAGGAAGTGGGGG |
| Chr1 | 176665050 | 31488 | ACCACTGAGGATGGGGGTGGAGG |
| Chr20 | 19620239 | 31159 | CGCACTGGGGCTGCAGGTGGAGG |
| Chr5 | 171087054 | 30547 | GGGACTGCAGCTGGGGATGGGGG |
| Chr15 | 26125549 | 30509 | CAAACTGCAGCTGGAGATGGGAG |
| Chr12 | 114150540 | 29438 | CTGACTGCAGCTGGAGGTGGAGA |
| Chr7 | 157689941 | 28995 | GGCACTGGGGAAGGAGGTGGAGG |
| Chr22 | 44625614 | 28747 | GACACTGCTACTGGAGGCTGGGG |
| Chr18 | 60805450 | 27656 | GCACTGGCGGCTGGGAGTGGTGG |
| Chr22 | 18743056 | 27487 | AGCACTAGGGCAGGAGATGGGGG |
| Chr12 | 130659964 | 25960 | GAGAATGCGGATGGAGGTGGTGG |
| Chr14 | 24740271 | 25491 | GGCACTGCCACTGGGGGTGAGGG |
| Chr5 | 54469282 | 25319 | GCCACCGCGGCAGGAGGCGGAGG |
| Chr4 | 6094150 | 25223 | GAGCCTGCGGCTGCAGGTGGGTG |

| HBB | | | |
| --- | --- | --- | --- |
| Chr | Position | DNA cleavage Score | DNA seq at a cleavage sites |
| Chr12 | 124803834 | 1411410 | GCTGCCCCACAGGGCAGCAAAGG |
| Chr15 | 46598129 | 1107004 | GTTGCCCCTCAGGACAGTACAGG |
| Chr22 | 17230623 | 974499 | TGTGCCCCACAGAGCACTAAGGG |
| Chr9 | 104595883 | 923681 | TCAGCCCCACAGGGCAGTAAGGG |
| Chr17 | 8370253 | 877014 | TTGCTCCCACAGGGCAGTAAACG |
| Chr14 | 94585327 | 837188 | ATGGCCCCACAAGGCAGAAATGG |
| Chr11 | 5248215 | 818187 | CTTGCCCCACAGGGCAGTAACGG |
| Chr12 | 93549202 | 583920 | ATTGCCCCACGGGGCAGTGACGG |
| Chr1 | 177593980 | 229317 | TCTACCCCACATGGCAGTAATGG |
| ChrX | 75006257 | 165922 | GTGGCCCCACAGGGCAGGAATGG |
| Chr6 | 50041372 | 142026 | TCTGCCCCACATGGCAGTAATGA |
| Chr6 | 23709579 | 114576 | GAAGCCCTACAGGGCAGCAATGG |
| Chr2 | 121715240 | 97571 | GTGTCCCCACAGGGCAGGAAAGG |
| Chr8 | 24931381 | 97489 | AGTGCCACACACAGCAGTAAGGG |
| Chr14 | 36889538 | 53050 | GTTATCCCACAGGACAGTGAGGG |
| Chr1 | 17346702 | 51982 | CGGTCCCCACAGGGTCAGTAAGG |
| Chr20 | 39992928 | 49369 | AGTGGCCCCAGGGCAGTGAGGG |
| Chr10 | 95791920 | 47520 | ACTCTCCCACAAGGCAGTAAGGG |
| Chr4 | 148531374 | 37943 | GTTACCTCACAGAGCAGAAAGGG |
| Chr5 | 53613387 | 32279 | TCACCCCCACAGGCCAGTAAAGG |

GUIDE-seq and other methods require a filtering step that removes about 90% of the detection sites that lack homology to the on-target site, but the multiplex Digenome-seq does not filter sites but are aligned based on edit distance.

The 964 sites were clearly divided into 11 groups. Furthermore, each of the 11 groups for *in vitro* cleavage site was has a high homology to one of 11 target sequences. Accordingly, a de novo motif or sequence logo, obtained by comparing sequences within each group, matched the target sequence at almost all nucleotide sites (Fig. 15a).

The results show that although it is less than the protospacer-adjacent motif (PAM) sequence and the PAM-proximal 10-nt "seed" site recognized by Cas9, the 10-nt site of the 5'-end at the 23-nt target sequence contributes to the specificity of RGEN. Further, it was identified that all sites except one of the 964 sites cleaved by the 11 RGEN have the PAM sequence of 5'-NGG-3' or the sequences similar to PAM of 5'-NNG-3'/5'-NGN-3'. Accordingly, the multiple Digenome-seq can be used to accurately find *in vitro* cleavage sites without program searches for homologous sequences and is simple, can be applied to a plurality of programmable nucleases, and has many advantages as compared to the other known methods such as GUIDE-seq and HTGTS.

Next, it was identified whether each sgRNA was capable of cleaving on-target and off-target sites. 17 sites (= 57%) of 30 sites cleaved by treatment with Cas9 (300 nM) at a high concentration (900 nM) of HBB-specific sgRNA were detected at the time of performing the multiplex Digenome-seq using the same sgRNA as low concentration (82 nM) (Figs. 15b and 16c). These results suggest that each of 11 sgRNAs can direct Cas9 to their on-target and off-target sites independently of each other, and it can be understood that Digenome-seq has complexity.

Experimental Example 8: *In vitro* cleavage site

The 11 sgRNAs showed a wide range of specificities on a genomic scale; The number of cleavage sites per sgRNA in the human genome ranged from 13 to 302 (Fig. 16a and Tables 3 to 12). As expected, all of the on-target sites identified in the human genome using the Cas-OFFinder, and each on-target site and the most of the sites having one or two nucleotides were detected when the multiplex Digenome-seq was performed (Fig. 16b). However, few sites with three or more nucleotide mismatches were detected. That is, the ratio of sites detected by Digenome-seq decreased exponentially as the number of nucleotide mismatches increased from 3 to 6 (Fig. 16b). In addition, the sites with two or more nucleotide mismatches in the seed region were not cleaved *in vitro* than positions with zero or one mismatch ($P < 0.01$, Student's t-test).

On the other hand, it was identified that the number of sites detected with Digenome-seq and the number of homologous sites (defined as "orthogonality") having a nucleotide mismatch of 6 or less in the human genome had a significant correlation ($R^2 = 0.93$) (Fig. 16c). That is, 5 sgRNAs with 16,000 or more of homologous sites in the human genome cleave 63 or more (161 on average per sgRNA) *in vitro,* whereas 6 sgRNAs with 13,000 or less of homologous sites cleave 46 or less *in vitro* (28 on average per sgRNA), and thus is relatively more specific ($P < 0.01$, Student's test) (Fig. 16c). The results are different from the lack of correlation ($R^2 = 0.29$) observed between the number of GUIDE-seq positive sites and the orthogonality of the on-target site for the human genome (Fig. 17). However, the 5 most specific sgRNAs identified as GUIDE-seq, which cleave 10 or fewer sites in the cells, were consistent with the most specific sgRNA identified by Digenome-seq.

The results suggest that certain sites in the human genome where there are fewer than 13,000 nucleotide mismatches with 6 homologous sites or less and no homologous sites with 2 nucleotide mismatches or less are desirable to minimize off-target effects. In this regard, 368 sites (= 21.5%) among the 1715 targetable sites including the 5'-NGG-3' PAM sequence correspond to the above concept for 4 genes tested in the present disclosure (Table 13).

[Table 13]

| Gene | Exon | No. of PAM (NGG) -containing sites | No. of sites with no homologous sites harboring 0 or 1 mismatch in the human genome & No. of sites with fewer than 13,000 homologous sites harboring up to 6 mismatches |
|---|---|---|---|
| VEGFA | Exon1 | 235 | 79 |
| | Exon2 | 8 | 0 |
| | Exon3 | 26 | 18 |
| | Exon4 | 6 | 0 |
| | Exon5 | 1 | 0 |
| | Exon6 | 14 | 5 |
| | Exon7 | 8 | 4 |
| | Exon8 | 252 | 34 |
| | Total | 550 | 140 |

(continued)

| Gene | Exon | No. of PAM (NGG)-containing sites | No. of sites with no homologous sites harboring 0 or 1 mismatch in the human genome & No. of sites with fewer than 13,000 homologous sites harboring up to 6 mismatches |
|---|---|---|---|
| EMX1 | Exon1 | 238 | 73 |
| | Exon2 | 29 | 8 |
| | Exon3 | 245 | 37 |
| | Total | 512 | 118 |
| FANCF | Exon1 | 373 | 90 |
| | Total | 373 | 90 |
| RNF2 | Exon1 | 50 | 12 |
| | Exon2 | 4 | 0 |
| | Exon3 | 8 | 0 |
| | Exon4 | 14 | 0 |
| | Exon5 | 21 | 0 |
| | Exon6 | 10 | 0 |
| | Exon7 | 173 | 8 |
| | Total | 280 | 20 |
| Total | | 1715 | 368 |

Experimental Example 9: Digenome-seq. vs. other methods

On average, the multiplex Digenome-seq successfully identified $80 \pm 8\%$ of the sites detected by the conventional GUIDE-seq (Fig. 16a). For example, all sites detected with GUIDE-seq using three sgRNAs specific for VEGFA1, RNF2, and HEK293-3 were also identified as Digenome-seq. In addition, the multiplex Digenome-seq detected 703 new sites (70 averages per sgRNA) that were not detected by GUIDE-seq (Fig. 16A). As a result, GUIDE-seq detected $25 \pm 6\%$ of the sites detected by multiplex Digenome-seq. RNF2 specific sgRNA is a good example showing the advantages of Digenome-seq. Previous studies have done two independent GUIDE-seq analyzes, but could not detect off-target sites for this sgRNA. However, Digenome-seq identified 12 cleavage sites in addition to the on-target site. Furthermore, a lack of correlation ($R^2$ = 0.20) was observed between the number of Digenome-seq positive sites and the number of GUIDE-positive sites (Fig. 16d).

Digenome-seq can obtain more off-target site candidates than GUIDE-seq for 9 of the 10 sgRNAs, but this is not a comprehensive result. That is, HBB sgRNA was not analyzed by GUIDE-seq. Overall, GUIDE-seq detected a total of 168 sites that were not detected in Digenome-seq.

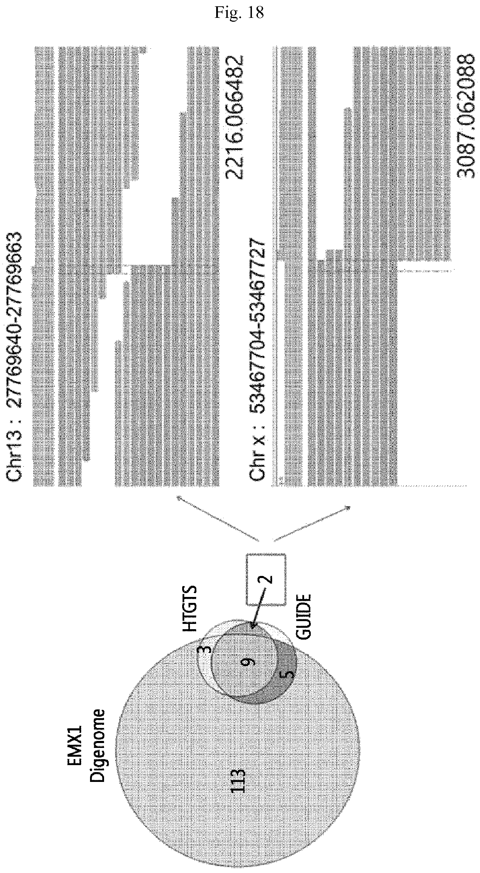

On the other hand, HTGTS was also performed for two sgRNAs targeting VEGFA 1 and EMX1 sites (Fig. 16a). Most of the sites detected by at least one of the other two methods (GUIDE-seq and HTGTS) (31 of 40 in VEGFA 1 and 17 of 19 in EMX1) were also investigated as Digenome-seq, but 9 of VEGFA and 2 of EMX1 were not detected. It is because that some sites are artifact results by PCR primers or false positives arising from naturally occurring DSBs, which are the inherent limitations of GUIDE-seq and HTGTS. However, the two EMX1 off-target sites commonly found in this position, most commonly in the other two methods, are sgRNAs having a low sequencing depth (Fig. 18) or a low concentration (82 nM) at the specific site, and thus were not identified in the multiplex Digenome-seq. This problem could be overcome by performing WGS multiple times to increase the average sequencing depth and merging with sequence read obtained by using sgRNA of a high concentration in a single analysis.

VEGFA 2 specific sgRNAs are the only exception to the rule that Digenome-seq can detect more candidate sites than GUIDE-seq. That is, GUIDE-seq identified 122 sites that were not detected in Digenome-seq. The target sequence is an uncommon sequence consisting of cytosine stretch. Multiple sequence reads obtained with WGS at homopolymer sites could be removed from the mapping program. On the other hand, GUIDE-seq will be able to detect these positions using PCR to amplify the detected oligonucleotide sites.

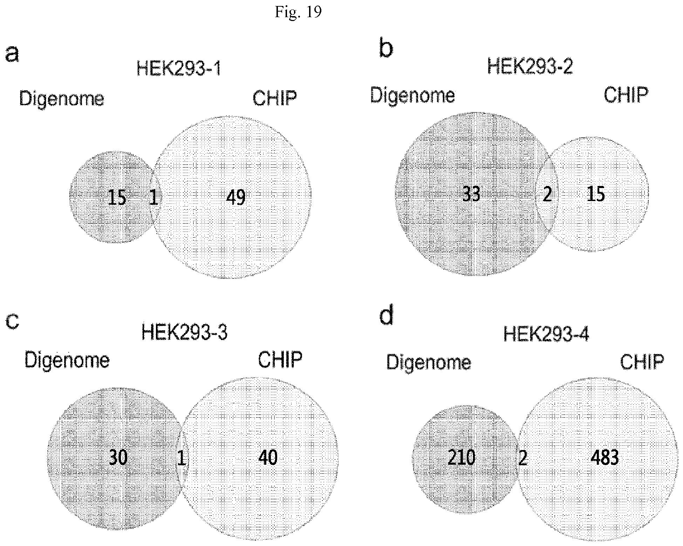

Next, the cleavage sites identified in the present disclosure were compared with those detected with ChiP-seq (chromatin immunoprecipitation sequencing). First, ChiP-seq was performed on the four sgRNAs used in the present disclosure. DCas9 did not bind to the majority of the Cas9-cleavage sites (288, 98%) identified as Digenome-seq (Fig. 19). The results show that DNA binding of Cas9 is a concept separated from DNA cleavage, and ChiP-seq using dCas9 is useful for examining the specificity of dCas9-based transcription factors and epigenome regulators, but it is inappropriate to analyze the genomic scale specificity of Cas9 RGEN.

Experimental Example 10: Identification of intracellular off-target site

Next, using the next-generation sequencing (NGS) platform, it was identified whether each sgRNA and Cas9 protein for some sites of the sites (Table 14 to Table 23) identified in Digenome-seq and GUIDE-seq induces off-target indels in human cells.

[Table 14]

| | | Digenome only | Digenome and GUIDE | GUIDE only |
|---|---|---|---|---|
| VEGFA1 | Total captured sites | 57 | 22 | 0 |
| | Number of NGS-tested sites | 15 | 22 | 0 |
| | Number of validated sites | 6 | 20 | 0 |
| VEGFA2 | Total captured sites | 33 | 30 | 122 |
| | Number of NGS-tested sites | 8 | 22 | 14 |
| | Number of validated sites | 0 | 22 | 10 |
| VEGFA3 | Total captured sites | 256 | 46 | 14 |
| | Number of NGS-tested sites | 18 | 27 | 9 |
| | Number of validated sites | 4 | 22 | 5 |
| EMX1 | Total captured sites | 129 | 14 | 2 |
| | Number of NGS-tested sites | 16 | 12 | 2 |
| | Number of validated sites | 3 | 9 | 2 |
| FANCF | Total captured sites | 38 | 8 | 1 |
| | Number of NGS-tested sites | 8 | 8 | 1 |
| | Number of validated sites | 1 | 8 | 0 |
| RNF2 | Total captured sites | 12 | 1 | 0 |
| | Number of NGS-tested sites | 12 | 1 | 0 |
| | Number of validated sites | 2 | 1 | 0 |
| HEK1 | Total captured sites | 8 | 8 | 2 |
| | Number of NGS-tested sites | 3 | 8 | 2 |
| | Number of validated sites | 1 | 7 | 2 |
| HEK2 | Total captured sites | 33 | 2 | 1 |
| | Number of NGS-tested sites | 16 | 2 | 1 |
| | Number of validated sites | 1 | 2 | 0 |
| HEK3 | Total captured sites | 25 | 6 | 0 |
| | Number of NGS-tested sites | 14 | 6 | 0 |
| | Number of validated sites | 2 | 6 | 0 |
| HEK4 | Total captured sites | 112 | 104 | 26 |
| | Number of NGS-tested sites | 17 | 24 | 16 |
| | Number of validated sites | 1 | 19 | 4 |
| Total | Total captured sites | 703 | 241 | 168 |
| | Number of NGS-tested sites | 127 | 132 | 45 |
| | Number of validated sites | 21 | 116 | 23 |

[Table 15]

| | Chromosome | Location | DNA seq at a Cleavage sites | Indel frequency (%) | | Validation |
|---|---|---|---|---|---|---|
| | | | | (-) RGEN | (+) RGEN | |
| On-Target | Chr6 | 43737290 | GGGTGGGGGGAGTTTGCTCCAGG | 0.01% | 21.77% | validated |
| VEGFA1_02 | Chr15 | 65537537 | GGATGGAGGGAGTTTGCTCCTGG | 0.01% | 25.28% | validated |
| VEGFA1_03 | Chr5 | 7067159 | GAGGGTGGGGAGTTTACTCCTGG | 0.01% | 0.09% | validated |
| VEGFA1_04 | Chr1 | 99347651 | GGGGAGGGGAAGTTTGCTCCTGG | 0.01% | 13.64% | validated |
| VEGFA1_05 | Chr12 | 1988077 | CGGGGGAGGGAGTTTGCTCCTGG | 0.00% | 11.73% | validated |
| VEGFA1_06 | Chr22 | 37215276 | GGGTGGGGGGAGTTTGCCCCAGG | 0.09% | 1.03% | validated |
| VEGFA1_07 | Chr17 | 32996325 | GGGGGTGGGGACTTTGCTCCAGG | 0.04% | 0.02% | invalidated |
| VEGFA1_08 | Chr12 | 26641302 | AGTTTGGGGGAGTTTGCCCCAGG | 0.12% | 0.12% | invalidated |
| VEGFA1_09 | Chr1 | 233157354 | GGAGGAGGGGAGTCTGCTCCAGG | 0.01% | 0.05% | validated |
| VEGFA1_10 | Chr10 | 124731416 | AGCTGGAGGGAGTTTGCCCCAGG | 0.13% | 0.25% | validated |
| VEGFA1_11 | Chr12 | 131690199 | GGGAGGGTGGAGTTTGCTCCTGG | 0.00% | 6.70% | validated |
| VEGFA1_12 | Chr11 | 71497119 | AGGAAGGAGGAGTTAGCTCCTGG | 0.00% | 0.02% | invalidated |
| VEGFA1_13 | Chr17 | 39796328 | TAGTGGAGGGAGCTTGCTCCTGG | 0.00% | 16.90% | validated |
| VEGFA1_14 | Chr4 | 8453803 | GAGTGGGTGGAGTTTGCTACAGG | 0.01% | 0.13% | validated |
| VEGFA1_15 | Chr9 | 93925190 | GGGGGTGGGGAGCATGCTCCAGG | 0.01% | 0.02% | validated |
| VEGFA1_16 | Chr3 | 125633992 | AGGAAGGAGGAGTTAGCTCCTGG | 0.02% | 0.01% | invalidated |
| VEGFA1_17 | Chr16 | 8763213 | AAGTAAGGGAAGTTTGCTCCTGG | 0.01% | 0.01% | invalidated |
| VEGFA1_18 | Chr20 | 56175356 | AGGGAGGAGGAATTTGCTCCTGG | 0.00% | 0.72% | validated |
| VEGFA1_19 | Chr15 | 93140401 | GGGGGAGGGAAGTTTCCTCCAGG | 0.02% | 0.01% | invalidated |
| VEGFA1_20 | Chr3 | 128234321 | AGGTGGTGGGAGCTTGTTCCTGG | 0.00% | 0.14% | validated |
| VEGFA1_21 | Chr5 | 32945275 | GCGTGGGGGGTGTTTGCTCCCGG | 0.03% | 1.00% | validated |
| VEGFA1_22 | Chr6 | 14316373 | GTGGGGGTAGAGTTTGCTCCAGG | 0.02% | 6.10% | validated |
| VEGFA1_23 | Chr13 | 26202812 | GGTTGAGGGGAGTCTGCTCCAGG | 0.01% | 0.17% | validated |
| VEGFA1_24 | Chr5 | 139263024 | TTGGGGGGGGCAGTTTGCTCCTGG | 2.33% | 7.19% | validated |
| VEGFA1_25 | Chr2 | 96056645 | GGGTGGGGAGAGTTTCTTCCTGG | 0.00% | 0.00% | invalidated |
| VEGFA1_26 | Chr3 | 195871254 | GGTGGGGGAGAGCTAGCTCCGGG | 0.00% | 0.20% | validated |
| VEGFA1_27 | Chr11 | 3445204 | AGGAAGGAGGAGTTAGCTCCTGG | 0.02% | 0.04% | validated |
| VEGFA1_28 | ChrX | 19185601 | GGGAGGGGAGAGTTTGTTCCAGG | 0.01% | 0.02% | invalidated |
| VEGFA1_29 | Chr11 | 67574262 | AGGAAGGAGGGAGTTAGCTCCTGG | 0.01% | 0.73% | validated |
| VEGFA1_30 | Chr17 | 47317539 | CTGGTGGGGAGCTTGCTCCAGG | 1.64% | 4.14% | validated |
| VEGFA1_31 | Chr22 | 19598463 | GAGGGGGAGCAGTTTGCTCCAGG | 0.01% | 0.56% | validated |
| VEGFA1_32 | Chr21 | 37116659 | AAGTGGGAAGAGTTTGTTCCAGG | 0.03% | 0.01% | invalidated |
| VEGFA1_33 | Chr11 | 117461208 | GGGCAAGGGGAGGTTGCTCCTGG | 0.01% | 0.35% | validated |
| VEGFA1_34 | Chr5 | 56172079 | GGTGGGGGTGGGTTTGCTCCTGG | 0.00% | 3.94% | validated |
| VEGFA1_35 | Chr1 | 33543288 | GGGTGGGTGGAGTTTGCTACTGG | 0.00% | 0.30% | validated |
| VEGFA1_36 | Chr6 | 29483353 | AAGTGGGAGGAGACTGCTCCAGG | 0.01% | 0.02% | invalidated |
| VEGFA1_37 | Chr22 | 38219333 | AGGTCGGGGGAGTTAGATCCCGG | 0.01% | 0.02% | invalidated |

[Table 16]

| | Chromosome | Location | DNA seq at a Cleavage sites | Indel frequency (%) | | Validation |
|---|---|---|---|---|---|---|
| | | | | (-) RGEN | (+) RGEN | |
| On-Target | Chr6 | 43738562 | GACCCCCTCCACCCCGCCTCCGG | 0.00% | 96.41% | validated |
| VEGFA2_02 | Chr11 | 31617483 | GGGCCCCTCCACCCCGCCTCTGG | 0.04% | 2.50% | validated |
| VEGFA2_03 | Chr5 | 6715119 | CTACCCCTCCACCCCGCCTCCGG | 0.00% | 6.24% | validated |
| VEGFA2_04 | Chr17 | 4356752 | TACCCCCCACACCCCGCCTCTGG | 0.01% | 0.74% | validated |
| VEGFA2_05 | Chr9 | 27338675 | GACCCCTCCCACCCCGACTCCGG | 0.00% | 0.87% | validated |
| VEGFA2_06 | Chr16 | 21358569 | GCCCCCACCCACCCCGCCTCTGG | 0.00% | 34.17% | validated |
| VEGFA2_07 | ChrX | 118865483 | GTCCTCCACCACCCCGCCTCTGG | 0.00% | 0.05% | validated |
| VEGFA2_08 | Chr2 | 242214507 | ATTCCCCCCCACCCCGCCTCAGG | 0.78% | 6.77% | validated |
| VEGFA2_09 | Chr9 | 103599649 | ACACCCCCCCCACCCCGCCTCAGG | 0.00% | 3.35% | validated |
| VEGFA2_10 | Chr15 | 56563429 | TGCCCCCCCCCACCCCACCTCTGG | 0.03% | 3.82% | validated |
| VEGFA2_11 | Chr11 | 71948605 | GCTTCCCTCCACCCCGCATCCGG | 0.01% | 0.44% | validated |
| VEGFA2_12 | Chr17 | 40044757 | TGCCCCTCCCACCCCGCCTCTGG | 0.00% | 0.77% | validated |
| VEGFA2_13 | Chr10 | 116294256 | CCCCACCCCCACCCCGCCTCAGG | 0.15% | 63.43% | validated |
| VEGFA2_14 | Chr10 | 13514994B | CGCCCTCCCCACCCCGCCTCCGG | 0.01% | 8.44% | validated |
| VEGFA2_15 | Chr3 | 140398601 | CAACCCCCCCACCCCGCTTCAGG | 0.03% | 1.38% | validated |
| VEGFA2_17 | Chr12 | 26025096 | CATTCCCCCCACCCCACCTCAGG | 0.03% | 18.64% | validated |
| VEGFA2_18 | Chr10 | 72536218 | CAGTCCCCCCACCCCACCTCTGG | 0.01% | 0.57% | validated |
| VEGFA2_19 | Chr9 | 131706562 | AGCCAACCCCACCCCGCCTCTGG | 0.01% | 0.06% | validated |
| VEGFA2_22 | Chr13 | 13122189 | GCCCCCCACCACCCCACCTCGGG | 0.00% | 1.86% | validated |
| VEGFA2_33 | Chr2 | 12744776 | GACACACCCCACCCCACCTCAGG | 0.01% | 0.39% | validated |
| VEGFA2_34 | Chr13 | 100546989 | CCCCCCCCCCCCCCCGCCTCAGG | 4.45% | 13.82% | validated |
| VEGFA2_39 | Chr4 | 38537628 | CTCCCCACCCACCCCGCCTCAGG | 0.00% | 69.10% | validated |
| VEGFA2_40 | Chr12 | 101603788 | GCCAGCCCTCACCCCGCCTCGGG | 0.00% | 0.00% | Invalidated |
| VEGFA2_42 | Chr6 | 10582454 | CCCTCTCCACCCCCACCCTCTGG | 0.00% | 0.00% | Invalidated |
| VEGFA2_43 | Chr16 | 13492458 | TCCGCCCCCCACCCCACCTCCGG | 0.04% | 0.03% | Invalidated |
| VEGFA2_44 | Chr1 | 111860603 | TAAATCCTCCACCCCCACCTCAGG | 0.01% | 0.00% | Invalidated |
| VEGFA2_46 | Chr6 | 167929603 | GCTGTCTCCCACCCCGCCTCAGG | 0.00% | 0.01% | Invalidated |
| VEGFA2_50 | Chr17 | 29983010 | CATCTTCCCCACCCCGCCTCTGG | 0.24% | 0.26% | Invalidated |
| VEGFA2_51 | Chr14 | 75098723 | CCTCACCCCCACCCCACCTCTGG | 0.00% | 0.00% | Invalidated |
| VEGFA2_54 | Chr20 | 25240252 | CCCACACCCCACCCCACCTCCGG | 0.00% | 0.01% | Invalidated |

[Table 17]

| | On-Target | Chromosome | Location | DNA seq at a Cleavage sites | VEGFA3 | | Validation |
|---|---|---|---|---|---|---|---|
| | | | | | Indel frequency (%) | | |
| | | | | | (-) RGEN | (+) RGEN | |
| | On-Target | Chr6 | 43737471 | GGTGAGTGAGTGTGTGCGTGTGG | 0.01% | 41.86% | validated |
| | VEGFA3_02 | Chr14 | 66569159 | AGTGAGTGAGTGTGTGTGTGGGG | 0.28% | 35.20% | validated |
| | VEGFA3_03 | Chr5 | 89440969 | AGAGAGTGAGTGTGTGCATGAGG | 0.00% | 18.71% | validated |
| | VEGFA3_04 | Chr5 | 115434676 | TGTGGGTGAGTGTGTGCGTGAGG | 0.01% | 30.86% | validated |
| | VEGFA3_05 | Chr22 | 37662624 | GCTGAGTGAGTGTATGCGTGTGG | 0.00% | 24.48% | validated |
| | VEGFA3_06 | Chr11 | 68851139 | GGTGAGTGAGTGCGTGCGGGTGG | 1.79% | 11.15% | validated |
| | VEGFA3_07 | Chr10 | 98760588 | GTTGAGTGAATGTGTGCGTGAGG | 0.00% | 19.92% | validated |
| | VEGFA3_08 | Chr3 | 193893884 | AGTGAATGAGTGTGTGTGTGTGG | 0.40% | 23.67% | validated |
| | VEGFA3_09 | Chr14 | 62076773 | TGTGAGTAAGTGTGTGTGTGTGG | 0.57% | 20.05% | validated |
| | VEGFA3_10 | Chr19 | 40561867 | ACTGTGTGAGTGTGTGCGTGAGG | 0.02% | 0.72% | validated |
| | VEGFA3_11 | Chr20 | 20178284 | AGTGTGTGAGTGTGTGCGTGTGG | 0.25% | 34.56% | validated |
| | VEGFA3_12 | Chr9 | 23824554 | TGTGGGTGAGTGTGTGCGTGAGA | 0.00% | 0.32% | validated |
| | VEGFA3_14 | Chr14 | 105029032 | GGTGAGTGAGTGTGTGTGTGAGG | 0.03% | 2.39% | validated |
| | VEGFA3_15 | Chr19 | 47730492 | CTGGAGTGAGTGTGTGTGTGTGG | 0.01% | 0.00% | Invalidated |
| | VEGFA3_16 | Chr9 | 18733635 | AGCGAGTGAGTGTGTGTGTGGGG | 0.20% | 32.70% | validated |
| | VEGFA3_17 | Chr2 | 73317050 | GGTGAGTCAGTGTGTGAGTGAGG | 2.29% | 2.66% | Invalidated |
| | VEGFA3_18 | Chr4 | 58326608 | AGTGAGTGAGTGAGTGAGTGAGG | 0.02% | 0.00% | Invalidated |
| | VEGFA3_19 | Chr8 | 46997806 | GTAGAGTGAGTGTGTGTGTGTGG | 0.45% | 5.11% | validated |
| | VEGFA3_20 | Chr14 | 74353497 | AGCGAGTGGGTGTGTGCGTGGGG | 0.01% | 12.60% | validated |
| | VEGFA3_21 | Chr22 | 49740001 | GGTGTGTGAGTGTGTGTGTGTGG | 0.46% | 2.89% | validated |
| | VEGFA3_23 | Chr16 | 84032646 | GGTGAATGAGTGTGTGCTCTGGG | 0.01% | 0.68% | validated |
| | VEGFA3_24 | Chr10 | 5749657 | AGTGAGTATGTGTGTGTGTGGGG | 1.31% | 1.86% | validated |
| | VEGFA3_27 | Chr4 | 62067619 | GATGAGTGTGTGTGTGTGTGAGG | 0.45% | 0.36% | Invalidated |
| | VEGFA3_29 | Chr2 | 230506241 | GGTGAGCAAGTGTGTGTGTGTGG | 0.46% | 51.82% | validated |
| | VEGFA3_31 | Chr17 | 33323069 | TGTGAGTGAGTATGTACATGTGG | 0.00% | 0.01% | Invalidated |
| | VEGFA3_32 | Chr7 | 51294279 | AGTGAGTAAGTGAGTGAGTGAGG | 0.00% | 0.00% | Invalidated |
| | VEGFA3_34 | Chr16 | 73585926 | AATGAGTGAGTGTGTGTGTGTGA | 0.77% | 0.97% | Invalidated |
| | VEGFA3_36 | Chr2 | 18696225 | AGTGAGAAAGTGTGTGCATGCGG | 0.00% | 0.16% | validated |
| | VEGFA3_37 | Chr19 | 6660674 | TGTGAGTGAGTGAGTGAATGTGG | 0.06% | 0.18% | validated |
| | VEGFA3_39 | Chr10 | 67367984 | GGTGTGTGAGTGTGTGCATGTTG | 0.22% | 0.23% | Invalidated |
| | VEGFA3_40 | Chr12 | 114752937 | TGTGAGTGAGTGTGTGCATGTGA | 0.32% | 0.36% | Invalidated |
| | VEGFA3_41 | Chr14 | 98442534 | GGTGAGTGTGTGTGTGAGTGTGG | 0.00% | 0.00% | Invalidated |
| | VEGFA3_42 | Chr19 | 15569487 | TGTGTGAGTGAGTGTGTGTGTGG | 0.07% | 0.22% | validated |
| | VEGFA3_43 | Chr5 | 34462076 | TGTGTGAGTGTGTGTGTGCGTGG | 0.18% | 0.13% | Invalidated |
| | VEGFA3_44 | ChrX | 41726218 | GGTGAGTGAGTGAGTGAGTGAGG | 0.01% | 0.03% | Invalidated |
| | VEGFA3_45 | Chr10 | 105307473 | TGAGTGTGAGTGTGTGCGTGGGG | 0.00% | 0.01% | Invalidated |
| | VEGFA3_46 | Chr11 | 12159166 | TGTGTGAGTGTGTGTGTGGGGGG | 0.40% | 0.34% | Invalidated |
| | VEGFA3_47 | Chr11 | 75330150 | TGTGTGTGAGTGTGTGCATGAGG | 0.30% | 0.32% | Invalidated |
| | VEGFA3_48 | Chr16 | 6130265 | TGTGAGTGAATGTGTGTGTGTGG | 0.15% | 0.25% | Invalidated |
| | VEGFA3_49 | Chr16 | 73266082 | CATGAGTGGGTGTGTGCGTGGAG | 0.03% | 0.03% | Invalidated |
| | VEGFA3_50 | Chr19 | 40595585 | GGACTGTGAGTGTGTGCGTGAGG | 0.01% | 0.00% | Invalidated |
| | VEGFA3_52 | Chr2 | 163092036 | GATGTGTGAGTGTGGCCTGTGG | 0.01% | 0.07% | validated |
| | VEGFA3_53 | Chr20 | 2650069 | GGTGTATGAGTGTGTGCGTCGGA | 1.25% | 1.30% | Invalidated |
| | VEGFA3_54 | Chr3 | 10207131 | GGTGTGTGTGTGTGTGTGTGTGG | 0.10% | 0.09% | Invalidated |
| | VEGFA3_55 | Chr5 | 98946319 | GGTGTAGTGGTGTGTGCTTGTGG | 0.00% | 0.00% | Invalidated |
| | VEGFA3_56 | Chr6 | 39028642 | GGTGTGTGAGTGTGTGCATTGGG | 0.00% | 0.09% | validated |

[Table 18]

| | | EMX1 | | | | |
|---|---|---|---|---|---|---|
| | | | | | Indel frequency (%) | |
| | Chromosome | Location | DNA seq at a Cleavage sites | (-) RGEN | (+) RGEN | Validation |
| On-Target | Chr2 | 73160999 | GAGTCCGAGCAGAAGAAGAAGGG | 0.23% | 61.61% | validated |
| EMX1_02 | Chr5 | 45359067 | GAGTTAGAGCAGAAGAAGAAAGG | 0.02% | 47.11% | validated |
| EMX1_03 | Chr15 | 44109764 | GAGTCTAAGCAGAAGAAGAAGAG | 0.42% | 39.41% | validated |
| EMX1_04 | Chr2 | 219845073 | GAGGCCGAGCAGAAGAAAGACGG | 0.01% | 6.38% | validated |
| EMX1_05 | Chr8 | 128801260 | GAGTCCTAGCAGGAGAAGAAGAG | 0.03% | 6.67% | validated |
| EMX1_06 | Chr5 | 146633190 | GAGCCGGAGCAGAAGAAGGAGGG | 0.03% | 0.78% | validated |
| EMX1_07 | Chr1 | 23720516 | AAGTCCGAGGAGAGGAAGAAAGG | 0.03% | 0.06% | Invalidated |
| EMX1_08 | Chr6 | 9118799 | ACGTCTGAGCAGAAGAAGAATGG | 0.03% | 0.75% | validated |
| EMX1_09 | Chr15 | 100292479 | AAGTCCCGGCAGAGGAAGAAGGG | 0.01% | 0.09% | validated |
| EMX1_10 | Chr10 | 58846723 | GAGCACGAGCAAGAGAAGAAGGG | 0.00% | 0.00% | Invalidated |
| EMX1_11 | Chr2 | 218378108 | GAGTCTAAGCAGGAGAATAAAGG | 0.06% | 0.14% | validated |
| EMX1_12 | Chr3 | 96690186 | TCATCCAAGCAGAAGAAGAAGAG | 0.45% | 0.51% | Invalidated |
| EMX1_15 | Chr14 | 48932120 | GAGTCCCAGCAAAAGAAGAAAAG | 0.05% | 0.03% | Invalidated |
| EMX1_16 | Chr1 | 113741471 | GAGGTAGAGCAGAAGAAGAAGCG | 0.06% | 0.06% | Invalidated |
| EMX1_17 | Chr1 | 231750743 | GAGTCAGAGCAAAAGAAGTAGTG | 0.00% | 0.00% | Invalidated |
| EMX1_18 | Chr1 | 234492964 | GAAGTAGAGCAGAAGAAGAAGCG | 0.07% | 0.06% | Invalidated |
| EMX1_19 | Chr2 | 172374203 | GAAGTAGAGCAGAAGAAGAAGCG | 0.07% | 0.07% | Invalidated |
| EMX1_20 | Chr11 | 62365273 | GAATCCAAGCAGAAGAAGAGAAG | 0.02% | 0.13% | validated |
| EMX1_21 | Chr3 | 16077518 | GAGGCAGAGAGAAAGAAGAAAGG | 0.01% | 0.01% | Invalidated |
| EMX1_22 | Chr1 | 33606480 | GAGCCTGAGCAGAAGGAGAAGGG | 0.01% | 0.08% | validated |
| EMX1_23 | Chr1 | 221522625 | GAGTTTGAGTAGAAGAAGAAGAG | 0.72% | 0.70% | Invalidated |
| EMX1_24 | Chr3 | 34042974 | GAGTTCAAGCAGAGAAGAAAGGG | 1.09% | 1.10% | Invalidated |
| EMX1_25 | Chr4 | 44622977 | AAGTCTGAGAAGAAGAAGAAAGA | 0.02% | 0.03% | Invalidated |
| EMX1_26 | Chr4 | 87256692 | GAGTAAGAGAAGAAGAAGAAGGG | 0.08% | 0.09% | Invalidated |
| EMX1_28 | Chr15 | 61646878 | AAGTCAGAGGAGAAGAAGAAGGG | 0.26% | 0.47% | validated |
| EMX1_30 | Chr17 | 54421043 | GAGTCCCAGGAGAAGAAGAGAGG | 0.01% | 0.01% | Invalidated |
| EMX1_31 | Chr19 | 24250503 | GAGTCCAAGCAGTAGAGGAAGGG | 0.01% | 0.02% | Invalidated |
| EMX1_33 | Chr20 | 6653999 | AAGTCCAGACAGAAGAAGAAGGA | 0.11% | 0.14% | Invalidated |

[Table 19]

| | | FANCF | | | | |
|---|---|---|---|---|---|---|
| | | | | | Indel frequency (%) | |
| | Chromosome | Location | DNA seq at a Cleavage sites | (-) RGEN | (+) RGEN | Validation |
| On-Target | Chr11 | 22647338 | GGAATCCCTTCTGCAGCACCTGG | 0.06% | 54.37% | validated |
| FANCF_02 | Chr6 | 8707528 | GGAACCCCGTCTGCAGCACCAGG | 0.05% | 27.79% | validated |
| FANCF_03 | Chr10 | 43410031 | GGAGTCCCTCCTACAGCACCAGG | 0.01% | 5.41% | validated |
| FANCF_04 | Chr17 | 78923978 | AGAGGCCCCTCTGCAGCACCAGG | 0.01% | 3.09% | validated |
| FANCF_05 | ChrX | 86355180 | ACCATCCCTCCTGCAGCACCAGG | 0.02% | 0.35% | validated |
| FANCF_06 | Chr10 | 73463136 | TGAATCCCATCTCCAGCACCAGG | 0.01% | 0.34% | validated |
| FANCF_07 | Chr10 | 37953200 | GGAGTCCCTCCTCAGCACCAGG | 0.01% | 2.75% | validated |
| FANCF_08 | Chr16 | 49671025 | GGAGTCCCTCCTGCAGCACCTGA | 0.00% | 0.82% | validated |
| FANCF_11 | Chr16 | 28615201 | GGCTTCCCTTCTGCAGCCCCAGG | 0.11% | 0.12% | Invalidated |
| FANCF_12 | Chr11 | 66475045 | GGAACACCTTCTGCAGCTCCAGG | 0.00% | 0.07% | validated |
| FANCF_15 | Chr17 | 39675789 | GGGAGTCCATCTGCAGCACCAGG | 0.01% | 0.02% | Invalidated |
| FANCF_16 | Chr17 | 34985068 | GGGTCCGCTTCTGCAGCACCTGG | 0.00% | 0.00% | Invalidated |
| FANCF_17 | Chr17 | 3960376 | GGAACCCCCTCTGCAGCTTCTGG | 0.00% | 0.00% | Invalidated |
| FANCF_18 | Chr13 | 109802140 | AAAATACCTTCTGCAGTACCAGG | 0.02% | 0.01% | Invalidated |
| FANCF_19 | Chr12 | 115467808 | AGGGTCCCTTCTGCAGCCCCTGG | 0.04% | 0.06% | Invalidated |
| FANCF_21 | Chr12 | 2719895 | ACACTCCCTTCTGCAGCACCATG | 0.00% | 0.01% | Invalidated |

[Table 20]

| HEK293-1 | | | | Indel frequency (%) | | |
|---|---|---|---|---|---|---|
| | Chromosome | Location | DNA seq at a Cleavage sites | (-) RGEN | (+) RGEN | Validation |
| On-Target | Chr9 | 110103705 | GGGAAAGACCCAGCATCCGTGGG | 0.04% | 48.67% | validated |
| HEK1_02 | Chr1 | 201992441 | GGGAAAGTCCCAGCATCCTTTGG | 0.05% | 42.76% | validated |
| HEK1_03 | Chr8 | 21121524 | GGGAAGGACCCAGCATCCTGGGG | 0.01% | 21.46% | validated |
| HEK1_04 | Chr9 | 129621098 | GGGAAATACCCAGCATCCAATGG | 0.01% | 1.61% | validated |
| HEK1_05 | Chr8 | 48879627 | GAGAAAAGCCCAGCATCCTTAGG | 0.02% | 0.26% | validated |
| HEK1_06 | Chr22 | 47970525 | GGAAAGACCAAGCATCAGTGGG | 0.00% | 0.06% | validated |
| HEK1_07 | Chr13 | 31633478 | ATGAAAGACCCAGCATCCATTGA | 0.00% | 0.01% | invalidated |
| HEK1_08 | Chr10 | 123054947 | GGGAAAGCCCAGCATCCCTTGG | 1.62% | 17.96% | validated |
| HEK1_14 | Chr12 | 5555206 | GGAGAAAGACCAGCATCCATAGG | 0.00% | 0.01% | invalidated |
| HEK1_15 | Chr11 | 75956264 | TTATAAGACCCAGCATCCGTAAG | 0.01% | 0.09% | validated |
| HEK1_16 | Chr10 | 86303625 | TGGAAAGAAACAGCATCCGTACG | 0.00% | 0.01% | invalidated |

[Table 21]

| HEK293-2 | | | | Indel frequency (%) | | |
|---|---|---|---|---|---|---|
| | Chromosome | Location | DNA seq at a Cleavage sites | (-) RGEN | (+) RGEN | Validation |
| On-Target | Chr5 | 67240614 | GAACACAAAGCATAGACTGCGGG | 0.01% | 59.05% | validated |
| HEK2_02 | Chr4 | 90522184 | GAACACAATGCATAGATTGCCGG | 0.01% | 16.33% | validated |
| HEK2_04 | Chr4 | 53536210 | GAATACTAAGCATAGACTCCAGG | 0.01% | 0.03% | invalidated |
| HEK2_05 | Chr11 | 128509577 | GAATTCAAAGCATAGATTGCAGG | 0.00% | 0.01% | invalidated |
| HEK2_06 | Chr13 | 113428467 | CAATACAAAGGATAGACTGCAGG | 0.01% | 0.02% | invalidated |
| HEK2_07 | Chr20 | 97641 | GAATTCAAAGCATAGATTGCAGG | 0.01% | 0.01% | invalidated |
| HEK2_08 | ChrX | 36949815 | GAAAACAAACATAGAGTGCTGG | 0.00% | 0.00% | invalidated |
| HEK2_09 | Chr1 | 77190507 | TCACACAAACCATAGACTGAGGG | 0.00% | 0.00% | invalidated |
| HEK2_10 | Chr5 | 125365455 | CCACACCAAGCATAGACTTCTGG | 0.00% | 0.01% | invalidated |
| HEK2_11 | Chr5 | 131174461 | AAATACAATGCATAGACTGCTAG | 0.53% | 0.52% | invalidated |
| HEK2_12 | Chr6 | 139353019 | CCAAACAAAACATAGACTGCTGG | 0.00% | 0.01% | invalidated |
| HEK2_13 | Chr9 | 290166 | AAACATAAAGAATAGACTGCAAG | 0.00% | 0.00% | invalidated |
| HEK2_16 | Chr18 | 22360702 | GGAATCAAAGCACAGACTGCAGG | 0.00% | 0.00% | invalidated |
| HEK2_17 | Chr18 | 56307003 | AAGAACAAAACATAGACTGCAGG | 0.01% | 0.04% | validated |
| HEK2_19 | Chr20 | 23101380 | ATACACAGAGCAAAGACTGCAGG | 0.00% | 0.00% | invalidated |
| HEK2_20 | Chr9 | 97332609 | GTAATTAAAGCACAGACTGCTGG | 0.00% | 0.00% | invalidated |
| HEK2_21 | Chr2 | 19844956 | AACTCCAAAGCATATACTGCTGG | 0.01% | 0.01% | invalidated |
| HEK2_22 | Chr15 | 65377019 | GAGCGATAAGCACAGACTGCTGG | 0.00% | 0.00% | invalidated |

[Table 22]

| HEK293-3 | | | | Indel frequency (%) | | |
|---|---|---|---|---|---|---|
| | Chromosome | Location | DNA seq at a Cleavage sites | (-) RGEN | (+) RGEN | Validation |
| On-Target | Chr9 | 110184637 | GGCCCAGACTGAGCACGTGATGG | 0.01% | 66.99% | validated |
| HEK3_02 | Chr1 | 34163192 | ATTCTAGACTGAGCACGTGCAAG | 0.01% | 0.02% | validated |
| HEK3_03 | Chr11 | 134582415 | GGCGCAGACAGAGCACGTGACGA | 0.00% | 0.00% | invalidated |
| HEK3_04 | Chr1 | 47005705 | AGCTCAGACTGAGCAAGTGAGGG | 0.01% | 16.23% | validated |
| HEK3_05 | Chr10 | 131593121 | GAGCCAGAATGAGCACGTGAGGG | 0.00% | 1.17% | validated |
| HEK3_06 | Chr15 | 79749931 | CACCCAGACTGAGCACGTGCTGG | 0.00% | 33.14% | validated |
| HEK3_07 | Chr6 | 103918240 | AAATAAGACTGAGCACGTGGTGG | 0.01% | 0.02% | invalidated |
| HEK3_08 | Chr7 | 66968042 | GACACAGACCGGGCACGTGAGGG | 0.01% | 0.15% | validated |
| HEK3_09 | ChrX | 114764149 | AGACCAGACTGAGCAAGAGAGGG | 0.01% | 0.20% | validated |
| HEK3_10 | Chr15 | 35402774 | CCTAAAGACTGAGCAAGTGAAGG | 0.01% | 0.01% | invalidated |
| HEK3_11 | Chr9 | 137839236 | CAGCCAGACAGAGCACGTGGAGG | 0.02% | 0.02% | invalidated |
| HEK3_12 | Chr6 | 79968440 | AACAAAGACTGAGCACGTTAGGG | 0.01% | 0.01% | invalidated |
| HEK3_13 | Chr2 | 130402896 | GACCCAGAATGAGCACAAAAGGG | 0.10% | 0.10% | invalidated |
| HEK3_14 | Chr2 | 97163211 | CCCATGGACTGAGCACATGAAGG | 0.06% | 0.08% | invalidated |
| HEK3_15 | Chr10 | 22896606 | GAAGGAGACTGAGCATGTGAGGG | 0.00% | 0.00% | invalidated |
| HEK3_16 | Chr8 | 20947876 | TCTCCAGACTGAGCCCATGAGGG | 0.04% | 0.03% | invalidated |
| HEK3_17 | Chr2 | 240026760 | GGCTCAGACTGAGCACCTGAGAG | 0.01% | 0.11% | validated |
| HEK3_18 | Chr14 | 102517106 | CTCGGAGACTGACCACGTGAGGG | 0.04% | 0.05% | invalidated |
| HEK3_19 | Chr10 | 23135503 | ACTCCAGACTGAGCAACTGAGGG | 0.01% | 0.01% | invalidated |
| HEK3_20 | ChrX | 16606309 | TTCCCAGACAAAGCACGCGAAGG | 2.26% | 2.14% | invalidated |

[Table 23]

| | Chromosome | Location | DNA seq at a Cleavage sites | (-) RGEN | (+) RGEN | Validation |
|---|---|---|---|---|---|---|
| | | | HEK293-4 | Indel frequency (%) | | |
| On-Target | Chr20 | 31349773 | GGCACTGCGGCTGGAGGTGGGGG | 0.00% | 82.90% | validated |
| HEK4_02 | Chr19 | 33382081 | GGCTCTGCGGCTGGAGGGGGTGG | 0.14% | 2.84% | validated |
| HEK4_03 | Chr10 | 126694875 | GGCACGACGGCTGGAGGTGGGGG | 0.06% | 11.61% | validated |
| HEK4_04 | Chr15 | 41044242 | GGCGCTGCGGCGGGAGGTGGAGG | 0.02% | 5.25% | validated |
| HEK4_05 | Chr5 | 160517581 | GGCACTGCTGCTGGGGGTGGTGG | 0.15% | 5.38% | validated |
| HEK4_06 | Chr13 | 27629410 | GGCACTGGGGTTGGAGGTGGGGG | 0.02% | 2.16% | validated |
| HEK4_07 | Chr20 | 45343011 | GGCACTGAGGGTGGAGGTGGGGG | 0.02% | 1.55% | validated |
| HEK4_08 | Chr20 | 1151854 | GGCACTGTGGCTGCAGGTGGAGG | 0.01% | 1.44% | validated |
| HEK4_10 | Chr4 | 56815199 | GGCAATGCGGCTGGAGGCGGAGG | 0.02% | 11.90% | validated |
| HEK4_11 | Chr20 | 60010563 | TGCACTGCGGCGGGAGGAGGTGG | 0.01% | 2.83% | validated |
| HEK4_12 | Chr10 | 77103120 | GGCATCACGGCTGGAGGTGGAGG | 0.04% | 5.09% | validated |
| HEK4_13 | Chr19 | 38616186 | GGCACTGAGACTGGGGGTGGGGG | 0.02% | 17.00% | validated |
| HEK4_14 | Chr13 | 39262929 | AGCAGTGCGGCTAGAGGTGGTGG | 0.03% | 12.34% | validated |
| HEK4_15 | Chr10 | 13692637 | GGCACTGGGGCTGGGGGAGGGGG | 0.14% | 0.25% | invalidated |
| HEK4_16 | Chr7 | 54561438 | AGGACTGCGGCTGGGGGTGGTGG | 0.24% | 8.72% | validated |
| HEK4_17 | Chr19 | 41220525 | GGCAATGTGGCTGAAGGTGGGGG | 0.01% | 0.58% | validated |
| HEK4_18 | Chr20 | 60895571 | GGCACAGCAGCTGGAGGTGCTGG | 0.02% | 0.59% | validated |
| HEK4_19 | Chr1 | 171018460 | GCCACTGGGGCTGGGGGTGGGGG | 0.26% | 2.32% | validated |
| HEK4_20 | Chr17 | 176302 | TGCACTGTGGCTGGAGATGGGGG | 0.01% | 1.02% | validated |
| HEK4_21 | Chr13 | 88900992 | CACACTGCAGCTGGAGGTGGTGG | 0.55% | 0.85% | validated |
| HEK4_29 | Chr15 | 89469252 | GGCACTGCGGGAGGAGGTGGCGG | 0.06% | 0.09% | invalidated |
| HEK4_31 | Chr14 | 24740271 | GGCACTGCCACTGGGGGTGAGGG | 0.40% | 0.45% | invalidated |
| HEK4_41 | Chr10 | 1295239 | GGCCCTTCGGCTGGAGGTGGCAG | 0.02% | 0.01% | invalidated |
| HEK4_42 | Chr10 | 60003488 | GGCACGCGGCTGGGAGGTGGAGG | 0.07% | 0.07% | invalidated |
| HEK4_43 | Chr12 | 90904707 | GGCATGCGGCTGGGAGGTGGAGG | 0.03% | 0.03% | invalidated |
| HEK4_45 | Chr15 | 75632142 | GCACCTGCGGCTGGAGGTGGCAG | 0.02% | 0.01% | invalidated |
| HEK4_46 | Chr1 | 2933843 | GGCCCTGAGACTGCAGGTGGAGG | 0.01% | 0.02% | invalidated |
| HEK4_48 | Chr3 | 16815640 | CGCACTGGGGCTGCAGGTGGAGG | 0.66% | 0.74% | invalidated |
| HEK4_50 | Chr4 | 155491955 | TTCACTGTGGCTGGAGGTGGGGA | 0.12% | 0.10% | invalidated |
| HEK4_51 | Chr5 | 41968123 | GGAAGTGCGGCAGGAGGTGGAGG | 0.02% | 0.02% | invalidated |
| HEK4_52 | Chr5 | 177928896 | CCCACTGCGGGTGGAGGTGGAAG | 0.02% | 0.02% | invalidated |
| HEK4_53 | Chr6 | 33950129 | GGCTCTGAGGCTGGTGGTGGGGG | 0.46% | 0.42% | invalidated |
| HEK4_54 | Chr6 | 159190938 | GGCCCTGCAGCTGGAGGAGGAGA | 0.06% | 0.05% | invalidated |
| HEK4_55 | Chr7 | 157889941 | GGCACTGGGGAAGGAGGTGGAGG | 1.81% | 1.90% | invalidated |
| HEK4_56 | Chr8 | 1241126 | GGCACTGTTGCTGGAGGAGGCAG | 0.01% | 0.00% | invalidated |
| HEK4_57 | Chr8 | 11479079 | GGCCCTGCAGCTGGAGATGGAAG | 0.67% | 0.72% | invalidated |
| HEK4_58 | Chr8 | 145730111 | GGCACATGGGCTGGGGGTGGGGG | 0.06% | 0.07% | invalidated |
| HEK4_59 | Chr10 | 36109441 | GGCATTGCTGCTGGTGGTGGTGG | 0.00% | 0.00% | invalidated |
| HEK4_60 | Chr10 | 127971444 | GGAACTGGGGCTGGGGGTGGGGG | 0.01% | 0.20% | validated |

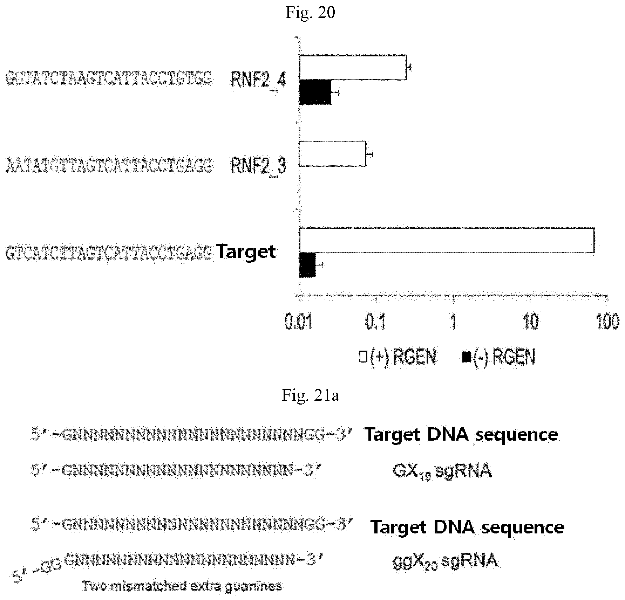

Indels were detected above the background noise level caused by sequencing errors at 116 sites (= 88%) of the 132 sites commonly detected in Digenome-seq and GUIDE-seq. On the other hand, most of the locations detected in Digenome-seq and only in GUIDE-seq were not identified by targeting deep sequencing. On the other hand, the most of the sites detected only in Digenome-seq and GUIDE-seq did not identify indels by targeting deep sequencing. That is, 21 (= 17%) of the 127 sites detected only in the Digenome-seq and 23 (= 51%) of the 45 sites detected only in the GUIDE-seq induced indels above the noise level. It was identified that both of the two methods are not general methods. In most of the validated sites, the indel frequency was less than 1%, much lower than that identified at the corresponding on-target site. For example, RNF2-targeted sgRNAs induced indels at the on-target site and two off-target sites validated in the present disclosure, which showed frequencies of 68%, 0.25%, and 0.09%, respectively (Fig. 20). It can be seen that indels can be induced at a frequency lower than the noise level (0.001% to 4% depending on the site) at sites that are not identified in NGS.

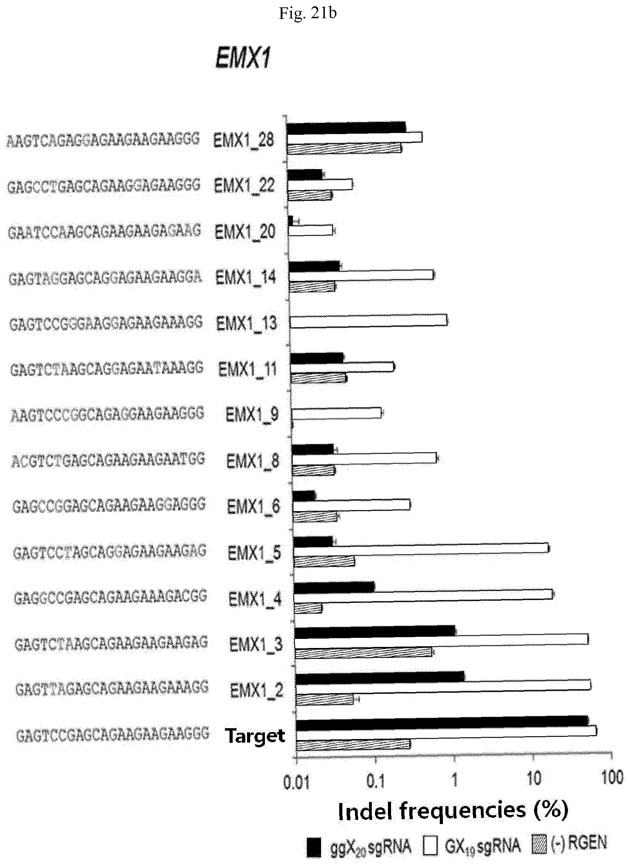

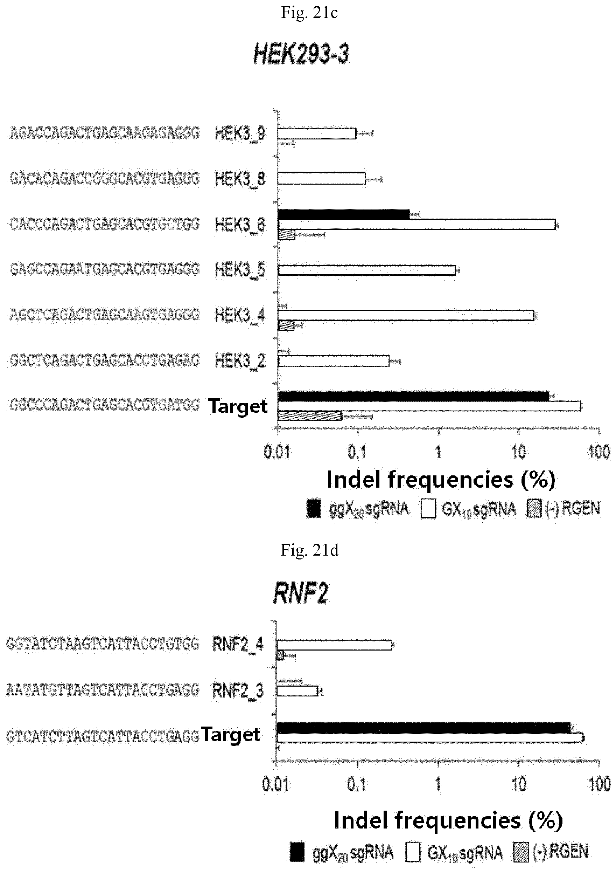

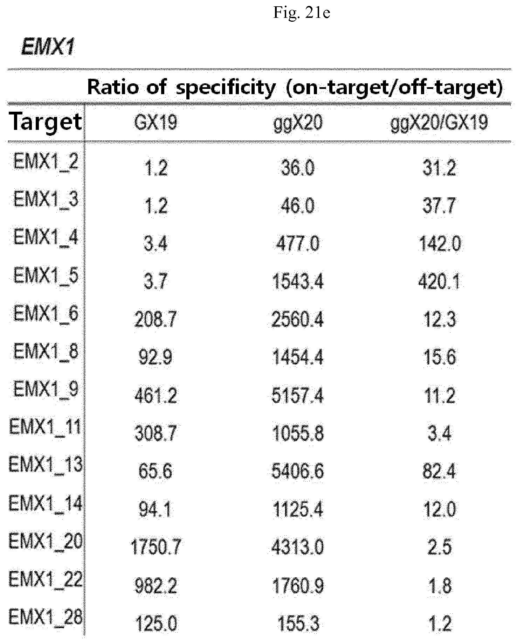

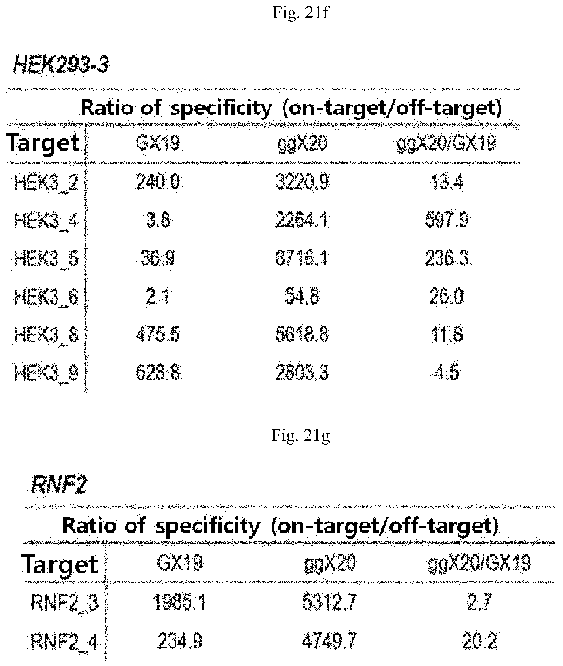

In order to reduce off-target effects, sgRNA (referred to as $ggX_{20}$ sgRNA) including two guanines was additionally used at the 5' end (Fig. 21a). The modified sgRNA was 598 times more specific than the corresponding $GX_{19}$ sgRNA (Figs. 21b-22g). RNF2-specific $ggX_{20}$ sgRNA did not detect off-target indels above the noise level (Fig. 21d).

Experimental Example 11: Indel frequency at an off-target site

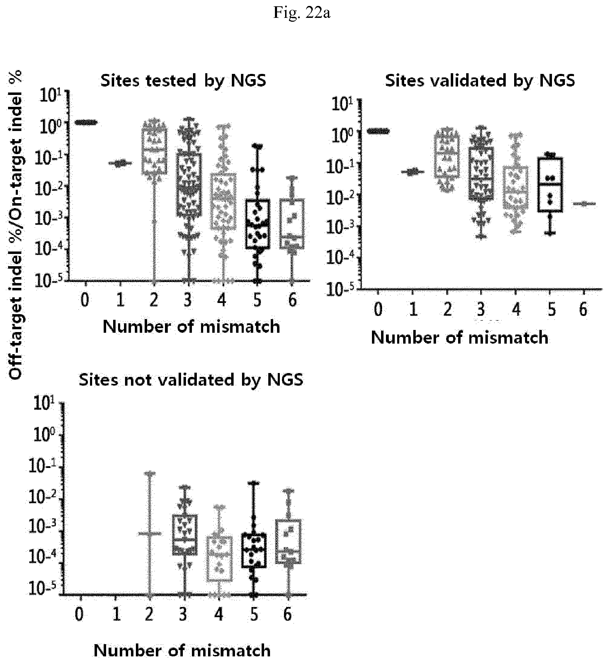

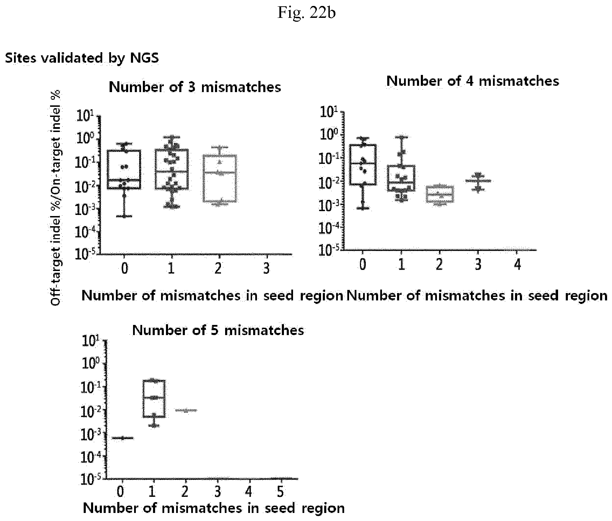

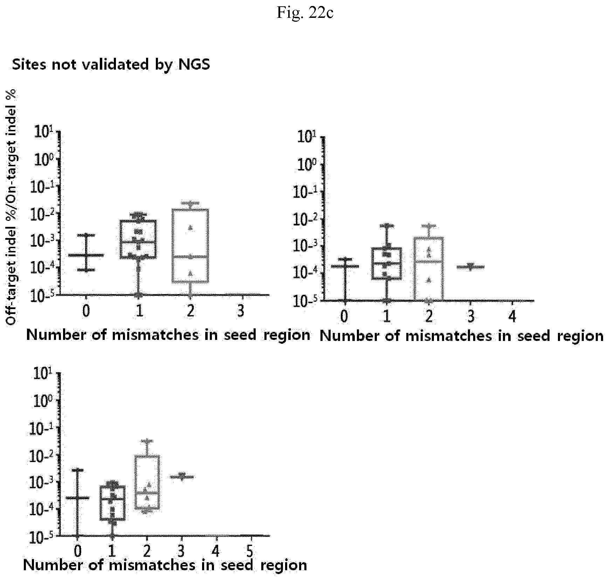

The indel frequency at off-target sites validated by NGS (= 160) and non-validated off-target sites (= 144) were specially used to identify off-target effects. It was identified that the number of mismatch nucleotide and off-target sites with a nucleotide mismatch of 2 or less in the plot of indel frequency of on-target sites and off-target sites were found to be effectively cleaved intracellularly (average indel frequency = 5.38%), and that are not well cleaved in case of having 3 or more nucleotide mismatches (average indel frequency = 0.14% or less) (Fig. 22A). The indel frequency was 60 ± 7% at an on-target site. At validated or unvalidated sites, nucleotide mismatches were distributed almost evenly in the PAM-distal and PAM-proximal regions. The validated or unvalidated sites with 3 or more nucleotide mismatches were as important as PAM-distal sites (Fig. 22b and 23c). That is, at a site having 0 or 1 nucleotide mismatch at the seed site, the indel frequency was as low as the site having 2 or more mismatches.

The results show that the number of potential off-target sites in a genome, the ratio of sites identified by Digenome-seq (Fig. 16a), and the off-target scores (Table 24) calculated from the average indelible frequency of the site (Fig. 20a) were calculated.

[Table 24] Calculation of off-target scores on EMX1 target sequences (5'-GAGTCCGAGCAGAAGAAGAANGG-3') in human genomes

| Number of mismatch nucleotide | Number of mismatch nucleotide at the seed site | Number of potential off-target sites" | Ratio identified by Digenome-seq[b] | Average indelible frequency[c] | Number of potential off-target sites X Ratio identified by Digenome-seq X Average indelible frequency |
|---|---|---|---|---|---|
| 0 | - | 1 | 1.0 | 0.0 | 0.0 |
| 1 or 2 | - | 1 | 1.0 | 0.15 | 0.15 |
| 3 | 0 | 7 | 0.56 | 0.030 | 0.12 |
| | 1 | 7 | 0.44 | 0.0077 | 0.024 |
| | 2 | 4 | 0.12 | 0.0030 | 0.0014 |
| | 3 | 0 | 0.0020 | 0.00010 | 0.0 |
| 4 | 0 | 68 | 0.22 | 0.030 | 0.45 |
| | 1 | 73 | 0.062 | 0.0039 | 0.018 |
| | 2 | 115 | 0.010 | 0.00088 | 0.0010 |
| | 3 | 16 | 0.0013 | 0.00088 | 0.0000 18 |
| | 4 | 4 | 0.0 | 0.0 | 0.0 |
| 5 | 0 | 136 | 0.010 | 0.00067 | 0.00091 |
| | 1 | 674 | 0.010 | 0.00067 | 0.0045 |
| | 2 | 888 | 0.0015 | 0.00067 | 0.00089 |
| | 3 | 521 | 0.00025 | 0.00067 | 0.000087 |
| | 4 | 91 | 0.0 | 0.0 | 0.0 |
| | 5 | 3 | 0.0 | 0.0 | 0.0 |
| 6 | 0 | 426 | 0.0067 | 0.00026 | 0.00074 |
| | 1 | 2641 | 0.0017 | 0.00026 | 0.0012 |
| | 2 | 5673 | 0.000047 | 0.00026 | 0.000069 |
| | 3 | 4954 | 0.000047 | 0.00026 | 0.000061 |
| | 4 | 1846 | 0.0 | 0.0 | 0.0 |
| | 5 | 197 | 0.0 | 0.0 | 0.0 |
| | 6 | 10 | 0.0 | 0.0 | 0.0 |

(continued)

| Number of mismatch nucleotide | Number of mismatch nucleotide at the seed site | Number of potential off-target sites" | Ratio identified by Digenome-seq[b] | Average indelible frequency[c] | Number of potential off-target sites X Ratio identified by Digenome-seq X Average indelible frequency |
|---|---|---|---|---|---|
| | | | | off-target score: | 0.77 |

[a] Obtained by using Cas-OFFinder
[b] Identified as shown in Fig. 16b
[c] Identified by targeted deep sequencing (Fig. 22a).

To summarize the above results, the present inventors have developed a Digenome-seq method capable of detecting the off-target site of the programmable nuclease, which is highly reproducible compared to other conventional methods, and is configured to easily detect off-target sites. Furthermore, the present inventors developed an *in vitro* DNA cleavage scoring system and developed an enhanced Digenome-seq that can reduce false positive and false negative site numbers using sgRNA transcribed from a plasmid template rather than a synthetic oligonucleotide double strand. In addition, a multiplex Digenome-seq was performed by cleaving genomic DNA with 11 sgRNA mixtures, and an average of 70 additional cleavage sites per sgRNA, which were not detected in GUIDE-seq, were identified. Off-target indels were induced in many of these sites in RGEN-transformed human cells. Thus, by examining the indel frequency, the number of nucleotide mismatches, and the site of mismatches in hundreds of off-target sites, it was identified that the PAM-distal region in the RGEN specificity is as important as the seed region. In addition, it has been identified that sites having two or more nucleotide mismatches at the seed site are not cleaved *in vitro* compared to the case where the total mismatch nucleotide number is none or one.

Experimental Example 12: Large scale multiplex Digenome-seq

The present inventors tried to identify whether off-target sites can be efficiently detected even in case of expanding the target of the multiplex Digenome-seq on a large scale.

Specifically, the multiplex Digenome-seq was performed for each different 100 on-target sites. Even if on-target sites were expanded to 100, off-target sites for the 100 targets could be efficiently detected through Digenome-seq.

In this regard, after fining the sites having 6 or less of nucleotide mismatch(es) with respect to an on-target sites through a computer program, this portion was classified as a cleavage site by RGEN and non-cleavage site. Next, the difference between the sequence of the cleavage site and the sequence of the non-cleavage site was analyzed through machine learning based on the neural network, and a program capable of predicting the off-target site with respect to the on-target site was produced. It was found that a larger number of off-target sites can be detected in comparison with other programs (crop-it) that have been developed through the program (Fig. 23).

Experimental Example 13: Digenome-seq for ZFN

Furthermore, the present inventors also tried to detect off-target sites of ZFN instead of RGEN by the same approach.

Like RGEN, ZFN protein was treated by cell-free genomic DNA isolated *in vitro* and then WGS was performed. In the case of ZFN, it was identified that vertical alignment occurred when the on-target site was observed through the IGV (Fig. 24a), and a cleavage score was given on the entire genomic scale (Fig. 24b). It was identified that the sequence logo obtained by comparing the DNA sequence around the cleavage site *in vitro* coincides with the target sequence at most sites (Fig. 24c and Fig. 24d).

Targeted deep sequencing was performed after transformation through ZFN for a portion of the on-target site and off-target site candidates resulting from Digenome-seq that has 4 or less nucleotide mismatch regions (Table 25).

[Table 25]

| | 1st | | 2nd | | | 1st | | 2nd | |
|---|---|---|---|---|---|---|---|---|---|
| | (-) ZFN | (+) ZFN | (-) ZFN | (+) ZFN | | (-) ZFN | (+) ZFN | (-) ZFN | (+) ZFN |
| ZFN-224_01 | 0.004% | 5.690% | 0.002% | 5.920% | ZFN-224_28 | 0.000% | 1.441% | 0.000% | 1.971% |
| ZFN-224_02 | 0.000% | 4.057% | 0.000% | 4.240% | ZFN-224_29 | 0.000% | 0.432% | 0.000% | 0.429% |
| ZFN-224_03 | 0.000% | 1.940% | 0.000% | 1.866% | ZFN-224_32 | 0.000% | 0.059% | 0.006% | 0.047% |
| ZFN-224_04 | 0.006% | 0.055% | 0.015% | 0.038% | ZFN-224_33 | 0.000% | 0.078% | 0.000% | 0.076% |
| ZFN-224_05 | 0.000% | 0.218% | 0.000% | 0.218% | ZFN-224_34 | 0.000% | 0.046% | 0.000% | 0.026% |
| ZFN-224_06 | 0.000% | 0.678% | 0.009% | 0.717% | ZFN-224_35 | 0.000% | 0.281% | 0.000% | 0.274% |
| ZFN-224_07 | 0.000% | 0.162% | 0.014% | 0.151% | ZFN-224_37 | 0.005% | 0.073% | 0.014% | 0.088% |
| ZFN-224_08 | 0.000% | 0.084% | 0.003% | 0.086% | ZFN-224_44 | 0.017% | 0.031% | 0.017% | 0.036% |
| ZFN-224_10 | 0.007% | 0.107% | 0.004% | 0.110% | ZFN-224_45 | 0.000% | 0.080% | 0.000% | 0.130% |
| ZFN-224_11 | 0.000% | 0.075% | 0.003% | 0.042% | ZFN-224_46 | 0.031% | 0.346% | 0.022% | 0.258% |
| ZFN-224_12 | 0.000% | 0.179% | 0.019% | 0.163% | ZFN-224_48 | 0.020% | 1.510% | 0.021% | 1.426% |
| ZFN-224_14 | 0.016% | 0.094% | 0.040% | 0.130% | ZFN-224_49 | 0.000% | 0.226% | 0.013% | 0.252% |
| ZFN-224_17 | 0.022% | 0.169% | 0.016% | 0.161% | ZFN-224_51 | 0.000% | 2.507% | 0.004% | 2.827% |
| ZFN-224_19 | 0.008% | 0.029% | 0.000% | 0.030% | ZFN-224_55 | 0.006% | 0.048% | 0.016% | 0.048% |
| ZFN-224_22 | 0.000% | 0.067% | 0.032% | 0.192% | ZFN-224_56 | 0.000% | 1.261% | 0.007% | 1.217% |
| ZFN-224_23 | 0.006% | 0.030% | 0.000% | 0.025% | ZFN-224_59 | 0.010% | 0.042% | 0.003% | 0.139% |
| ZFN-224_24 | 0.000% | 0.116% | 0.003% | 0.121% | ZFN-224_62 | 0.008% | 0.074% | 0.020% | 0.086% |
| ZFN-224_25 | 0.000% | 0.199% | 0.000% | 0.173% | | | | | |

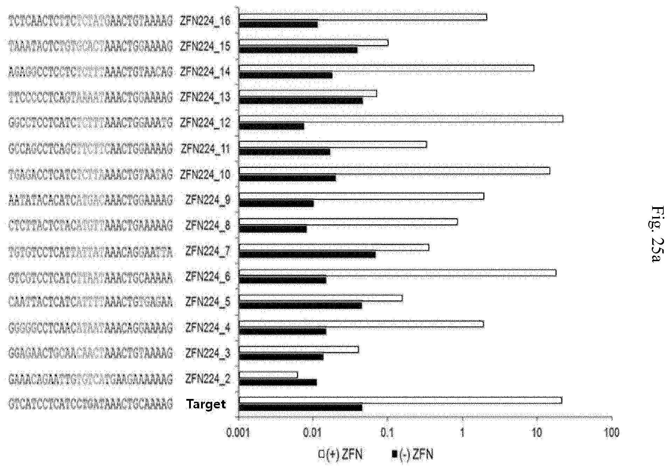

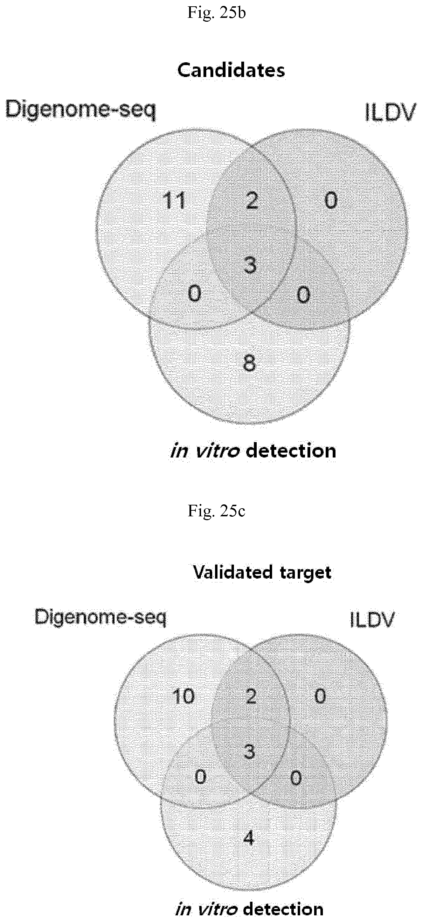

As a result, it was identified that indels were present in 35 on-target and off-target sites out of 62 off-target site candidates. Specifically, it was identified that 0.028% to 5.9% was induced (Table 25). This shows that the Digenome-seq method also predicts the off-target site of the ZFN. In the case of ZFN made by modifying (KK or EL) at the FokI site, the specificity was increased (Fig. 24). As such, a total of 16 off-target site candidates were found when Digenome-seq was performed through FokI modified ZFN. It was also identified that indels occurred at 15 of 16 off-target site candidates in cells transformed by using FokI-modified ZFN, and this indicates that a large number of off-targets sites can be found compared to the conventional other methods (ILDV, in vitro selection) (Fig. 25).

In conclusion, the above results suggest that the Digenome-seq of the present disclosure can be applied to any programmable nuclease that can have RGEN, ZFN as well as on-target and off-target sites.

**Claims**

1.  An *in vitro* method for detecting an off-target site in a genome comprising:

    (a) cleaving an isolated genomic DNA with a target-specific programmable nuclease;
    (b) performing next generation sequencing of the cleaved DNA; and
    (c) determining a cleaved site in a sequence read obtained by the sequencing,
    wherein the method further comprises determining the cleaved site as an off-target site, in case where the cleaved site is not an on-target site, and wherein the cleaved site is a site where the 5' end is vertically aligned by aligning the obtained sequence reads or a site showing double-peak patterns at the 5' end plot, and
    wherein the method further comprises determining a site where 20% or more of sequence reads is vertically aligned and the number of sequence reads having the same 5' end in each of the Watson and Crick strands is 10 or more as an off-target site.

2.  The method according to claim 1, wherein the genomic DNA is isolated from cells expressing or not expressing the target-specific programmable nuclease.

3. The method according to claim 1, wherein the aligning is performed by mapping the sequence read to the reference genome and then analyzing with an analysis program.

4. The method according to claim 1, further comprising determining a site where two or more sequence reads corresponding to Watson strand and Crick strand are separately aligned vertically as an off-target site.

5. The method according to claim 1, wherein the isolated genomic DNA is isolated from cells expressing a programmable nuclease, and further comprising determining an off-target effect by identifying indels (insertion and deletion) at an off-target site of the DNA, preferably the indels are identified by performing a mutant detection using T7E1 analysis on the off-target site and Cel-I enzyme, or targeted deep sequencing.

6. The method according to claim 1, wherein the off-target site has one or more nucleotide mismatch(es) to the target site, preferably the off-target site has 1 to 6 nucleotide mismatch(es) to the target site.

7. The method according to claim 1, wherein the step (c) is performed by calculating cleavage score with a formula as follows at each cleaved site:

$$\text{Score at the } i \text{ site } =$$

$$\sum_{a=1}^{5} \frac{C(F_i-1)}{D_i} \times \frac{C(R_{i-4+a}-1)}{D_{i-4+a}} \times (F_i + R_{i-4+a} - 2)$$

$$+ \sum_{a=1}^{5} \frac{C(R_{i-1}-1)}{D_{i-1}} \times \frac{C(F_{i-3+a}-1)}{D_{i-3+a}} \times (R_{i-a} + F_{i-3+a} - 2)$$

$F_i$ : Number of forward sequence reads starting at the $i$ site
$R_i$ : Number of reverse sequence reads starting at the $i$ site
$Di$ : Sequencing depth at the /site
$C$ : Arbitrary constant

8. The method according to claim 7 further comprising determining the cleaved site as an off-target site, when the constant C is 100 and the calculated score is 25,000 or more in the formula.

9. The method according to claim 1, wherein the programmable nuclease is a mixture of programmable nucleases for 2 or more targets, preferably the programmable nuclease is a mixture of programmable nucleases for 2 to 100 targets.

10. The method according to claim 9 further comprising classifying an off-target site according to the edit distance to the on-target site.

11. The method according to claim 1, wherein the programmable nuclease is selected from the group consisting of meganuclease, ZFN (zinc finger nuclease), TALEN (transcription activator-like effector nuclease), RGEN (RNA-guided engineered nuclease), and Cpfl, preferably

(i) the meganuclease is selected from the group consisting of I-Scel, I-Ceul, PI-Pspl and PI-Scel; or
(ii) the Cpfl is derived from one selected from the group consisting of *CandidatusPaceibacter, Lachnospira genus, Butyrivibrio genus, Peregrinibacteria, Acidominococcus genus, Porphyromonas genus, Prevotella genus, Francisella genus, Candidatus methanoplasma,* and *Eubacterium genus.*

12. The method according to claim 11, wherein RGEN comprises a guide RNA binding specifically to a sequence of a target gene and Cas protein, preferably

(i) the guide RNA is transcribed from an oligonucleotide double strand or a plasmid template; or
(ii) the guide RNA is a dual RNA comprising a crRNA and a tracrRNA or a single chain guide RNA; or
(iii) the Cas protein is Cas9 protein or a variant of Cas9 protein; or
(iv) the Cas protein is derived from one selected from the group consisting of a genus *Streptococcus,* a genus *Neisseria,* a genus *Pasteurella,* a genus *Francisella,* and a genus *Campylobacter.*

**Patentansprüche**

1. In-vitro-Verfahren zum Nachweis einer Off-Target-Stelle in einem Genom, umfassend:

   (a) Spalten einer isolierten genomischen DNA mit einer zielspezifischen programmierbaren Nuclease;
   (b) Durchführen einer Sequenzierung der nächsten Generation mit der gespaltenen DNA; und
   (c) Bestimmen einer Spaltstelle in einer durch die Sequenzierung erhaltenen Sequenzablesung,
   wobei das Verfahren weiterhin das Bestimmen der Spaltstelle als Off-Target-Stelle umfasst, und zwar wenn die Spaltstelle keine On-Target-Stelle ist, und
   wobei die Spaltstelle eine Stelle ist, wo das 5'-Ende durch Alignieren der erhaltenen Sequenzablesungen vertikal aligniert ist, oder eine Stelle ist, die im Plot des 5'-Endes Doppel-Peak-Muster zeigt; und
   wobei das Verfahren weiterhin das Bestimmen einer Stelle, an der 20% oder mehr der Sequenzablesungen vertikal aligniert sind und die Anzahl der Sequenzablesungen, die im Watson- und im Crick-Strang jeweils dasselbe 5'-Ende aufweisen, 10 oder mehr beträgt, als Off-Target-Stelle umfasst.

2. Verfahren gemäß Anspruch 1, wobei die genomische DNA aus Zellen isoliert ist, die die zielspezifische programmierbare Nuclease exprimieren oder auch nicht.

3. Verfahren gemäß Anspruch 1, wobei das Alignieren dadurch erfolgt, dass man die Sequenzablesung auf das Referenzgenom abbildet und dann mit einem Analyseprogramm analysiert.

4. Verfahren gemäß Anspruch 1, weiterhin umfassend das Bestimmen einer Stelle, wo zwei oder mehr Sequenzablesungen, die dem Watson-Strang und dem Crick-Strang entsprechen, als Off-Target-Stelle vertikal separat aligniert sind.

5. Verfahren gemäß Anspruch 1, wobei die isolierte genomische DNA aus Zellen isoliert ist, die eine programmierbare Nuclease exprimieren, weiterhin umfassend das Bestimmen eines Off-Target-Effekts durch Identifizieren von Indels (Insertion und Deletion) an einer Off-Target-Stelle der DNA, wobei vorzugsweise die Indels dadurch identifiziert werden, dass man einen Mutantennachweis mit Hilfe einer T7E1-Analyse der Off-Target-Stelle und eines Cel-I-Enzyms oder mit Hilfe einer Targeted Deep Sequenzierung durchführt.

6. Verfahren gemäß Anspruch 1, wobei die Off-Target-Stelle eine oder mehrere Nucleotidfehlpaarungen mit der Zielstelle aufweist, wobei vorzugsweise die Off-Target-Stelle 1 bis 6 Nucleotidfehlpaarungen mit der Zielstelle aufweist.

7. Verfahren gemäß Anspruch 1, wobei Schritt (c) durchgeführt wird, indem man an jeder Spaltstelle mit der folgenden Formel einen Spaltungs-Score berechnet:

$$\text{Score an der Stelle i} =$$

$$\sum_{a=1}^{5} \frac{C(F_i - 1)}{D_i} \times \frac{C(R_{i-4+a} - 1)}{D_{i-4+a}} \times (F_i + R_{i-4+a} - 2)$$

$$+ \sum_{a=1}^{5} \frac{C(R_{i-1} - 1)}{D_{i-1}} \times \frac{C(F_{i-3+a} - 1)}{D_{i-3+a}} \times (R_{i-a} + F_{i-3+a} - 2)$$

$F_i$: Zahl der Vorwärtssequenzablesungen beginnend an der Stelle i
$R_i$: Zahl der Rückwärtssequenzablesungen beginnend an der Stelle i
$D_i$: Sequenzierungstiefe an der Stelle i
$C$: willkürliche Konstante.

8. Verfahren gemäß Anspruch 7, weiterhin umfassend das Bestimmen der Spaltstelle als Off-Target-Stelle, wenn die Konstante C 100 beträgt und der berechnete Score in der Formel 25 000 oder mehr beträgt.

**9.** Verfahren gemäß Anspruch 1, wobei die programmierbare Nuclease ein Gemisch von programmierbaren Nucleasen für 2 oder mehr Ziele ist, wobei vorzugsweise die programmierbare Nuclease ein Gemisch von programmierbaren Nucleasen für 2 bis 100 Ziele ist.

**10.** Verfahren gemäß Anspruch 9, weiterhin umfassend das Klassifizieren einer Off-Target-Stelle gemäß der Edit-Distanz zur On-Target-Stelle.

**11.** Verfahren gemäß Anspruch 1, wobei die programmierbare Nuclease aus der Gruppe ausgewählt ist, die aus Meganuclease, ZFN (Zinkfinger-Nuclease), TALEN (transkriptionsaktivatorartiger Effektornuclease), RGEN (RNA-gesteuerter rekombinanter Nuclease) und Cpfl besteht, wobei vorzugsweise

(i) die Meganuclease aus der Gruppe ausgewählt ist, die aus I-SceI, I-CeuI, PI-PspI und PI-SceI besteht; oder
(ii) das Cpfl von einem Organismus stammt, der aus der Gruppe ausgewählt ist, die aus Candidatus *Paceibacter,* der Gattung *Lachnospira,* der Gattung *Butyrivibrio,* Peregrinibacteria, der Gattung *Acidominococcus,* der Gattung *Porphyromonas,* der Gattung *Prevotella,* der Gattung *Francisella,* Candidatus *Methanoplasma* und der Gattung *Eubacterium* besteht.

**12.** Verfahren gemäß Anspruch 11, wobei RGEN eine Guide-RNA umfasst, die spezifisch an eine Sequenz eines Zielgens und an Cas-Protein bindet, wobei vorzugsweise

(i) die Guide-RNA ausgehend von einem Oligonucleotid-Doppelstrang oder einer Plasmidmatrize transkribiert wird; oder
(ii) die Guide-RNA eine duale RNA, die eine crRNA und eine tracrRNA umfasst, oder eine einzelkettige Guide-RNA ist; oder
(iii) das Cas-Protein ein Cas9-Protein oder eine Variante des Cas9-Proteins ist; oder
(iv) das Cas-Protein von einem Organismus stammt, der aus der Gruppe ausgewählt ist, die aus der Gattung *Streptococcus,* der Gattung *Neisseria,* der Gattung *Pasteurella,* der Gattung *Francisella* und der Gattung *Campylobacter* besteht.

## Revendications

**1.** Procédé in vitro pour détecter une site hors cible dans un génome, comprenant les étapes consistant à :

(a) cliver un ADN génomique isolé avec une nucléase programmable spécifique de cible,
(b) effectuer un séquençage de nouvelle génération avec l'ADN clivé, et
déterminer une site clivée dans une lecture de séquence obtenue dans le séquençage,
dans lequel ledit procédé comprend en outre l'étape consistant à déterminer la site clivée comme site hors cible dans un cas où la site clivée n'est pas une site sur cible, et
dans lequel ladite site clivée est une site où l'extrémité 5' est alignée verticalement en alignant les lectures de séquence obtenues, ou une site présentant des motifs à double pic sur le tracé 5', et
dans lequel ledit procédé comprend en outre l'étape consistant à déterminer une site où 20 % ou plus de lectures de séquence sont alignées verticalement, et le nombre de lectures de séquence ayant la même extrémité 5' dans chacun des brins Watson et Crick est 10 ou plus comme site hors cible.

**2.** Procédé selon la revendication 1, dans lequel ledit ADN génomique est isolé de cellules exprimant ou non ladite nucléase programmable spécifique de cible.

**3.** Procédé selon la revendication 1, dans lequel ladite étape consistant à aligner est effectuée en appliquant la lecture de séquence sur le génome de référence, et ensuite analysant avec un programme d'analyse.

**4.** Procédé selon la revendication 1, comprenant en outre l'étape consistant à déterminer une site deux ou plusieurs lectures de séquence correspondant au brin Watson et au brin Crick sont alignées verticalement séparément comme site hors cible.

**5.** Procédé selon la revendication 1, dans lequel ledit ADN génomique isolé est isolé de cellules exprimant une nucléase programmable, comprenant en outre l'étape consistant à déterminer un effet hors cible en identifiant des indels (insertion et délétion) sur une site hors cible dudit ADN, de préférence les indels sont identifiés en effectuant une détection de mutantes en utilisant une analyse T7E1 de la site hors cible et de l'enzyme Cel-I, ou par séquençage profond ciblé.

**6.** Procédé selon la revendication 1, dans lequel la site hors cible présente un ou plusieurs mésappariements de nucléotides avec la site cible, de préférence la site hors cible présente 1 à 6 mésappariements de nucléotides avec la site cible.

**7.** Procédé selon la revendication 1, dans lequel l'étape (c) est effectuée en calculant un score de clivage avec la formule suivante à chaque site clivée :

$$\text{score au site } i =$$

$$\sum_{a=1}^{5} \frac{C(F_i - 1)}{D_i} \times \frac{C(R_{i-4+a} - 1)}{D_{i-4+a}} \times (F_i + R_{i-4+a} - 2)$$

$$+ \sum_{a=1}^{5} \frac{C(R_{i-1} - 1)}{D_{i-1}} \times \frac{C(F_{i-3+a} - 1)}{D_{i-3+a}} \times (R_{i-a} + F_{i-3+a} - 2)$$

$F_i$ : nombre des lectures de séquence sens commençant au site i
$R_i$: nombre des lectures de séquence anti-sens commençant au site i
$D_i$: profondeur de séquençage au site i
C: constante arbitraire.

**8.** Procédé selon la revendication 7, comprenant en outre l'étape consistant à déterminer la site clivée comme site hors cible, lorsque la constante C est 100 et le score calculé est 25 000 ou plus dans la formule.

**9.** Procédé selon la revendication 1, dans lequel la nucléase programmable est un mélange de nucléases programmables pour 2 ou plusieurs cibles, de préférence la nucléase programmable est un mélange de nucléases programmables pour 2 à 100 cibles.

**10.** Procédé selon la revendication 9, comprenant en outre l'étape consistant à classer une site hors cible selon la distance d'édition au site sur cible.

**11.** Procédé selon la revendication 1, dans lequel la nucléase programmable est choisie dans le groupe consistant en méganucléase, ZFN (nucléase type doigt de zinc), TALEN (nucléase-effecteur de type activateur de transcription), RGEN (nucléase modifiée guidée par ARN), et Cpfl, de préférence

(i) ladite méganucléase est choisie dans le groupe consistant en I-SceI, I-CeuI, PI-PspI et PI-SceI, ou
(ii) ledit Cpfl est dérivé d'un organisme choisi dans le groupe consistant en Candidatus *Paceibacter,* le genre *Lachnospira,* le genre *Butyrivibrio,* Peregrinibacteria, le genre *Acidominococcus,* le genre *Porphyromonas,* le genre *Prevotella,* le genre *Francisella,* Candidatus *Methanoplasma,* et le genre *Eubacterium.*

**12.** Procédé selon la revendication 11, dans lequel RGEN comprend un ARN de guide se liant spécifiquement à une séquence d'un gène cible et la protéine Cas, de préférence

(i) ledit ARN de guide est transcrit d'un brin double d'oligonucléotide ou d'une matrice de plasmide, ou
(ii) ledit ARN de guide est un ARN double comprenant un crRNA et un tracrRNA, ou un ARN de guide à simple chaîne, ou
(iii) ladite protéine Cas est une protéine Cas9, ou une variante de la protéine Cas9, ou
(iv) ladite protéine Cas est dérivée d'un organisme choisi dans le groupe consistant en le genre *Streptococcus,* le genre *Neisseria,* le genre *Pasteurella,* le genre *Francisella,* et le genre *Campylobacter.*

Fig. 1a

Fig. 1b

| | Target sequences |
|---|---|
| Target | CTTGCCCCACAGGGCAGTAACGG |
| OT1 | TCAGCCCCACAGGGCAGTAAGGG |
| OT3 | GCTGCCCCACAGGGCAGCAAAGG |
| OT7 | CCTCTCCCACAGGGCAGTAAAGG |
| OT12 | CCTGTCCCACAGGGCAGGAAGGG |

Fig. 1c

Indel frequencies (%)

Fig. 1d

Indel frequencies (%)

Fig. 2a

Fig. 2b

Fig. 2c

Fig. 3a

## OT1

| Non-transformed cell | RGEN-transformed cell |

(-) RGEN

(+) RGEN

Fig. 3b

## OT3

| Non-transformed cell | RGEN-transformed cell |

(-) RGEN

(+) RGEN

**Wild type** GCTGCCCCACAGGGCAG-CAAAGG
**Mutant type** GCTGCCCCACAGGGCAGCCAAAGG +1

Fig. 3c

## OT7

Fig. 3d

## OT12

Fig. 4a

5' T G T A G C T A C A G T G G A G G T T A C T G A T G G C A C C T C T C T T G C T A G 3'

Reverse sequence reads starting
from this point (number : 12)

Forward sequence reads starting
from this point (number : 14)

Depth of this point: 19

Depth of this point: 21

Fig. 4b

# OT1

Fig. 4c

OT3

Fig. 5a

**Non-transformed** Digenome
(300nM Cas9)

**RGEN-transformed** Digenome
(300nM Cas9)

4    59    50

15

0        1

1

**Non-transformed** Digenome
(3nM Cas9)

Fig. 5b

C T T G C C C A C A G G G C A G T A A N G G

Fig. 5c

Fig. 5d

| Total | | Site detected by Digenome | | Site not-detected by Digenome | |
|---|---|---|---|---|---|
| Number of mismatch | Number of sites | Total number | Validated number of sites | Total number | Validated number of sites |
| 0 | 1 | 1 | 1 | 0 | 0 |
| 1 | 0 | 0 | 0 | 0 | 0 |
| 2 | 0 | 0 | 0 | 0 | 0 |
| 3 | 15 | 7 | 4 | 8 | 0 |
| 4 | 142 | 14 | 0 | 128 | N.D. |
| 5 | 1,191 | 15 | 0 | 1,176 | N.D. |
| 6 | 7,896 | 1 | 0 | 7,895 | N.D. |

Fig. 5e

Fig. 6

**a** Non-transformed HAP1

(chr17:20,082,220-20,082,620)

▲

10-bp insertion

```
hg19 : TGGCT----------AATAA
HAP1 : TGGCTTAGGAAGAAAAATAA
```

**b** RGEN-transformed HAP1

(chr15:86,453,445-86,453,845)

▲

32-bp insertion

```
hg19 : AGT--------------------------------TCT
HAP1 : AGTACTGCTCAGCATTACGTGGAGGAACAGCTGAATCT
```

**c** Cas9 only treated HAP1

(chr17:21,251,293-21,251,493)

▲

10-bp insertion

```
hg19 : AGCAC----------AATAA
HAP1 : AGCACTGTAAAGAAAAATAA
```

Fig. 7

**a** *HBB_*48

```
GCTATTGCCCCACGGGGCAG-TGACGGTAC      WT
GCTATTGCCCCACGGGGCAG------GGTAC     -4
GCTATTG--------------------ACGGTAC  -15
GCTATTGCCCCACGG------------TAC      -11
GCTATTGCCCCACGGGGCAGTTGACGGTAC      +1
GCTATTGCCCCACGGGGCAGATGACGGTAC      +1
```

**b** *HBB_*75

```
GTTGTGGCCCCACAGGGCAG-GAATGGCAGCG    WT
GTTGTGGCCCCACAGGGCAG----------CG    -9
GTTGTGGCCCCACAGGGCAGGGAATGGCAGCG    +1
GTTGTGGCCCCACAGGGCAG--AATGGCAGCG    -1
GTTGTGGC--------------------AGCG    -19
GTTGTGG---------------AATGGCAGCG    -14
```

Fig. 8a

Fig. 8b

Fig. 8c

Fig. 8d

| Total | | Site detected by Digenome | | Site not-detected by Digenome | |
|---|---|---|---|---|---|
| Number of mismatch | Number of sites | Total number | Validated number of sites | Total number | Validated number of sites |
| 0 | 1 | 1 | 1 | 0 | 0 |
| 1 | 1 | 1 | 1 | 0 | 0 |
| 2 | 4 | 3 | 3 | 1 | 0 |
| 3 | 32 | 5 | 2 | 27 | 0 |
| 4 | 283 | 12 | 1 | 271 | N.D. |
| 5 | 2,176 | 7 | 0 | 2,169 | N.D. |
| 6 | 13,892 | 6 | 1 | 13,886 | N.D. |

Fig. 8e

Fig. 9a

VEGF-A_ Target

Fig. 9b

## *VEGF-A_03*

Fig. 9c

## VEGF-A_58

Fig. 9d

# *VEGF-A_63*

Fig. 10

**a** *VEGF-A 26*

```
TATGCGTGGGGGGTGTTTGC-TCCCGGGCA   WT
AG-----------(-37bp)--------GT   -37
TATGCGTGGG---(-29bp)--------AG   -29
TATGCGTGGGGGGTGTTTGCTTCCCGGGCA   +1
TATGCGTCCCGGG---------------CA   -14
TATGCGTGGGGGGTGTTTGC--------A    -8
```

**b** *VEGF-A 76*

```
CAGCCAGGA-GCAAGCTCCCTCCACTAAAC   WT
CAGCCAGGAGGCAAGCTCCCTCCACTAAAC   +1
------------AAGCTCCCTCCACTAAAC   -11
CA----------(-25bp)------CTAAAC  -25
CAGC-------------TCCCTCCACTAAAC  -11
CAGC----------AAGCTCCCTCCACTAAAC -7
```

**c** *VEGF-A 35*

```
CTGCCTGGA-GCAAGCTTCCCCCCGGGCCC   WT
CTGCCTGGAAGCAAGCTTCCCCCCGGGCCC   +1
CTGCCTGGAGGCAAGCTTCCCCCCGGGCCC   +1
CTGCCTGA--GCAAGCTTCCCCCCGGGCCC   -1
---------------GAATTCCCCCCGGGCCC -15, +3
```

**d** *VEGF-A 84*

```
CGGTGGGTGGGGGGGAGTTTGCCCCAGGCCA  WT
AC----------(-42bp)---------GA   -42
CGGTGGGTGGGG----------------GA   -33
AG----------(-36bp)------GCCA    -36
CGGTGGGTGGGGGGGAGTTTGCC-------A  -7
CGGTGGGTG------------------CC    -23
```

Fig. 11

Score at the $i$ site $=$

$$\sum_{a=1}^{5} \frac{C(F_i-1)}{D_i} \times \frac{C(R_{i-4+a}-1)}{D_{i-4+a}} \times (F_i + R_{i-4+a} - 2)$$

$$+ \sum_{a=1}^{5} \frac{C(R_{i-1}-1)}{D_{i-1}} \times \frac{C(F_{i-3+a}-1)}{D_{i-3+a}} \times (R_{i-a} + F_{i-3+a} - 2)$$

$F_i$ : Number of forward sequence reads starting at the $i$ site
$R_i$ : Number of reverse sequence reads starting at the $i$ site
$Di$ : Sequencing depth at the $i$ site

# Example:

| Number | | 75006253 | 75006254 | 75006255 | 75006256 | 75006257 | 75006258 | 75006259 |
|---|---|---|---|---|---|---|---|---|
| Number | Reverse | 0 | 0 | 1 | 22 | 0 | 0 | 0 |
| | Forward | 0 | 0 | 0 | 9 | 9 | 1 | 0 |
| Depth | | 23 | 23 | 23 | 31 | 18 | 19 | 19 |

## Score at 75006256 site

= 10000 * (22-1)/31 * [(0-1)/23 * (22+0-2) + (9-1)/31 * (22+9-2) + (9-1)/18 * (22+9-2) + (1-1)/19 * (22+1-2) + (0-1)/19 * (22+0-2)]

+ 10000 * (9-1)/18 * [(0-1)/23 * (9+0-2) + (1-1)/23 * (9+1-2) + (22-1)/31 * (9+22-2) + (0-1)/18 * (9+0-2) + (0-1)/19 * (9+0-2)]

= 206,600

Fig. 12a

Fig. 12b

Fig. 12c

**Oligonucleotide template**

**Plasmid template**

Fig. 13

## DNA cleavage score

Fig. 14

```
5'- GTTACCCC-CAGGGAAGTATAGG -3'  Off-target 1
5'- CTTGCCCCACAGGGCAGTAACGG -3'  Target sequence

5'-CGTGCCCCACAGGG-AGTGAGGG -3'   Off-target 3
5'-CTTGCCCCACAGGGCAGTAACGG -3'   Target sequence

5'-TGTGCCCCACA-GGCAGTAGATG -3'   Off-target 2
5'-CTTGCCCCACAGGGCAGTAACGG -3'   Target sequence

5'-CTT-CCCCAATATCC-AGTAGGG -3'   Off-target 4
5'-CTTGCCCCACAGGGCAGTAACGG -3'   Target sequence
```

Fig. 15a

Fig. 15b

**HBB**

**Single**        **Multiplex**

(900 nM sgRNA)        (82 nM sgRNA)

13    17    3

Fig. 15c

Fig. 16a

Fig. 16b

Fig. 16c

Number of sites having mismatch of less than or equal to 6 nucleotides

Fig. 16d

Fig. 17

Fig. 18

Fig. 19

a   HEK293-1

Digenome          CHIP

15  1      49

b   HEK293-2

Digenome          CHIP

33   2   15

c   HEK293-3

Digenome          CHIP

30  1      40

d   HEK293-4

Digenome          CHIP

210  2   483

Fig. 20

GGTATCTAAGTCATTACCTGTGG   RNF2_4

AATATGTTAGTCATTACCTGAGG   RNF2_3

GTCATCTTAGTCATTACCTGAGG   **Target**

0.01     0.1     1     10     100

□(+) RGEN    ■(-) RGEN

Fig. 21a

5'-GNNNNNNNNNNNNNNNNNNNNNGG-3'   **Target DNA sequence**

5'-GNNNNNNNNNNNNNNNNNNNNNN-3'   GX$_{19}$ sgRNA

5'-GNNNNNNNNNNNNNNNNNNNNNNNGG-3'   **Target DNA sequence**

5'-GG GNNNNNNNNNNNNNNNNNNNNNN-3'   ggX$_{20}$ sgRNA
     Two mismatched extra guanines

Fig. 21b

*EMX1*

AAGTCAGAGGAGAAGAAGAAGGG   EMX1_28

GAGCCTGAGCAGAAGGAGAAGGG   EMX1_22

GAATCCAAGCAGAAGAAGAGAAG   EMX1_20

GAGTAGGAGCAGGAGAAGAAGGA   EMX1_14

GAGTCCGGGAAGGAGAAGAAAGG   EMX1_13

GAGTCTAAGCAGGAGAATAAAGG   EMX1_11

AAGTCCCGGCAGAGGAAGAAGGG   EMX1_9

ACGTCTGAGCAGAAGAAGAATGG   EMX1_8

GAGCCGGAGCAGAAGAAGGAGGG   EMX1_6

GAGTCCTAGCAGGAGAAGAAGAG   EMX1_5

GAGGCCGAGCAGAAGAAAGACGG   EMX1_4

GAGTCTAAGCAGAAGAAGAAGAG   EMX1_3

GAGTTAGAGCAGAAGAAGAAAGG   EMX1_2

GAGTCCGAGCAGAAGAAGAAGGG   **Target**

**Indel frequencies (%)**

■ ggX₂₀ sgRNA   □ GX₁₉ sgRNA   ▨ (-) RGEN

Fig. 21c

## *HEK293-3*

AGACCAGACTGAGCAAGAGAGGG HEK3_9

GACACAGACCGGGCACGTGAGGG HEK3_8

CACCCAGACTGAGCACGTGCTGG HEK3_6

GAGCCAGAATGAGCACGTGAGGG HEK3_5

AGCTCAGACTGAGCAAGTGAGGG HEK3_4

GGCTCAGACTGAGCACCTGAGAG HEK3_2

GGCCCAGACTGAGCACGTGATGG **Target**

**Indel frequencies (%)**

■ ggX₂₀ sgRNA  □ GX₁₉ sgRNA  ▨ (-) RGEN

Fig. 21d

## *RNF2*

GGTATCTAAGTCATTACCTGTGG RNF2_4

AATATGTTAGTCATTACCTGAGG RNF2_3

GTCATCTTAGTCATTACCTGAGG **Target**

**Indel frequencies (%)**

■ ggX₂₀ sgRNA  □ GX₁₉ sgRNA  ▨ (-) RGEN

Fig. 21e

*EMX1*

| Target | Ratio of specificity (on-target/off-target) | | |
|---|---|---|---|
| | GX19 | ggX20 | ggX20/GX19 |
| EMX1_2 | 1.2 | 36.0 | 31.2 |
| EMX1_3 | 1.2 | 46.0 | 37.7 |
| EMX1_4 | 3.4 | 477.0 | 142.0 |
| EMX1_5 | 3.7 | 1543.4 | 420.1 |
| EMX1_6 | 208.7 | 2560.4 | 12.3 |
| EMX1_8 | 92.9 | 1454.4 | 15.6 |
| EMX1_9 | 461.2 | 5157.4 | 11.2 |
| EMX1_11 | 308.7 | 1055.8 | 3.4 |
| EMX1_13 | 65.6 | 5406.6 | 82.4 |
| EMX1_14 | 94.1 | 1125.4 | 12.0 |
| EMX1_20 | 1750.7 | 4313.0 | 2.5 |
| EMX1_22 | 982.2 | 1760.9 | 1.8 |
| EMX1_28 | 125.0 | 155.3 | 1.2 |

Fig. 21f

*HEK293-3*

| Target | Ratio of specificity (on-target/off-target) | | |
| --- | --- | --- | --- |
| | GX19 | ggX20 | ggX20/GX19 |
| HEK3_2 | 240.0 | 3220.9 | 13.4 |
| HEK3_4 | 3.8 | 2264.1 | 597.9 |
| HEK3_5 | 36.9 | 8716.1 | 236.3 |
| HEK3_6 | 2.1 | 54.8 | 26.0 |
| HEK3_8 | 475.5 | 5618.8 | 11.8 |
| HEK3_9 | 628.8 | 2803.3 | 4.5 |

Fig. 21g

*RNF2*

| Target | Ratio of specificity (on-target/off-target) | | |
| --- | --- | --- | --- |
| | GX19 | ggX20 | ggX20/GX19 |
| RNF2_3 | 1985.1 | 5312.7 | 2.7 |
| RNF2_4 | 234.9 | 4749.7 | 20.2 |

Fig. 22a

Fig. 22b

**Sites validated by NGS**

Number of 3 mismatches

Number of 4 mismatches

Number of 5 mismatches

Number of mismatches in seed region

Number of mismatches in seed region

Number of mismatches in seed region

Off-target indel %/On-target indel %

Fig. 22c

Sites not validated by NGS

Fig. 23a

Fig. 23b

**VEGFA site 2 (143)**

**VEGFA site 3 (48)**

**EMX 1 (14)**

**HEK site 3 (6)**

**FANCF (8)**

Fig. 24a

# Target site

(-) ZFN-224

(+) ZFN-224

Fig. 24b

(-) ZFN
(+) ZFN-224
WT FokI
(+) ZFN-224
KKEL FokI

Fig. 24c

(+) ZFN-224 WT FokI

Fig. 24d

Fig. 25a

Fig. 25b

**Candidates**

Fig. 25c

**Validated target**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013169398 A2
- US 20050064474
- US 20060188987
- US 6479626 B
- US 6903185 B
- US 7153949 B
- WO 2012093833 A
- US 20130217131

### Non-patent literature cited in the description

- *Nucleic acids research,* 2013, vol. 41 (20), 9584-9592
- *Genome Res,* 2014, vol. 24, 132-141
- *Proc Natl Acad Sci,* 2009, vol. 106, 10620-10625
- **CRADICK, T.J. ; FINE, E.J. ; ANTICO, C.J. ; BAO, G.** CRISPR/Cas9 systems targeting β-globin and CCR5 genes have substantial off-target activity. *Nucleic acids research,* 2013, vol. 41 (20), 9584-9592
- **MONET et al.** *Biochem. Biophysics Res. Common.,* 1999, vol. 255, 88-93
- **BEERLI et al.** *Nature Biotechnol.,* 2002, vol. 20, 135-141
- **PABO et al.** *Ann. Rev. Biochem.,* 2001, vol. 70, 313-340
- **ISALAN et al.** *Nature Biotechnol.,* 2001, vol. 19, 656-660
- **SEGAL et al.** *Curr. Opin. Biotechnol.,* 2001, vol. 12, 632-637
- **CHOO et al.** *Curr. Opin. Struct. Biol.,* 2000, vol. 10, 411-416
- *Cell,* 2015, vol. 163 (3), 759-71